# EUROPEAN PATENT SPECIFICATION

(11) **EP 0 907 731 B1**
(45) Date of publication and mention of the grant of the patent: **02.03.2005**
(21) Application number: 97908260.9
(22) Date of filing: 19.03.1997
(51) Int. Cl.: C12N 15/12, C07K 14/705, C07K 16/28, G01N 33/68, C07F 9/30, C12N 15/11, A01K 67/027

(54) **METABOTROPIC GABA(B) RECEPTORS, RECEPTOR-SPECIFIC LIGANDS AND THEIR USES**
METABOTROPISCHE GABA(B) REZEPTOREN, REZEPTOREN-SPEZIFISCHE LIGANDEN UND DEREN ANWENDUNGEN
RECEPTEURS METABOTROPES DE GABA(B), LIGANDS SPECIFIQUES DE CEUX-CI ET UTILISATIONS ASSOCIEES

(30) Priority: 30.05.1996 US 655716; 22.11.1996 US 756091
(43) Date of publication of application: 14.04.1999
(73) Proprietor: Novartis AG, 4056 Basel (CH); Novartis Pharma GmbH, 1230 Wien (AT)
(72) Inventor: KAUPMANN, Klemens, D-79539 Lörrach (DE); BETTLER, Bernhard, CH-4123 Allschwil (CH); BITTIGER, Helmut, D-79104 Freiburg (DE); FRÖSTL, Wolfgang, CH-4051 Basel (CH); MICKEL, Stuart, John, CH-4415 Lausen (CH)
(74) Representative: Grubb, Philip William
(86) International application number: PCT/EP1997/001370
(87) International publication number: WO 1997/046675

(56) References cited:
- EP-A- 0 569 333
- KAUPMANN K ET AL: "Expression cloning of GABA(B) receptors uncovers similarity to metabotropic glutamate receptors [see comments]" NATURE, MAR 20 1997, 386 (6622) P239-46, ENGLAND, XP002032306
- NAKAYASU H ET AL: "Immunoaffinity purification and characterization of gamma-aminobutyric acid (GABA)B receptor from bovine cerebral cortex." J BIOL CHEM, APR 25 1993, 268 (12) P8658-64, UNITED STATES, XP002032307
- KURIYAMA K ET AL: "Structure and function of cerebral GABAA and GABAB receptors." NEUROSCI RES, JUL 1993, 17 (2) P91-9, IRELAND, XP000674902
- HIROUCHI, MASAAKI ET AL: "Molecular biological approaches to the GABAB receptor" PHARMACOL. REV. COMMUN., 1996, 151, XP000675068
- GASPARINI P.: "Hereditary hemochromatosis : generation of a transcription map within a refined and extended map of HLA 1 class region" GENOMICS, vol. 31, 1996, pages 319-326, XP000675389

### SEQUENCE LISTING

(1) GENERAL INFORMATION:
   (i) APPLICANT:
      (A) NAME: NOVARTIS AG
      (B) STREET: SCHWARZWALDALLEE
      (C) CITY: Basel
      (E) COUNTRY: Switzerland
      (F) POSTAL CODE (ZIP): 4002
      (G) TELEPHONE: +41 61 696 11 11
      (H) TELEFAX: +41 61 696 79 76
      (I) TELEX: 962 991
   (ii) TITLE OF INVENTION: Novel Receptors
   (iii) NUMBER OF SEQUENCES: 8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 4376 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Rattus norvegicus
   (vii) IMMEDIATE SOURCE:
      (B) CLONE: GABABRla rat
   (ix) FEATURE:
      (A) NAME/KEY: CDS
      (B) LOCATION:182..3061
   (ix) FEATURE:
      (A) NAME/KEY: mat_peptide
      (B) LOCATION:182..3061
   (xi) SEQUENCE DESCRIPTION: SEQ ID NO: 1:
   GTGGGGTTTG CGGGTAGCGA TCGAGAAGGG GAGAGACCCC GGCCAGGCAG GAGCCTGGAT      60
   TCCTGTGGAA GAAGAACAGG GGGAGGGGAA GCTGGAGGAC CGGGAGGGAG AACGGGGAGC     120
   CGCGGCCGGG CCTGGGGCCT TGAGGCCCGG GGAGAGCCGC GGAGCGGGAC CGGCCGCCGA     180
   G ATG CTG CTG CTG CTG CTG GTG CCT CTC TTC CTC CGC CCC CTG GGC        226
     Met Leu Leu Leu Leu Leu Val Pro Leu Phe Leu Arg Pro Leu Gly
       1               5                  10                  15
   GCT GGC GGG GCG CAG ACC CCC AAC GCC ACC TCG GAA GGT TGC CAG ATT      274
   Ala Gly Gly Ala Gln Thr Pro Asn Ala Thr Ser Glu Gly Cys Gln Ile
                    20                  25                  30
   ATA CAT CCG CCC TGG GAA GGT GGC ATC AGG TAC CGT GGC TTG ACT CGC      322
   Ile His Pro Pro Trp Glu Gly Gly Ile Arg Tyr Arg Gly Leu Thr Arg
                35                  40                  45
   GAC CAG GTG AAG GCC ATC AAC TTC CTG CCT GTG GAC TAT GAG ATC GAA      370
   Asp Gln Val Lys Ala Ile Asn Phe Leu Pro Val Asp Tyr Glu Ile Glu
               50                  55                  60
   TAT GTG TGC CGA GGG GAG CGC GAG GTG GTG GGC CCC AAG GTG CGC AAA      418
   Tyr Val Cys Arg Gly Glu Arg Glu Val Val Gly Pro Lys Val Arg Lys
           65                  70                  75
   TGC CTG GCC AAC GGC TCC TGG ACG GAT ATG GAC ACA CCC AGC CGC TGT      466
   Cys Leu Ala Asn Gly Ser Trp Thr Asp Met Asp Thr Pro Ser Arg Cys
    80                  85                  90                  95
   GTC CGA ATC TGC TCC AAG TCT TAT TTG ACC CTG GAA AAT GGG AAG GTT      514
   Val Arg Ile Cys Ser Lys Ser Tyr Leu Thr Leu Glu Asn Gly Lys Val
                   100                 105                 110
   TTC CTG ACG GGT GGG GAC CTC CCA GCT CTG GAT GGA GCC CGG GTG GAG      562
   Phe Leu Thr Gly Gly Asp Leu Pro Ala Leu Asp Gly Ala Arg Val Glu
               115                 120                 125
   TTC CGA TGT GAC CCC GAC TTC CAT CTG GTG GGC AGC TCC CGG AGC GTC      610
   Phe Arg Cys Asp Pro Asp Phe His Leu Val Gly Ser Ser Arg Ser Val
           130                 135                 140
   TGT AGT CAG GGC CAG TGG AGC ACC CCC AAG CCC CAC TGC CAG GTG AAT      658
   Cys Ser Gln Gly Gln Trp Ser Thr Pro Lys Pro His Cys Gln Val Asn
       145                 150                 155
   CGA ACG CCA CAC TCA GAA CGG CGT GCA GTA TAC ATC GGG GCG CTG TTT      706
   Arg Thr Pro His Ser Glu Arg Arg Ala Val Tyr Ile Gly Ala Leu Phe
   160                 165                 170                 175
   CCC ATG AGC GGG GGC TGG CCG GGG GGC CAG GCC TGC CAG CCC GCG GTG      754
   Pro Met Ser Gly Gly Trp Pro Gly Gly Gln Ala Cys Gln Pro Ala Val
                   180                 185                 190
   GAG ATG GCG CTG GAG GAC GTT AAC AGC CGC AGA GAC ATC CTG CCG GAC 802
   Glu Met Ala Leu Glu Asp Val Asn Ser Arg Arg Asp Ile Leu Pro Asp
   195 200 205
   TAC GAG CTC AAG CTT ATC CAC CAC GAC AGC AAG TGT GAC CCA GGG CAA 850
   Tyr Glu Leu Lys Leu Ile His His Asp Ser Lys Cys Asp Pro Gly Gln
   210 215 220
   GCC ACC AAG TAC TTG TAC GAA CTA CTC TAC AAT GAC CCC ATC AAG ATC 898
   Ala Thr Lys Tyr Leu Tyr Glu Leu Leu Tyr Asn Asp Pro Ile Lys Ile
   225 230 235
   ATT CTC ATG CCT GGC TGT AGT TCT GTC TCC ACA CTT GTA GCT GAG GCT 946
   Ile Leu Met Pro Gly Cys Ser Ser Val Ser Thr Leu Val Ala Glu Ala
   240 245 250 255
   GCC CGG ATG TGG AAC CTT ATT GTG CTC TCA TAT GGC TCC AGT TCA CCA 994
   Ala Arg Met Trp Asn Leu Ile Val Leu Ser Tyr Gly Ser Ser Ser Pro
   260 265 270
   GCC TTG TCA AAC CGA CAG CGG TTT CCC ACG TTC TTC CGG ACG CAT CCA 1042
   Ala Leu Ser Asn Arg Gln Arg Phe Pro Thr Phe Phe Arg Thr His Pro
   275 280 285
   TCC GCC ACA CTC CAC AAT CCC ACC CGG GTG AAA CTC TTC GAA AAG TGG 1090
   Ser Ala Thr Leu His Asn Pro Thr Arg Val Lys Leu Phe Glu Lys Trp
   290 295 300
   GGC TGG AAG AAG ATC GCT ACC ATC CAA CAG ACC ACC GAG GTC TTC ACC 1138
   Gly Trp Lys Lys Ile Ala Thr Ile Gln Gln Thr Thr Glu Val Phe Thr
   305 310 315
   TCA ACG CTG GAT GAC CTG GAG GAG CGA GTG AAA GAG GCT GGG ATC GAG 1186
   Ser Thr Leu Asp Asp Leu Glu Glu Arg Val Lys Glu Ala Gly Ile Glu
   320 325 330 335
   ATC ACT TTC CGA CAG AGT TTC TTC TCG GAT CCA GCT GTG CCT GTT AAA      1234
   Ile Thr Phe Arg Gln Ser Phe Phe Ser Asp Pro Ala Val Pro Val Lys
                   340                 345                 350
   AAC CTG AAG CGT CAA GAT GCT CGA ATC ATC GTG GGA CTT TTC TAT GAG      1282
   Asn Leu Lys Arg Gln Asp Ala Arg Ile Ile Val Gly Leu Phe Tyr Glu
               355                 360                 365
   ACG GAA GCC CGG AAA GTT TTT TGT GAG GTC TAT AAG GAA AGG CTC TTT      1330
   Thr Glu Ala Arg Lys Val Phe Cys Glu Val Tyr Lys Glu Arg Leu Phe
           370                 375                 380
   GGG AAG AAG TAC GTC TGG TTC CTC ATC GGG TGG TAT GCT GAC AAC TGG      1378
   Gly Lys Lys Tyr Val Trp Phe Leu Ile Gly Trp Tyr Ala Asp Asn Trp
       385                 390                 395
   TTC AAG ACC TAT GAC CCG TCA ATC AAT TGT ACA GTG GAA GAA ATG ACC      1426
   Phe Lys Thr Tyr Asp Pro Ser Ile Asn Cys Thr Val Glu Glu Met Thr
   400                 405                 410                 415
   GAG GCG GTG GAG GGC CAC ATC ACC ACG GAG ATT GTC ATG CTG AAC CCT      1474
   Glu Ala Val Glu Gly His Ile Thr Thr Glu Ile Val Met Leu Asn Pro
                   420                 425                 430
   GCC AAC ACC CGA AGC ATT TCC AAC ATG ACG TCA CAG GAA TTT GTG GAG      1522
   Ala Asn Thr Arg Ser Ile Ser Asn Met Thr Ser Gln Glu Phe Val Glu
               435                 440                 445
   AAA CTA ACC AAG CGG CTG AAA AGA CAC CCC GAG GAG ACT GGA GGC TTC      1570
   Lys Leu Thr Lys Arg Leu Lys Arg His Pro Glu Glu Thr Gly Gly Phe
           450                 455                 460
   CAG GAG GCA CCA CTG GCC TAT GAT GCT ATC TGG GCC TTG GCT TTG GCC      1618
   Gln Glu Ala Pro Leu Ala Tyr Asp Ala Ile Trp Ala Leu Ala Leu Ala
       465                 470                 475
   TTG AAC AAG ACG TCT GGA GGA GGT GGT CGT TCC GGC GTG CGC CTG GAG        1666
   Leu Asn Lys Thr Ser Gly Gly Gly Gly Arg Ser Gly Val Arg Leu Glu
   480                 485                 490                 495
   GAC TTT AAC TAC AAC AAC CAG ACC ATT ACA GAC CAG ATC TAC CGG GCC        1714
   Asp Phe Asn Tyr Asn Asn Gln Thr Ile Thr Asp Gln Ile Tyr Arg Ala
                   500                 505                 510
   ATG AAC TCC TCC TCC TTT GAG GGC GTT TCT GGC CAT GTG GTC TTT GAT        1762
   Met Asn Ser Ser Ser Phe Glu Gly Val Ser Gly His Val Val Phe Asp
               515                 520                 525
   GCC AGC GGC TCC CGG ATG GCA TGG ACA CTT ATC GAG CAG CTA CAG GGC        1810
   Ala Ser Gly Ser Arg Met Ala Trp Thr Leu Ile Glu Gln Leu Gln Gly
           530                 535                 540
   GGC AGC TAC AAG AAG ATC GGC TAC TAC GAC AGC ACC AAG GAT GAT CTT        1858
   Gly Ser Tyr Lys Lys Ile Gly Tyr Tyr Asp Ser Thr Lys Asp Asp Leu
       545                 550                 555
   TCC TGG TCC AAA ACG GAC AAG TGG ATT GGA GGG TCT CCC CCA GCT GAC        1906
   Ser Trp Ser Lys Thr Asp Lys Trp Ile Gly Gly Ser Pro Pro Ala Asp
   560                 565                 570                 575
   CAG ACC TTG GTC ATC AAG ACA TTC CGT TTC CTG TCT CAG AAA CTC TTT        1954
   Gln Thr Leu Val Ile Lys Thr Phe Arg Phe Leu Ser Gln Lys Leu Phe
                   580                 585                 590
   ATC TCC GTC TCA GTT CTC TCC AGC CTG GGC ATT GTT CTT GCT GTT GTC        2002
   Ile Ser Val Ser Val Leu Ser Ser Leu Gly Ile Val Leu Ala Val Val
               595                 600                 605
   TGT CTG TCC TTT AAC ATC TAC AAC TCC CAC GTT CGT TAT ATC CAG AAC        2050
   Cys Leu Ser Phe Asn Ile Tyr Asn Ser His Val Arg Tyr Ile Gln Asn
           610                 615                 620
   TCC CAG CCC AAC CTG AAC AAT CTG ACT GCT GTG GGC TGC TCA CTG GCA        2098
   Ser Gln Pro Asn Leu Asn Asn Leu Thr Ala Val Gly Cys Ser Leu Ala
       625                 630                 635
   CTG GCT GCT GTC TTC CCT CTC GGG CTG GAT GGT TAC CAC ATA GGG AGA        2146
   Leu Ala Ala Val Phe Pro Leu Gly Leu Asp Gly Tyr His Ile Gly Arg
   640                 645                 650                 655
   AGC CAG TTC CCG TTT GTC TGC CAG GCC CGC CTT TGG CTC TTG GGC TTG        2194
   Ser Gln Phe Pro Phe Val Cys Gln Ala Arg Leu Trp Leu Leu Gly Leu
                   660                 665                 670
   GGC TTT AGT CTG GGC TAT GGC TCT ATG TTC ACC AAG ATC TGG TGG GTC        2242
   Gly Phe Ser Leu Gly Tyr Gly Ser Met Phe Thr Lys Ile Trp Trp Val
               675                 680                 685
   CAC ACA GTC TTC ACG AAG AAG GAG GAG AAG AAG GAG TGG AGG AAG ACC        2290
   His Thr Val Phe Thr Lys Lys Glu Glu Lys Lys Glu Trp Arg Lys Thr
           690                 695                 700
   CTA GAG CCC TGG AAA CTC TAT GCC ACT GTG GGC CTG CTG GTG GGC ATG        2338
   Leu Glu Pro Trp Lys Leu Tyr Ala Thr Val Gly Leu Leu Val Gly Met
       705                 710                 715
   GAT GTC CTG ACT CTT GCC ATC TGG CAG ATT GTG GAC CCC TTG CAC CGA        2386
   Asp Val Leu Thr Leu Ala Ile Trp Gln Ile Val Asp Pro Leu His Arg
   720                 725                 730                 735
   ACC ATT GAG ACT TTT GCC AAG GAG GAA CCA AAG GAA GAC ATC GAT GTC        2434
   Thr Ile Glu Thr Phe Ala Lys Glu Glu Pro Lys Glu Asp Ile Asp Val
                   740                 745                 750
   TCC ATT CTG CCC CAG TTG GAG CAC TGC AGC TCC AAG AAG ATG AAT ACG        2482
   Ser Ile Leu Pro Gln Leu Glu His Cys Ser Ser Lys Lys Met Asn Thr
               755                 760                 765
   TGG CTT GGC ATT TTC TAT GGT TAC AAG GGG CTG CTG CTG CTG CTG GGA       2530
   Trp Leu Gly Ile Phe Tyr Gly Tyr Lys Gly Leu Leu Leu Leu Leu Gly
           770                     775                     780
   ATC TTT CTT GCT TAC GAA ACC AAG AGC GTG TCC ACT GAA AAG ATC AAT       2578
   Ile Phe Leu Ala Tyr Glu Thr Lys Ser Val Ser Thr Glu Lys Ile Asn
       785                     790                     795
   GAC CAC AGG GCC GTG GGC ATG GCT ATC TAC AAT GTC GCG GTC CTG TGT       2626
   Asp His Arg Ala Val Gly Met Ala Ile Tyr Asn Val Ala Val Leu Cys
   800                     805                     810                     815
   CTC ATC ACT GCT CCT GTG ACC ATG ATC CTT TCC AGT CAG CAG GAC GCA       2674
   Leu Ile Thr Ala Pro Val Thr Met Ile Leu Ser Ser Gln Gln Asp Ala
                   820                     825                     830
   GCC TTT GCC TTT GCC TCT CTG GCC ATC GTG TTC TCT TCC TAC ATC ACT       2722
   Ala Phe Ala Phe Ala Ser Leu Ala Ile Val Phe Ser Ser Tyr Ile Thr
               835                     840                     845
   CTG GTT GTG CTC TTT GTG CCC AAG ATG CGC AGG CTG ATC ACC CGA GGG       2770
   Leu Val Val Leu Phe Val Pro Lys Met Arg Arg Leu Ile Thr Arg Gly
           850                     855                     860
   GAA TGG CAG TCT GAA ACG CAG GAC ACC ATG AAA ACA GGA TCA TCC ACC       2818
   Glu Trp Gln Ser Glu Thr Gln Asp Thr Met Lys Thr Gly Ser Ser Thr
       865                     870                     875
   AAC AAC AAC GAG GAA GAG AAG TCC CGA CTG TTG GAG AAG GAA AAC CGA       2866
   Asn Asn Asn Glu Glu Glu Lys Ser Arg Leu Leu Glu Lys Glu Asn Arg
   880                     885                     890                     895
   GAA CTG GAA AAG ATC ATC GCT GAG AAA GAG GAG CGC GTC TCT GAA CTG       2914
   Glu Leu Glu Lys Ile Ile Ala Glu Lys Glu Glu Arg Val Ser Glu Leu
                   900                     905                     910
   CGC CAT CAG CTC CAG TCT CGG CAG CAA CTC CGC TCA CGG CGC CAC CCC 2962
   Arg His Gln Leu Gln Ser Arg Gln Gln Leu Arg Ser Arg Arg His Pro
   915 920 925
   CCA ACA CCC CCA GAT CCC TCT GGG GGC CTT CCC AGG GGA CCC TCT GAG 3010
   Pro Thr Pro Pro Asp Pro Ser Gly Gly Leu Pro Arg Gly Pro Ser Glu
   930 935 940
   CCC CCT GAC CGG CTT AGC TGT GAT GGG AGT CGA GTA CAT TTG CTT TAC 3058
   Pro Pro Asp Arg Leu Ser Cys Asp Gly Ser Arg Val His Leu Leu Tyr
   945 950 955
   AAG TGAGGGGGCA TGGAGAAGGA TCAAGCCAGT AGGGGAGGGA AGGGTCTGGG 3111
   Lys
   960
   AAGAGGGTGG GGGCCTGGGA GGAGGGTAAG GACTCCTATC TCCAACCTGG AGAGCACACG 3171
   CTCCAATCCC CCTCTTATAA ATACATGTCG CTCTGTGCAT CTGGGGTTAT TTGGGTCTCC 3231
   AGTACTCTGG GAAACAGACT GTTTTCTTTC TCCCCTATAA TTTTATATCT CCACTTCACA 3291
   GGTTTTGTTT GAACCCTGCT TGGAGTTATT ATTCACTCAT GGCTCCAGAG GGGCATCTCA 3351
   TTTTTCTCCG GTAGCCTGTC TTGTACAGTT ACCACAGCAA CTCCTGTCAT TTCAGCAGCA 3411
   GGGGTCTTCC TACACTAGCA GGGCTCTCGC TCTCTCCATT TTTCAGCCTC AGAATCTCCT 3471
   TCCATTATTC TTCTCCTTCT ACATGTCTCC ATGGCTTCCT CTCCCAGGGG ACTCGTTCTA 3531
   CACACATACA CACACACACA CACACACACA CACACACACA CACACACACA CACCCCGCAT 3591
   CGTGCCCTCT CCTAGGCAGC TGCATGTCGT CCTGTACAAA TGTGCTCGCT TCTGAGTGCT 3651
   TTGTGCGGCC GTTCACTTGT GCTGTCTGCA TAAGCTGCGT CTGTGAGTGC ACGGTGGTTT 3711
   GTGGGTGCGT GAAGTGGCAT GCTCCGGTAG GTGTGTATGA TGCGTTGAGC ACGCTACGCT 3771
   GTCTCCCTCA TGTGCACGCA TTGTGTCTGC TTATGTTTTA CTTGTATGCC TCTGTGTACT 3831
   GTGTGTGTGT GTGTGTGTGC CCACGCGTGC GCCCGTGTGC ATGCGTTCGT GTTGCCCTGA 3891
   CTGGCTGTCT CAGCCTTCTG AGTAATTGGG ATTCCAGTTG TCTGTCTAGC TCATGTCCTG 3951
   TCTTCTTCCA GTAGAGCCGT GAACACCCAA CACACACAGT TAATCGGGCT CCCCCCAGTC 4011
   CATGTTTTCT GAGCCATCCA AAAACTCTCC TTGGCCTTAG GTTCATCTAC AAATGTTCCC 4071
   TCTGTTCTTT GCTCTCGTGC GTCCACCTTC ATTCTCTTCA GTCATTTCTC AGATCTGCTG 4131
   CGTCGTGGTT TCCTTTCCTT CATTATCATC GTCATTATTT TTCAGAACTT AAGGGAAAAA 4191
   GAAATGGGGA CAGGTTGGAG GCTGTTTCCA GTGGAATAGT GGGTGCGCGT CCTGACCAAA 4251
   TGAAGGCACG GACAGATGGA CTGACGGGGC GGGAGGCGGC GTCCCTTTCA CACTGTGGTG 4311
   TCTCTTGGGG GGGAAGGATC TCCCTGAATC TCAATAAAGC AGTGAACAGT AAAAAAAAAA 4371
   AAAAA 4376
(2) INFORMATION FOR SEQ ID NO: 2:
   (i) SEQUENCE CHARACTERISTICS:
      (A) LENGTH: 960 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Leu Leu Leu Leu Leu Val Pro Leu Phe Leu Arg Pro Leu Gly Ala
   1 5 10 15
   Gly Gly Ala Gln Thr Pro Asn Ala Thr Ser Glu Gly Cys Gln Ile Ile
   20 25 30
   His Pro Pro Trp Glu Gly Gly Ile Arg Tyr Arg Gly Leu Thr Arg Asp
   35 40 45
   Gln Val Lys Ala Ile Asn Phe Leu Pro Val Asp Tyr Glu Ile Glu Tyr
   50 55 60
   Val Cys Arg Gly Glu Arg Glu Val Val Gly Pro Lys Val Arg Lys Cys
   65 70 75 80
   Leu Ala Asn Gly Ser Trp Thr Asp Met Asp Thr Pro Ser Arg Cys Val
   85 90 95
   Arg Ile Cys Ser Lys Ser Tyr Leu Thr Leu Glu Asn Gly Lys Val Phe
   100 105 110
   Leu Thr Gly Gly Asp Leu Pro Ala Leu Asp Gly Ala Arg Val Glu Phe
   115 120 125
   Arg Cys Asp Pro Asp Phe His Leu Val Gly Ser Ser Arg Ser Val Cys
   130 135 140
   Ser Gln Gly Gln Trp Ser Thr Pro Lys Pro His Cys Gln Val Asn Arg
   145 150 155 160
   Thr Pro His Ser Glu Arg Arg Ala Val Tyr Ile Gly Ala Leu Phe Pro
   165 170 175
   Met Ser Gly Gly Trp Pro Gly Gly Gln Ala Cys Gln Pro Ala Val Glu
   180 185 190
   Met Ala Leu Glu Asp Val Asn Ser Arg Arg Asp Ile Leu Pro Asp Tyr
   195 200 205
   Glu Leu Lys Leu Ile His His Asp Ser Lys Cys Asp Pro Gly Gln Ala
   210 215 220
   Thr Lys Tyr Leu Tyr Glu Leu Leu Tyr Asn Asp Pro Ile Lys Ile Ile
   225 230 235 240
   Leu Met Pro Gly Cys Ser Ser Val Ser Thr Leu Val Ala Glu Ala Ala
   245 250 255
   Arg Met Trp Asn Leu Ile Val Leu Ser Tyr Gly Ser Ser Ser Pro Ala
   260 265 270
   Leu Ser Asn Arg Gln Arg Phe Pro Thr Phe Phe Arg Thr His Pro Ser
   275 280 285
   Ala Thr Leu His Asn Pro Thr Arg Val Lys Leu Phe Glu Lys Trp Gly
   290 295 300
   Trp Lys Lys Ile Ala Thr Ile Gln Gln Thr Thr Glu Val Phe Thr Ser
   305 310 315 320
   Thr Leu Asp Asp Leu Glu Glu Arg Val Lys Glu Ala Gly Ile Glu Ile
   325 330 335
   Thr Phe Arg Gln Ser Phe Phe Ser Asp Pro Ala Val Pro Val Lys Asn
   340 345 350
   Leu Lys Arg Gln Asp Ala Arg Ile Ile Val Gly Leu Phe Tyr Glu Thr
   355 360 365
   Glu Ala Arg Lys Val Phe Cys Glu Val Tyr Lys Glu Arg Leu Phe Gly
   370 375 380
   Lys Lys Tyr Val Trp Phe Leu Ile Gly Trp Tyr Ala Asp Asn Trp Phe
   385 390 395 400
   Lys Thr Tyr Asp Pro Ser Ile Asn Cys Thr Val Glu Glu Met Thr Glu
   405 410 415
   Ala Val Glu Gly His Ile Thr Thr Glu Ile Val Met Leu Asn Pro Ala
   420 425 430
   Asn Thr Arg Ser Ile Ser Asn Met Thr Ser Gln Glu Phe Val Glu Lys
   435 440 445
   Leu Thr Lys Arg Leu Lys Arg His Pro Glu Glu Thr Gly Gly Phe Gln
   450 455 460
   Glu Ala Pro Leu Ala Tyr Asp Ala Ile Trp Ala Leu Ala Leu Ala Leu
   465 470 475 480
   Asn Lys Thr Ser Gly Gly Gly Gly Arg Ser Gly Val Arg Leu Glu Asp
   485 490 495
   Phe Asn Tyr Asn Asn Gln Thr Ile Thr Asp Gln Ile Tyr Arg Ala Met
   500 505 510
   Asn Ser Ser Ser Phe Glu Gly Val Ser Gly His Val Val Phe Asp Ala
   515 520 525
   Ser Gly Ser Arg Met Ala Trp Thr Leu Ile Glu Gln Leu Gln Gly Gly
   530 535 540
   Ser Tyr Lys Lys Ile Gly Tyr Tyr Asp Ser Thr Lys Asp Asp Leu Ser
   545 550 555 560
   Trp Ser Lys Thr Asp Lys Trp Ile Gly Gly Ser Pro Pro Ala Asp Gln
   565 570 575
   Thr Leu Val Ile Lys Thr Phe Arg Phe Leu Ser Gln Lys Leu Phe Ile
   580 585 590
   Ser Val Ser Val Leu Ser Ser Leu Gly Ile Val Leu Ala Val Val Cys
   595 600 605
   Leu Ser Phe Asn Ile Tyr Asn Ser His Val Arg Tyr Ile Gln Asn Ser
   610 615 620
   Gln Pro Asn Leu Asn Asn Leu Thr Ala Val Gly Cys Ser Leu Ala Leu
   625 630 635 640
   Ala Ala Val Phe Pro Leu Gly Leu Asp Gly Tyr His Ile Gly Arg Ser
   645 650 655
   Gln Phe Pro Phe Val Cys Gln Ala Arg Leu Trp Leu Leu Gly Leu Gly
   660 665 670
   Phe Ser Leu Gly Tyr Gly Ser Met Phe Thr Lys Ile Trp Trp Val His
   675 680 685
   Thr Val Phe Thr Lys Lys Glu Glu Lys Lys Glu Trp Arg Lys Thr Leu
   690 695 700
   Glu Pro Trp Lys Leu Tyr Ala Thr Val Gly Leu Leu Val Gly Met Asp
   705 710 715 720
   Val Leu Thr Leu Ala Ile Trp Gln Ile Val Asp Pro Leu His Arg Thr
   725 730 735
   Ile Glu Thr Phe Ala Lys Glu Glu Pro Lys Glu Asp Ile Asp Val Ser
   740 745 750
   Ile Leu Pro Gln Leu Glu His Cys Ser Ser Lys Lys Met Asn Thr Trp
   755 760 765
   Leu Gly Ile Phe Tyr Gly Tyr Lys Gly Leu Leu Leu Leu Leu Gly Ile
   770 775 780
   Phe Leu Ala Tyr Glu Thr Lys Ser Val Ser Thr Glu Lys Ile Asn Asp
   785 790 795 800
   His Arg Ala Val Gly Met Ala Ile Tyr Asn Val Ala Val Leu Cys Leu
   805 810 815
   Ile Thr Ala Pro Val Thr Met Ile Leu Ser Ser Gln Gln Asp Ala Ala
   820 825 830
   Phe Ala Phe Ala Ser Leu Ala Ile Val Phe Ser Ser Tyr Ile Thr Leu
   835 840 845
   Val Val Leu Phe Val Pro Lys Met Arg Arg Leu Ile Thr Arg Gly Glu
   850 855 860
   Trp Gln Ser Glu Thr Gln Asp Thr Met Lys Thr Gly Ser Ser Thr Asn
   865 870 875 880
   Asn Asn Glu Glu Glu Lys Ser Arg Leu Leu Glu Lys Glu Asn Arg Glu
   885 890 895
   Leu Glu Lys Ile Ile Ala Glu Lys Glu Glu Arg Val Ser Glu Leu Arg
   900 905 910
   His Gln Leu Gln Ser Arg Gln Gln Leu Arg Ser Arg Arg His Pro Pro
   915 920 925
   Thr Pro Pro Asp Pro Ser Gly Gly Leu Pro Arg Gly Pro Ser Glu Pro
   930 935 940
   Pro Asp Arg Leu Ser Cys Asp Gly Ser Arg Val His Leu Leu Tyr Lys
   945 950 955 960
(2) INFORMATION FOR SEQ ID NO: 3:
   (i) SEQUENCE CHARACTERISTICS:
      (A) LENGTH: 2620 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: GABABRla/b human
   (ix) FEATURE:
      (A) NAME/KEY: CDS
      (B) LOCATION:1..2379
   (ix) FEATURE:
      (A) NAME/KEY: mat_peptide
      (B) LOCATION:1..2379
   (xi) SEQUENCE DESCRIPTION: SEQ ID NO: 3:
   GCA GTG TAC ATC GGG GCA CTG TTT CCC ATG AGC GGG GGC TGG CCA GGG      48
   Ala Val Tyr Ile Gly Ala Leu Phe Pro Met Ser Gly Gly Trp Pro Gly
     1               5                  10                  15
   GGC CAG GCC TGC CAG CCC GCG GTG GAG ATG GCG CTG GAG GAC GTG AAT 96
   Gly Gln Ala Cys Gln Pro Ala Val Glu Met Ala Leu Glu Asp Val Asn
   20 25 30
   AGC CGC AGG GAC ATC CTG CCG GAC TAT GAG CTC AAG CTC ATC CAC CAC 144
   Ser Arg Arg Asp Ile Leu Pro Asp Tyr Glu Leu Lys Leu Ile His His
   35 40 45
   GAC AGC AAG TGT GAT CCA GGC CAA GCC ACC AAG TAC CTA TAT GAG CTG 192
   Asp Ser Lys Cys Asp Pro Gly Gln Ala Thr Lys Tyr Leu Tyr Glu Leu
   50 55 60
   CTC TAC AAC GAC CCT ATC AAG ATC ATC CTT ATG CCT GGC TGC AGC TCT 240
   Leu Tyr Asn Asp Pro Ile Lys Ile Ile Leu Met Pro Gly Cys Ser Ser
   65 70 75 80
   GTC TCC ACG CTG GTG GCT GAG GCT GCT AGG ATG TGG AAC CTC ATT GTG 288
   Val Ser Thr Leu Val Ala Glu Ala Ala Arg Met Trp Asn Leu Ile Val
   85 90 95
   CTT TCC TAT GGC TCC AGC TCA CCA GCC CTG TCA AAC CGG CAG CGT TTC 336
   Leu Ser Tyr Gly Ser Ser Ser Pro Ala Leu Ser Asn Arg Gln Arg Phe
   100 105 110
   CCC ACT TTC TTC CGA ACG CAC CCA TCA GCC ACA CTC CAC AAC CCT ACC 384
   Pro Thr Phe Phe Arg Thr His Pro Ser Ala Thr Leu His Asn Pro Thr
   115 120 125
   CGC GTG AAA CTC TTT GAA AAG TGG GGC TGG AAG AAG ATT GCT ACC ATC 432
   Arg Val Lys Leu Phe Glu Lys Trp Gly Trp Lys Lys Ile Ala Thr Ile
   130 135 140
   CAG CAG ACC ACT GAG GTC TTC ACT TCG ACT CTG GAC GAC CTG GAG GAA 480
   Gln Gln Thr Thr Glu Val Phe Thr Ser Thr Leu Asp Asp Leu Glu Glu
   145 150 155 160
   CGA GTG AAG GAG GCT GGA ATT GAG ATT ACT TTC CGC CAG AGT TTC TTC        528
   Arg Val Lys Glu Ala Gly Ile Glu Ile Thr Phe Arg Gln Ser Phe Phe
                   165                 170                 175
   TCA GAT CCA GCT GTG CCC GTC AAA AAC CTG AAG CGC CAG GAT GCC CGA        576
   Ser Asp Pro Ala Val Pro Val Lys Asn Leu Lys Arg Gln Asp Ala Arg
               180                 185                 190
   ATC ATC GTG GGA CTT TTC TAT GAG ACT GAA GCC CGG AAA GTT TTT TGT        624
   Ile Ile Val Gly Leu Phe Tyr Glu Thr Glu Ala Arg Lys Val Phe Cys
           195                 200                 205
   GAG GTG TAC AAG GAG CGT CTC TTT GGG AAG AAG TAC GTC TGG TTC CTC        672
   Glu Val Tyr Lys Glu Arg Leu Phe Gly Lys Lys Tyr Val Trp Phe Leu
       210                 215                 220
   ATT GGG TGG TAT GCT GAC AAT TGG TTC AAG ATC TAC GAC CCT TCT ATC        720
   Ile Gly Trp Tyr Ala Asp Asn Trp Phe Lys Ile Tyr Asp Pro Ser Ile
   225                 230                 235                 240
   AAC TGC ACA GTG GAT GAG ATG ACT GAG GCG GTG GAG GGC CAC ATC ACA        768
   Asn Cys Thr Val Asp Glu Met Thr Glu Ala Val Glu Gly His Ile Thr
                   245                 250                 255
   ACT GAG ATT GTC ATG CTG AAT CCT GCC AAT ACC CGC AGC ATT TCC AAC        816
   Thr Glu Ile Val Met Leu Asn Pro Ala Asn Thr Arg Ser Ile Ser Asn
               260                 265                 270
   ATG ACA TCC CAG GAA TTT GTG GAG AAA CTA ACC AAG CGA CTG AAA AGA        864
   Met Thr Ser Gln Glu Phe Val Glu Lys Leu Thr Lys Arg Leu Lys Arg
           275                 280                 285
   CAC CCT GAG GAG ACA GGA GGC TTC CAG GAG GCA CCG CTG GCC TAT GAT        912
   His Pro Glu Glu Thr Gly Gly Phe Gln Glu Ala Pro Leu Ala Tyr Asp
       290                 295                 300
   GCC ATC TGG GCC TTG GCA CTG GCC CTG AAC AAG ACA TCT GGA GGA GGC        960
   Ala Ile Trp Ala Leu Ala Leu Ala Leu Asn Lys Thr Ser Gly Gly Gly
   305                 310                 315                 320
   GGC CGT TCT GGT GTG CGC CTG GAG GAC TTC AAC TAC AAC AAC CAG ACC       1008
   Gly Arg Ser Gly Val Arg Leu Glu Asp Phe Asn Tyr Asn Asn Gln Thr
                   325                 330                 335
   ATT ACC GAC CAA ATC TAC CGG GCA ATG AAC TCT TCG TCC TTT GAG GGT       1056
   Ile Thr Asp Gln Ile Tyr Arg Ala Met Asn Ser Ser Ser Phe Glu Gly
               340                 345                 350
   GTC TCT GGC CAT GTG GTG TTT GAT GCC AGC GGC TCT CGG ATG GCA TGG       1104
   Val Ser Gly His Val Val Phe Asp Ala Ser Gly Ser Arg Met Ala Trp
           355                 360                 365
   ACG CTT ATC GAG CAG CTT CAG GGT GGC AGC TAC AAG AAG ATT GGC TAC       1152
   Thr Leu Ile Glu Gln Leu Gln Gly Gly Ser Tyr Lys Lys Ile Gly Tyr
       370                 375                 380
   TAT GAC AGC ACC AAG GAT GAT CTT TCC TGG TCC AAA ACA GAT AAA TGG       1200
   Tyr Asp Ser Thr Lys Asp Asp Leu Ser Trp Ser Lys Thr Asp Lys Trp
   385                 390                 395                 400
   ATT GGA GGG TCC CCC CCA GCT GAC CAG ACC CTG GTC ATC AAG ACA TTC       1248
   Ile Gly Gly Ser Pro Pro Ala Asp Gln Thr Leu Val Ile Lys Thr Phe
                   405                 410                 415
   CGC TTC CTG TCA CAG AAA CTC TTT ATC TCC GTC TCA GTT CTC TCC AGC       1296
   Arg Phe Leu Ser Gln Lys Leu Phe Ile Ser Val Ser Val Leu Ser Ser
               420                 425                 430
   CTG GGC ATT GTC CTA GCT GTT GTC TGT CTG TCC TTT AAC ATC TAC AAC       1344
   Leu Gly Ile Val Leu Ala Val Val Cys Leu Ser Phe Asn Ile Tyr Asn
           435                 440                 445
   TCA CAT GTC CGT TAT ATC CAG AAC TCA CAG CCC AAC CTG AAC AAC CTG        1392
   Ser His Val Arg Tyr Ile Gln Asn Ser Gln Pro Asn Leu Asn Asn Leu
       450                 455                 460
   ACT GCT GTG GGC TGC TCA CTG GCT TTA GCT GCT GTC TTC CCC CTG GGG        1440
   Thr Ala Val Gly Cys Ser Leu Ala Leu Ala Ala Val Phe Pro Leu Gly
   465                 470                 475                 480
   CTC GAT GGT TAC CAC ATT GGG AGG AAC CAG TTT CCT TTC GTC TGC CAG        1488
   Leu Asp Gly Tyr His Ile Gly Arg Asn Gln Phe Pro Phe Val Cys Gln
                   485                 490                 495
   GCC CGC CTC TGG CTC CTG GGC CTG GGC TTT AGT CTG GGC TAC GGT TCC        1536
   Ala Arg Leu Trp Leu Leu Gly Leu Gly Phe Ser Leu Gly Tyr Gly Ser
               500                 505                 510
   ATG TTC ACC AAG ATT TGG TGG GTC CAC ACG GTC TTC ACA AAG AAG GAA        1584
   Met Phe Thr Lys Ile Trp Trp Val His Thr Val Phe Thr Lys Lys Glu
           515                 520                 525
   GAA AAG AAG GAG TGG AGG AAG ACT CTG GAA CCC TGG AAG CTG TAT GCC        1632
   Glu Lys Lys Glu Trp Arg Lys Thr Leu Glu Pro Trp Lys Leu Tyr Ala
       530                 535                 540
   ACA GTG GGC CTG CTG GTG GGC ATG GAT GTC CTC ACT CTC GCC ATC TGG        1680
   Thr Val Gly Leu Leu Val Gly Met Asp Val Leu Thr Leu Ala Ile Trp
   545                 550                 555                 560
   CAG ATC GTG GAC CCT CTG CAC CGG ACC ATT GAG ACA TTT GCC AAG GAG        1728
   Gln Ile Val Asp Pro Leu His Arg Thr Ile Glu Thr Phe Ala Lys Glu
                   565                 570                 575
   GAA CCT AAG GAA GAT ATT GAC GTC TCT ATT CTG CCC CAG CTG GAG CAT        1776
   Glu Pro Lys Glu Asp Ile Asp Val Ser Ile Leu Pro Gln Leu Glu His
               580                 585                 590
   TGC AGC TCC AGG AAG ATG AAT ACA TGG CTT GGC ATT TTC TAT GGT TAC        1824
   Cys Ser Ser Arg Lys Met Asn Thr Trp Leu Gly Ile Phe Tyr Gly Tyr
           595                 600                 605
   AAG GGG CTG CTG CTG CTG CTG GGA ATC TTC CTT GCT TAT GAG ACC AAG        1872
   Lys Gly Leu Leu Leu Leu Leu Gly Ile Phe Leu Ala Tyr Glu Thr Lys
       610                 615                 620
   AGT GTG TCC ACT GAG AAG ATC AAT GAT CAC CGG GCT GTG GGC ATG GCT        1920
   Ser Val Ser Thr Glu Lys Ile Asn Asp His Arg Ala Val Gly Met Ala
   625                 630                 635                 640
   ATC TAC AAT GTG GCA GTC CTG TGC CTC ATC ACT GCT CCT GTC ACC ATG        1968
   Ile Tyr Asn Val Ala Val Leu Cys Leu Ile Thr Ala Pro Val Thr Met
                   645                 650                 655
   ATT CTG TCC AGC CAG CAG GAT GCA GCC TTT GCC TTT GCC TCT CTT GCC        2016
   Ile Leu Ser Ser Gln Gln Asp Ala Ala Phe Ala Phe Ala Ser Leu Ala
               660                 665                 670
   ATA GTT TTC TCC TCC TAT ATC ACT CTT GTT GTG CTC TTT GTG CCC AAG        2064
   Ile Val Phe Ser Ser Tyr Ile Thr Leu Val Val Leu Phe Val Pro Lys
           675                 680                 685
   ATG CGC AGG CTG ATC ACC CGA GGG GAA TGG CAG TCG GAG GCG CAG GAC        2112
   Met Arg Arg Leu Ile Thr Arg Gly Glu Trp Gln Ser Glu Ala Gln Asp
       690                 695                 700
   ACC ATG AAG ACA GGG TCA TCG ACC AAC AAC AAC GAG GAG GAG AAG TCC        2160
   Thr Met Lys Thr Gly Ser Ser Thr Asn Asn Asn Glu Glu Glu Lys Ser
   705                 710                 715                 720
   CGG CTG TTG GAG AAG GAG AAC CGT GAA CTG GAA AAG ATC ATT GCT GAG        2208
   Arg Leu Leu Glu Lys Glu Asn Arg Glu Leu Glu Lys Ile Ile Ala Glu
                   725                 730                 735
   AAA GAG GAG CGT GTC TCT GAA CTG CGC CAT CAA CTC CAG TCT CGG CAG 2256
   Lys Glu Glu Arg Val Ser Glu Leu Arg His Gln Leu Gln Ser Arg Gln
   740 745 750
   CAG CTC CGC TCC CGG CGC CAC CCA CCG ACA CCC CCA GAA CCC TCT GGG 2304
   Gln Leu Arg Ser Arg Arg His Pro Pro Thr Pro Pro Glu Pro Ser Gly
   755 760 765
   GGC CTG CCC AGG GGA CCC CCT GAG CCC CCC GAC CGG CTT AGC TGT GAT 2352
   Gly Leu Pro Arg Gly Pro Pro Glu Pro Pro Asp Arg Leu Ser Cys Asp
   770 775 780
   GGG AGT CGA GTG CAT TTG CTT TAT AAG TGAGGGTAGG GTGAGGGAGG 2399
   Gly Ser Arg Val His Leu Leu Tyr Lys
   785 790
   ACAGGCCAGT AGGGGGAGGG AAAGGGAGAG GGGAAGGGCA GGGGACTCAG GAAGCAGGGG 2459
   GTCCCCATCC CCAGCTGGGA AGAACATGCT ATCCAATCTC ATCTCTTGTA AATACATGTC 2519
   CCCCTGTGAG TTCTGGGCTG ATTTGGGTCT CTCATACCTC TGGGAAACAG ACCTTTTTCT 2579
   CTCTTACTGC TTCATGTAAT TTTGGAATTC CACCACACTG G 2620
(2) INFORMATION FOR SEQ ID NO: 5:
   (i) SEQUENCE CHARACTERISTICS:
      (A) LENGTH: 2837 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Rattus norvegicus
   (vii) IMMEDIATE SOURCE:
      (B) CLONE: GABABR1b rat
   (ix) FEATURE:
      (A) NAME/KEY: CDS
      (B) LOCATION:228..2759
   (ix) FEATURE:
      (A) NAME/KEY: mat_peptide
      (B) LOCATION:228..2759
   (xi) SEQUENCE DESCRIPTION: SEQ ID NO: 5:
   AACGGCCGCC AGTGTGCTGG AAAGGGAGAG TCCGCGGTGG CGGGAGCGAA CGTCTCCTGG 60
   CCCTAGGAAG CCCACGTCTC TGCCTTCCCC GGGCTCTGGC CCCTCCTCCC CAATGAGACC      120
   GGGGATGGAG ACACCTCCCC GACGCCCTCC CAGAAGCCTT CCCCAGAAGA AGTGTCCCCC      180
   CTGAGCTGCC CCCCACCCCA AGGAGGCCGC CCCCGCCCCC CCTCGCC ATG GGC CCG        236
                                                       Met Gly Pro
                                                        1
   GGG GGA CCC TGT ACC CCA GTG GGG TGG CCG CTG CCT CTT CTG CTG GTG        284
   Gly Gly Pro Cys Thr Pro Val Gly Trp Pro Leu Pro Leu Leu Leu Val
        5                  10                  15
   ATG GCG GCT GGG GTG GCT CCG GTG TGG GCC TCT CAC TCC CCT CAT CTC        332
   Met Ala Ala Gly Val Ala Pro Val Trp Ala Ser His Ser Pro His Leu
    20                  25                  30                  35
   CCG CGG CCT CAC CCG AGG GTC CCC CCG CAC CCC TCC TCA GAA CGG CGT        380
   Pro Arg Pro His Pro Arg Val Pro Pro His Pro Ser Ser Glu Arg Arg
                    40                  45                  50
   GCA GTA TAC ATC GGG GCG CTG TTT CCC ATG AGC GGG GGC TGG CCG GGG        428
   Ala Val Tyr Ile Gly Ala Leu Phe Pro Met Ser Gly Gly Trp Pro Gly
                55                  60                  65
   GGC CAG GCC TGC CAG CCC GCG GTG GAG ATG GCG CTG GAG GAC GTT AAC        476
   Gly Gln Ala Cys Gln Pro Ala Val Glu Met Ala Leu Glu Asp Val Asn
            70                  75                  80
   AGC CGC AGA GAC ATC CTG CCG GAC TAC GAG CTC AAG CTT ATC CAC CAC        524
   Ser Arg Arg Asp Ile Leu Pro Asp Tyr Glu Leu Lys Leu Ile His His
        85                  90                  95
   GAC AGC AAG TGT GAC CCA GGG CAA GCC ACC AAG TAC TTG TAC GAA CTA        572
   Asp Ser Lys Cys Asp Pro Gly Gln Ala Thr Lys Tyr Leu Tyr Glu Leu
   100                 105                 110                 115
   CTC TAC AAT GAC CCC ATC AAG ATC ATT CTC ATG CCT GGC TGT AGT TCT        620
   Leu Tyr Asn Asp Pro Ile Lys Ile Ile Leu Met Pro Gly Cys Ser Ser
                   120                 125                 130
   GTC TCC ACA CTT GTA GCT GAG GCT GCC CGG ATG TGG AAC CTT ATT GTG        668
   Val Ser Thr Leu Val Ala Glu Ala Ala Arg Met Trp Asn Leu Ile Val
               135                 140                 145
   CTC TCA TAT GGC TCC AGT TCA CCA GCC TTG TCA AAC CGA CAG CGG TTT        716
   Leu Ser Tyr Gly Ser Ser Ser Pro Ala Leu Ser Asn Arg Gln Arg Phe
           150                 155                 160
   CCC ACG TTC TTC CGG ACG CAT CCA TCC GCC ACA CTC CAC AAT CCC ACC        764
   Pro Thr Phe Phe Arg Thr His Pro Ser Ala Thr Leu His Asn Pro Thr
       165                 170                 175
   CGG GTG AAA CTC TTC GAA AAG TGG GGC TGG AAG AAG ATC GCT ACC ATC        812
   Arg Val Lys Leu Phe Glu Lys Trp Gly Trp Lys Lys Ile Ala Thr Ile
   180                 185                 190                 195
   CAA CAG ACC ACC GAG GTC TTC ACC TCA ACG CTG GAT GAC CTG GAG GAG        860
   Gln Gln Thr Thr Glu Val Phe Thr Ser Thr Leu Asp Asp Leu Glu Glu
                   200                 205                 210
   CGA GTG AAA GAG GCT GGG ATC GAG ATC ACT TTC CGA CAG AGT TTC TTC        908
   Arg Val Lys Glu Ala Gly Ile Glu Ile Thr Phe Arg Gln Ser Phe Phe
               215                 220                 225
   TCG GAT CCA GCT GTG CCT GTT AAA AAC CTG AAG CGT CAA GAT GCT CGA        956
   Ser Asp Pro Ala Val Pro Val Lys Asn Leu Lys Arg Gln Asp Ala Arg
           230                 235                 240
   ATC ATC GTG GGA CTT TTC TAT GAG ACG GAA GCC CGG AAA GTT TTT TGT       1004
   Ile Ile Val Gly Leu Phe Tyr Glu Thr Glu Ala Arg Lys Val Phe Cys
       245                 250                 255
   GAG GTC TAT AAG GAA AGG CTC TTT GGG AAG AAG TAC GTC TGG TTC CTC 1052
   Glu Val Tyr Lys Glu Arg Leu Phe Gly Lys Lys Tyr Val Trp Phe Leu
   260 265 270 275
   ATC GGG TGG TAT GCT GAC AAC TGG TTC AAG ACC TAT GAC CCG TCA ATC 1100
   Ile Gly Trp Tyr Ala Asp Asn Trp Phe Lys Thr Tyr Asp Pro Ser Ile
   280 285 290
   AAT TGT ACA GTG GAA GAA ATG ACC GAG GCG GTG GAG GGC CAC ATC ACC 1148
   Asn Cys Thr Val Glu Glu Met Thr Glu Ala Val Glu Gly His Ile Thr
   295 300 305
   ACG GAG ATT GTC ATG CTG AAC CCT GCC AAC ACC CGA AGC ATT TCC AAC 1196
   Thr Glu Ile Val Met Leu Asn Pro Ala Asn Thr Arg Ser Ile Ser Asn
   310 315 320
   ATG ACG TCA CAG GAA TTT GTG GAG AAA CTA ACC AAG CGG CTG AAA AGA 1244
   Met Thr Ser Gln Glu Phe Val Glu Lys Leu Thr Lys Arg Leu Lys Arg
   325 330 335
   CAC CCC GAG GAG ACT GGA GGC TTC CAG GAG GCA CCA CTG GCC TAT GAT 1292
   His Pro Glu Glu Thr Gly Gly Phe Gln Glu Ala Pro Leu Ala Tyr Asp
   340 345 350 355
   GCT ATC TGG GCC TTG GCT TTG GCC TTG AAC AAG ACG TCT GGA GGA GGT 1340
   Ala Ile Trp Ala Leu Ala Leu Ala Leu Asn Lys Thr Ser Gly Gly Gly
   360 365 370
   GGT CGT TCC GGC GTG CGC CTG GAG GAC TTT AAC TAC AAC AAC CAG ACC 1388
   Gly Arg Ser Gly Val Arg Leu Glu Asp Phe Asn Tyr Asn Asn Gln Thr
   375 380 385
   ATT ACA GAC CAG ATC TAC CGG GCC ATG AAC TCC TCC TCC TTT GAG GGC 1436
   Ile Thr Asp Gln Ile Tyr Arg Ala Met Asn Ser Ser Ser Phe Glu Gly
   390 395 400
   GTT TCT GGC CAT GTG GTC TTT GAT GCC AGC GGC TCC CGG ATG GCA TGG 1484
   Val Ser Gly His Val Val Phe Asp Ala Ser Gly Ser Arg Met Ala Trp
   405 410 415
   ACA CTT ATC GAG CAG CTA CAG GGC GGC AGC TAC AAG AAG ATC GGC TAC 1532
   Thr Leu Ile Glu Gln Leu Gln Gly Gly Ser Tyr Lys Lys Ile Gly Tyr
   420 425 430 435
   TAC GAC AGC ACC AAG GAT GAT CTT TCC TGG TCC AAA ACG GAC AAG TGG 1580
   Tyr Asp Ser Thr Lys Asp Asp Leu Ser Trp Ser Lys Thr Asp Lys Trp
   440 445 450
   ATT GGA GGG TCT CCC CCA GCT GAC CAG ACC TTG GTC ATC AAG ACA TTC 1628
   Ile Gly Gly Ser Pro Pro Ala Asp Gln Thr Leu Val Ile Lys Thr Phe
   455 460 465
   CGT TTC CTG TCT CAG AAA CTC TTT ATC TCC GTC TCA GTT CTC TCC AGC 1676
   Arg Phe Leu Ser Gln Lys Leu Phe Ile Ser Val Ser Val Leu Ser Ser
   470 475 480
   CTG GGC ATT GTT CTT GCT GTT GTC TGT CTG TCC TTT AAC ATC TAC AAC 1724
   Leu Gly Ile Val Leu Ala Val Val Cys Leu Ser Phe Asn Ile Tyr Asn
   485 490 495
   TCC CAC GTT CGT TAT ATC CAG AAC TCC CAG CCC AAC CTG AAC AAT CTG 1772
   Ser His Val Arg Tyr Ile Gln Asn Ser Gln Pro Asn Leu Asn Asn Leu
   500 505 510 515
   ACT GCT GTG GGC TGC TCA CTG GCA CTG GCT GCT GTC TTC CCT CTC GGG 1820
   Thr Ala Val Gly Cys Ser Leu Ala Leu Ala Ala Val Phe Pro Leu Gly
   520 525 530
   CTG GAT GGT TAC CAC ATA GGG AGA AGC CAG TTC CCG TTT GTC TGC CAG 1868
   Leu Asp Gly Tyr His Ile Gly Arg Ser Gln Phe Pro Phe Val Cys Gln
   535 540 545
   GCC CGC CTT TGG CTC TTG GGC TTG GGC TTT AGT CTG GGC TAT GGC TCT      1916
   Ala Arg Leu Trp Leu Leu Gly Leu Gly Phe Ser Leu Gly Tyr Gly Ser
           550                 555                 560
   ATG TTC ACC AAG ATC TGG TGG GTC CAC ACA GTC TTC ACG AAG AAG GAG      1964
   Met Phe Thr Lys Ile Trp Trp Val His Thr Val Phe Thr Lys Lys Glu
       565                 570                 575
   GAG AAG AAG GAG TGG AGG AAG ACC CTA GAG CCC TGG AAA CTC TAT GCC      2012
   Glu Lys Lys Glu Trp Arg Lys Thr Leu Glu Pro Trp Lys Leu Tyr Ala
   580                 585                 590                 595
   ACT GTG GGC CTG CTG GTG GGC ATG GAT GTC CTG ACT CTT GCC ATC TGG      2060
   Thr Val Gly Leu Leu Val Gly Met Asp Val Leu Thr Leu Ala Ile Trp
                   600                 605                 610
   CAG ATT GTG GAC CCC TTG CAC CGA ACC ATT GAG ACT TTT GCC AAG GAG      2108
   Gln Ile Val Asp Pro Leu His Arg Thr Ile Glu Thr Phe Ala Lys Glu
               615                 620                 625
   GAA CCA AAG GAA GAC ATC GAT GTC TCC ATT CTG CCC CAG TTG GAG CAC      2156
   Glu Pro Lys Glu Asp Ile Asp Val Ser Ile Leu Pro Gln Leu Glu His
           630                 635                 640
   TGC AGC TCC AAG AAG ATG AAT ACG TGG CTT GGC ATT TTC TAT GGT TAC      2204
   Cys Ser Ser Lys Lys Met Asn Thr Trp Leu Gly Ile Phe Tyr Gly Tyr
       645                 650                 655
   AAG GGG CTG CTG CTG CTG CTG GGA ATC TTT CTT GCT TAC GAA ACC AAG      2252
   Lys Gly Leu Leu Leu Leu Leu Gly Ile Phe Leu Ala Tyr Glu Thr Lys
   660                 665                 670                 675
   AGC GTG TCC ACT GAA AAG ATC AAT GAC CAC AGG GCC GTG GGC ATG GCT      2300
   Ser Val Ser Thr Glu Lys Ile Asn Asp His Arg Ala Val Gly Met Ala
                   680                 685                 690
   ATC TAC AAT GTC GCG GTC CTG TGT CTC ATC ACT GCT CCT GTG ACC ATG      2348
   Ile Tyr Asn Val Ala Val Leu Cys Leu Ile Thr Ala Pro Val Thr Met
               695                     700                     705
   ATC CTT TCC AGT CAG CAG GAC GCA GCC TTT GCC TTT GCC TCT CTG GCC      2396
   Ile Leu Ser Ser Gln Gln Asp Ala Ala Phe Ala Phe Ala Ser Leu Ala
           710                     715                     720
   ATC GTG TTC TCT TCC TAC ATC ACT CTG GTT GTG CTC TTT GTG CCC AAG      2444
   Ile Val Phe Ser Ser Tyr Ile Thr Leu Val Val Leu Phe Val Pro Lys
       725                     730                     735
   ATG CGC AGG CTG ATC ACC CGA GGG GAA TGG CAG TCT GAA ACG CAG GAC      2492
   Met Arg Arg Leu Ile Thr Arg Gly Glu Trp Gln Ser Glu Thr Gln Asp
   740                     745                     750                     755
   ACC ATG AAA ACA GGA TCA TCC ACC AAC AAC AAC GAG GAA GAG AAG TCC      2540
   Thr Met Lys Thr Gly Ser Ser Thr Asn Asn Asn Glu Glu Glu Lys Ser
                   760                     765                     770
   CGA CTG TTG GAG AAG GAA AAC CGA GAA CTG GAA AAG ATC ATC GCT GAG      2588
   Arg Leu Leu Glu Lys Glu Asn Arg Glu Leu Glu Lys Ile Ile Ala Glu
               775                     780                     785
   AAA GAG GAG CGC GTC TCT GAA CTG CGC CAT CAG CTC CAG TCT CGG CAG      2636
   Lys Glu Glu Arg Val Ser Glu Leu Arg His Gln Leu Gln Ser Arg Gln
           790                     795                     800
   CAA CTC CGC TCA CGG CGC CAC CCC CCA ACA CCC CCA GAT CCC TCT GGG      2684
   Gln Leu Arg Ser Arg Arg His Pro Pro Thr Pro Pro Asp Pro Ser Gly
       805                     810                     815
   GGC CTT CCC AGG GGA CCC TCT GAG CCC CCT GAC CGG CTT AGC TGT GAT      2732
   Gly Leu Pro Arg Gly Pro Ser Glu Pro Pro Asp Arg Leu Ser Cys Asp
   820                     825                     830                     835
   GGG AGT CGA GTA CAT TTG CTT TAC AAG TGAGGGGGCA TGGAGAAGGA 2779
   Gly Ser Arg Val His Leu Leu Tyr Lys
   840
   TCTCCCTGAA TCTCAATAAA GCAGTGAACA GTAAACTTTC CAGCACACTG GCGGCCGC 2837
(2) INFORMATION FOR SEQ ID NO: 6:
   (i) SEQUENCE CHARACTERISTICS:
      (A) LENGTH: 844 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Gly Pro Gly Gly Pro Cys Thr Pro Val Gly Trp Pro Leu Pro Leu
   1 5 10 15
   Leu Leu Val Met Ala Ala Gly Val Ala Pro Val Trp Ala Ser His Ser
   20 25 30
   Pro His Leu Pro Arg Pro His Pro Arg Val Pro Pro His Pro Ser Ser
   35 40 45
   Glu Arg Arg Ala Val Tyr Ile Gly Ala Leu Phe Pro Met Ser Gly Gly
   50 55 60
   Trp Pro Gly Gly Gln Ala Cys Gln Pro Ala Val Glu Met Ala Leu Glu
   65 70 75 80
   Asp Val Asn Ser Arg Arg Asp Ile Leu Pro Asp Tyr Glu Leu Lys Leu
   85 90 95
   Ile His His Asp Ser Lys Cys Asp Pro Gly Gln Ala Thr Lys Tyr Leu
   100 105 110
   Tyr Glu Leu Leu Tyr Asn Asp Pro Ile Lys Ile Ile Leu Met Pro Gly
   115 120 125
   Cys Ser Ser Val Ser Thr Leu Val Ala Glu Ala Ala Arg Met Trp Asn
   130 135 140
   Leu Ile Val Leu Ser Tyr Gly Ser Ser Ser Pro Ala Leu Ser Asn Arg
   145 150 155 160
   Gln Arg Phe Pro Thr Phe Phe Arg Thr His Pro Ser Ala Thr Leu His
   165 170 175
   Asn Pro Thr Arg Val Lys Leu Phe Glu Lys Trp Gly Trp Lys Lys Ile
   180 185 190
   Ala Thr Ile Gln Gln Thr Thr Glu Val Phe Thr Ser Thr Leu Asp Asp
   195 200 205
   Leu Glu Glu Arg Val Lys Glu Ala Gly Ile Glu Ile Thr Phe Arg Gln
   210 215 220
   Ser Phe Phe Ser Asp Pro Ala Val Pro Val Lys Asn Leu Lys Arg Gln
   225 230 235 240
   Asp Ala Arg Ile Ile Val Gly Leu Phe Tyr Glu Thr Glu Ala Arg Lys
   245 250 255
   Val Phe Cys Glu Val Tyr Lys Glu Arg Leu Phe Gly Lys Lys Tyr Val
   260 265 270
   Trp Phe Leu Ile Gly Trp Tyr Ala Asp Asn Trp Phe Lys Thr Tyr Asp
   275 280 285
   Pro Ser Ile Asn Cys Thr Val Glu Glu Met Thr Glu Ala Val Glu Gly
   290 295 300
   His Ile Thr Thr Glu Ile Val Met Leu Asn Pro Ala Asn Thr Arg Ser
   305 310 315 320
   Ile Ser Asn Met Thr Ser Gln Glu Phe Val Glu Lys Leu Thr Lys Arg
   325 330 335
   Leu Lys Arg His Pro Glu Glu Thr Gly Gly Phe Gln Glu Ala Pro Leu
   340 345 350
   Ala Tyr Asp Ala Ile Trp Ala Leu Ala Leu Ala Leu Asn Lys Thr Ser
   355 360 365
   Gly Gly Gly Gly Arg Ser Gly Val Arg Leu Glu Asp Phe Asn Tyr Asn
   370 375 380
   Asn Gln Thr Ile Thr Asp Gln Ile Tyr Arg Ala Met Asn Ser Ser Ser
   385 390 395 400
   Phe Glu Gly Val Ser Gly His Val Val Phe Asp Ala Ser Gly Ser Arg
   405 410 415
   Met Ala Trp Thr Leu Ile Glu Gln Leu Gln Gly Gly Ser Tyr Lys Lys
   420 425 430
   Ile Gly Tyr Tyr Asp Ser Thr Lys Asp Asp Leu Ser Trp Ser Lys Thr
   435 440 445
   Asp Lys Trp Ile Gly Gly Ser Pro Pro Ala Asp Gln Thr Leu Val Ile
   450 455 460
   Lys Thr Phe Arg Phe Leu Ser Gln Lys Leu Phe Ile Ser Val Ser Val
   465 470 475 480
   Leu Ser Ser Leu Gly Ile Val Leu Ala Val Val Cys Leu Ser Phe Asn
   485 490 495
   Ile Tyr Asn Ser His Val Arg Tyr Ile Gln Asn Ser Gln Pro Asn Leu
   500 505 510
   Asn Asn Leu Thr Ala Val Gly Cys Ser Leu Ala Leu Ala Ala Val Phe
   515 520 525
   Pro Leu Gly Leu Asp Gly Tyr His Ile Gly Arg Ser Gln Phe Pro Phe
   530 535 540
   Val Cys Gln Ala Arg Leu Trp Leu Leu Gly Leu Gly Phe Ser Leu Gly
   545 550 555 560
   Tyr Gly Ser Met Phe Thr Lys Ile Trp Trp Val His Thr Val Phe Thr
   565 570 575
   Lys Lys Glu Glu Lys Lys Glu Trp Arg Lys Thr Leu Glu Pro Trp Lys
   580 585 590
   Leu Tyr Ala Thr Val Gly Leu Leu Val Gly Met Asp Val Leu Thr Leu
   595 600 605
   Ala Ile Trp Gln Ile Val Asp Pro Leu His Arg Thr Ile Glu Thr Phe
   610 615 620
   Ala Lys Glu Glu Pro Lys Glu Asp Ile Asp Val Ser Ile Leu Pro Gln
   625 630 635 640
   Leu Glu His Cys Ser Ser Lys Lys Met Asn Thr Trp Leu Gly Ile Phe
   645 650 655
   Tyr Gly Tyr Lys Gly Leu Leu Leu Leu Leu Gly Ile Phe Leu Ala Tyr
   660 665 670
   Glu Thr Lys Ser Val Ser Thr Glu Lys Ile Asn Asp His Arg Ala Val
   675 680 685
   Gly Met Ala Ile Tyr Asn Val Ala Val Leu Cys Leu Ile Thr Ala Pro
   690 695 700
   Val Thr Met Ile Leu Ser Ser Gln Gln Asp Ala Ala Phe Ala Phe Ala
   705 710 715 720
   Ser Leu Ala Ile Val Phe Ser Ser Tyr Ile Thr Leu Val Val Leu Phe
   725 730 735
   Val Pro Lys Met Arg Arg Leu Ile Thr Arg Gly Glu Trp Gln Ser Glu
   740 745 750
   Thr Gln Asp Thr Met Lys Thr Gly Ser Ser Thr Asn Asn Asn Glu Glu
   755 760 765
   Glu Lys Ser Arg Leu Leu Glu Lys Glu Asn Arg Glu Leu Glu Lys Ile
   770 775 780
   Ile Ala Glu Lys Glu Glu Arg Val Ser Glu Leu Arg His Gln Leu Gln
   785 790 795 800
   Ser Arg Gln Gln Leu Arg Ser Arg Arg His Pro Pro Thr Pro Pro Asp
   805 810 815
   Pro Ser Gly Gly Leu Pro Arg Gly Pro Ser Glu Pro Pro Asp Arg Leu
   820 825 830
   Ser Cys Asp Gly Ser Arg Val His Leu Leu Tyr Lys
   835 840
(2) INFORMATION FOR SEQ ID NO: 7:
   (i) SEQUENCE CHARACTERISTICS:
      (A) LENGTH: 2924 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: GABABR1b human
   (ix) FEATURE:
      (A) NAME/KEY: CDS
      (B) LOCATION:169..2700
   (ix) FEATURE:
      (A) NAME/KEY: mat_peptide
      (B) LOCATION:169..2700
   (xi) SEQUENCE DESCRIPTION: SEQ ID NO: 7:
   GGCCGTAGGA AGCCAACCTT CCCTGCTTCT CCGGGGCCCT CGCCCCCTCC TCCCCACAAA 60
   ATCAGGGATG GAGGCGCCTC CCCGGCACCC TCTTAGCAGC CCTCCCCAGG AAAAGTGTCC 120
   CCCCTGAGCT CCTAACGCTC CCCAACAGCT ACCCCTGCCC CCCACGCC ATG GGG CCC      177
                                                   Met Gly Pro
                                                    1
   GGG GCC CCT TTT GCC CGG GTG GGG TGG CCA CTG CCG CTT CTG GTT GTG      225
   Gly Ala Pro Phe Ala Arg Val Gly Trp Pro Leu Pro Leu Leu Val Val
        5                  10                  15
   ATG GCG GCA GGG GTG GCT CCG GTG TGG GCC TCC CAC TCC CCC CAT CTC      273
   Met Ala Ala Gly Val Ala Pro Val Trp Ala Ser His Ser Pro His Leu
    20                  25                  30                  35
   CCG CGG CCT CAC TCG CGG GTC CCC CCG CAC CCC TCC TCA GAA CGG CGC      321
   Pro Arg Pro His Ser Arg Val Pro Pro His Pro Ser Ser Glu Arg Arg
                    40                  45                  50
   GCA GTG TAC ATC GGG GCA CTG TTT CCC ATG AGC GGG GGC TGG CCA GGG      369
   Ala Val Tyr Ile Gly Ala Leu Phe Pro Met Ser Gly Gly Trp Pro Gly
                55                  60                  65
   GGC CAG GCC TGC CAG CCC GCG GTG GAG ATG GCG CTG GAG GAC GTG AAT      417
   Gly Gln Ala Cys Gln Pro Ala Val Glu Met Ala Leu Glu Asp Val Asn
            70                  75                  80
   AGC CGC AGG GAC ATC CTG CCG GAC TAT GAG CTC AAG CTC ATC CAC CAC      465
   Ser Arg Arg Asp Ile Leu Pro Asp Tyr Glu Leu Lys Leu Ile His His
        85                  90                  95
   GAC AGC AAG TGT GAT CCA GGC CAA GCC ACC AAG TAC CTA TAT GAG CTG      513
   Asp Ser Lys Cys Asp Pro Gly Gln Ala Thr Lys Tyr Leu Tyr Glu Leu
   100                 105                 110                 115
   CTC TAC AAC GAC CCT ATC AAG ATC ATC CTT ATG CCT GGC TGC AGC TCT      561
   Leu Tyr Asn Asp Pro Ile Lys Ile Ile Leu Met Pro Gly Cys Ser Ser
                   120                 125                 130
   GTC TCC ACG CTG GTG GCT GAG GCT GCT AGG ATG TGG AAC CTC ATT GTG        609
   Val Ser Thr Leu Val Ala Glu Ala Ala Arg Met Trp Asn Leu Ile Val
               135                 140                 145
   CTT TCC TAT GGC TCC AGC TCA CCA GCC CTG TCA AAC CGG CAG CGT TTC        657
   Leu Ser Tyr Gly Ser Ser Ser Pro Ala Leu Ser Asn Arg Gln Arg Phe
           150                 155                 160
   CCC ACT TTC TTC CGA ACG CAC CCA TCA GCC ACA CTC CAC AAC CCT ACC        705
   Pro Thr Phe Phe Arg Thr His Pro Ser Ala Thr Leu His Asn Pro Thr
       165                 170                 175
   CGC GTG AAA CTC TTT GAA AAG TGG GGC TGG AAG AAG ATT GCT ACC ATC        753
   Arg Val Lys Leu Phe Glu Lys Trp Gly Trp Lys Lys Ile Ala Thr Ile
   180                 185                 190                 195
   CAG CAG ACC ACT GAG GTC TTC ACT TCG ACT CTG GAC GAC CTG GAG GAA        801
   Gln Gln Thr Thr Glu Val Phe Thr Ser Thr Leu Asp Asp Leu Glu Glu
                   200                 205                 210
   CGA GTG AAG GAG GCT GGA ATT GAG ATT ACT TTC CGC CAG AGT TTC TTC        849
   Arg Val Lys Glu Ala Gly Ile Glu Ile Thr Phe Arg Gln Ser Phe Phe
               215                 220                 225
   TCA GAT CCA GCT GTG CCC GTC AAA AAC CTG AAG CGC CAG GAT GCC CGA        897
   Ser Asp Pro Ala Val Pro Val Lys Asn Leu Lys Arg Gln Asp Ala Arg
           230                 235                 240
   ATC ATC GTG GGA CTT TTC TAT GAG ACT GAA GCC CGG AAA GTT TTT TGT        945
   Ile Ile Val Gly Leu Phe Tyr Glu Thr Glu Ala Arg Lys Val Phe Cys
       245                 250                 255
   GAG GTG TAC AAG GAG CGT CTC TTT GGG AAG AAG TAC GTC TGG TTC CTC        993
   Glu Val Tyr Lys Glu Arg Leu Phe Gly Lys Lys Tyr Val Trp Phe Leu
   260                 265                 270                 275
   ATT GGG TGG TAT GCT GAC AAT TGG TTC AAG ATC TAC GAC CCT TCT ATC 1041
   Ile Gly Trp Tyr Ala Asp Asn Trp Phe Lys Ile Tyr Asp Pro Ser Ile
   280 285 290
   AAC TGC ACA GTG GAT GAG ATG ACT GAG GCG GTG GAG GGC CAC ATC ACA 1089
   Asn Cys Thr Val Asp Glu Met Thr Glu Ala Val Glu Gly His Ile Thr
   295 300 305
   ACT GAG ATT GTC ATG CTG AAT CCT GCC AAT ACC CGC AGC ATT TCC AAC 1137
   Thr Glu Ile Val Met Leu Asn Pro Ala Asn Thr Arg Ser Ile Ser Asn
   310 315 320
   ATG ACA TCC CAG GAA TTT GTG GAG AAA CTA ACC AAG CGA CTG AAA AGA 1185
   Met Thr Ser Gln Glu Phe Val Glu Lys Leu Thr Lys Arg Leu Lys Arg
   325 330 335
   CAC CCT GAG GAG ACA GGA GGC TTC CAG GAG GCA CCG CTG GCC TAT GAT 1233
   His Pro Glu Glu Thr Gly Gly Phe Gln Glu Ala Pro Leu Ala Tyr Asp
   340 345 350 355
   GCC ATC TGG GCC TTG GCA CTG GCC CTG AAC AAG ACA TCT GGA GGA GGC 1281
   Ala Ile Trp Ala Leu Ala Leu Ala Leu Asn Lys Thr Ser Gly Gly Gly
   360 365 370
   GGC CGT TCT GGT GTG CGC CTG GAG GAC TTC AAC TAC AAC AAC CAG ACC 1329
   Gly Arg Ser Gly Val Arg Leu Glu Asp Phe Asn Tyr Asn Asn Gln Thr
   375 380 385
   ATT ACC GAC CAA ATC TAC CGG GCA ATG AAC TCT TCG TCC TTT GAG GGT 1377
   Ile Thr Asp Gln Ile Tyr Arg Ala Met Asn Ser Ser Ser Phe Glu Gly
   390 395 400
   GTC TCT GGC CAT GTG GTG TTT GAT GCC AGC GGC TCT CGG ATG GCA TGG 1425
   Val Ser Gly His Val Val Phe Asp Ala Ser Gly Ser Arg Met Ala Trp
   405 410 415
   ACG CTT ATC GAG CAG CTT CAG GGT GGC AGC TAC AAG AAG ATT GGC TAC      1473
   Thr Leu Ile Glu Gln Leu Gln Gly Gly Ser Tyr Lys Lys Ile Gly Tyr
   420                 425                 430                 435
   TAT GAC AGC ACC AAG GAT GAT CTT TCC TGG TCC AAA ACA GAT AAA TGG      1521
   Tyr Asp Ser Thr Lys Asp Asp Leu Ser Trp Ser Lys Thr Asp Lys Trp
                   440                 445                 450
   ATT GGA GGG TCC CCC CCA GCT GAC CAG ACC CTG GTC ATC AAG ACA TTC      1569
   Ile Gly Gly Ser Pro Pro Ala Asp Gln Thr Leu Val Ile Lys Thr Phe
               455                 460                 465
   CGC TTC CTG TCA CAG AAA CTC TTT ATC TCC GTC TCA GTT CTC TCC AGC      1617
   Arg Phe Leu Ser Gln Lys Leu Phe Ile Ser Val Ser Val Leu Ser Ser
           470                 475                 480
   CTG GGC ATT GTC CTA GCT GTT GTC TGT CTG TCC TTT AAC ATC TAC AAC      1665
   Leu Gly Ile Val Leu Ala Val Val Cys Leu Ser Phe Asn Ile Tyr Asn
       485                 490                 495
   TCA CAT GTC CGT TAT ATC CAG AAC TCA CAG CCC AAC CTG AAC AAC CTG      1713
   Ser His Val Arg Tyr Ile Gln Asn Ser Gln Pro Asn Leu Asn Asn Leu
   500                 505                 510                 515
   ACT GCT GTG GGC TGC TCA CTG GCT TTA GCT GCT GTC TTC CCC CTG GGG      1761
   Thr Ala Val Gly Cys Ser Leu Ala Leu Ala Ala Val Phe Pro Leu Gly
                   520                 525                 530
   CTC GAT GGT TAC CAC ATT GGG AGG AAC CAG TTT CCT TTC GTC TGC CAG      1809
   Leu Asp Gly Tyr His Ile Gly Arg Asn Gln Phe Pro Phe Val Cys Gln
               535                 540                 545
   GCC CGC CTC TGG CTC CTG GGC CTG GGC TTT AGT CTG GGC TAC GGT TCC      1857
   Ala Arg Leu Trp Leu Leu Gly Leu Gly Phe Ser Leu Gly Tyr Gly Ser
           550                 555                 560
   ATG TTC ACC AAG ATT TGG TGG GTC CAC ACG GTC TTC ACA AAG AAG GAA      1905
   Met Phe Thr Lys Ile Trp Trp Val His Thr Val Phe Thr Lys Lys Glu
       565                 570                 575
   GAA AAG AAG GAG TGG AGG AAG ACT CTG GAA CCC TGG AAG CTG TAT GCC      1953
   Glu Lys Lys Glu Trp Arg Lys Thr Leu Glu Pro Trp Lys Leu Tyr Ala
   580                 585                 590                 595
   ACA GTG GGC CTG CTG GTG GGC ATG GAT GTC CTC ACT CTC GCC ATC TGG      2001
   Thr Val Gly Leu Leu Val Gly Met Asp Val Leu Thr Leu Ala Ile Trp
                   600                 605                 610
   CAG ATC GTG GAC CCT CTG CAC CGG ACC ATT GAG ACA TTT GCC AAG GAG      2049
   Gln Ile Val Asp Pro Leu His Arg Thr Ile Glu Thr Phe Ala Lys Glu
               615                 620                 625
   GAA CCT AAG GAA GAT ATT GAC GTC TCT ATT CTG CCC CAG CTG GAG CAT      2097
   Glu Pro Lys Glu Asp Ile Asp Val Ser Ile Leu Pro Gln Leu Glu His
           630                 635                 640
   TGC AGC TCC AGG AAG ATG AAT ACA TGG CTT GGC ATT TTC TAT GGT TAC      2145
   Cys Ser Ser Arg Lys Met Asn Thr Trp Leu Gly Ile Phe Tyr Gly Tyr
       645                 650                 655
   AAG GGG CTG CTG CTG CTG CTG GGA ATC TTC CTT GCT TAT GAG ACC AAG      2193
   Lys Gly Leu Leu Leu Leu Leu Gly Ile Phe Leu Ala Tyr Glu Thr Lys
   660                 665                 670                 675
   AGT GTG TCC ACT GAG AAG ATC AAT GAT CAC CGG GCT GTG GGC ATG GCT      2241
   Ser Val Ser Thr Glu Lys Ile Asn Asp His Arg Ala Val Gly Met Ala
                   680                 685                 690
   ATC TAC AAT GTG GCA GTC CTG TGC CTC ATC ACT GCT CCT GTC ACC ATG      2289
   Ile Tyr Asn Val Ala Val Leu Cys Leu Ile Thr Ala Pro Val Thr Met
               695                 700                 705
   ATT CTG TCC AGC CAG CAG GAT GCA GCC TTT GCC TTT GCC TCT CTT GCC        2337
   Ile Leu Ser Ser Gln Gln Asp Ala Ala Phe Ala Phe Ala Ser Leu Ala
               710                 715                 720
   ATA GTT TTC TCC TCC TAT ATC ACT CTT GTT GTG CTC TTT GTG CCC AAG        2385
   Ile Val Phe Ser Ser Tyr Ile Thr Leu Val Val Leu Phe Val Pro Lys
           725                 730                 735
   ATG CGC AGG CTG ATC ACC CGA GGG GAA TGG CAG TCG GAG GCG CAG GAC        2433
   Met Arg Arg Leu Ile Thr Arg Gly Glu Trp Gln Ser Glu Ala Gln Asp
   740                 745                 750                 755
   ACC ATG AAG ACA GGG TCA TCG ACC AAC AAC AAC GAG GAG GAG AAG TCC        2481
   Thr Met Lys Thr Gly Ser Ser Thr Asn Asn Asn Glu Glu Glu Lys Ser
                   760                 765                 770
   CGG CTG TTG GAG AAG GAG AAC CGT GAA CTG GAA AAG ATC ATT GCT GAG        2529
   Arg Leu Leu Glu Lys Glu Asn Arg Glu Leu Glu Lys Ile Ile Ala Glu
               775                 780                 785
   AAA GAG GAG CGT GTC TCT GAA CTG CGC CAT CAA CTC CAG TCT CGG CAG        2577
   Lys Glu Glu Arg Val Ser Glu Leu Arg His Gln Leu Gln Ser Arg Gln
               790                 795                 800
   CAG CTC CGC TCC CGG CGC CAC CCA CCG ACA CCC CCA GAA CCC TCT GGG        2625
   Gln Leu Arg Ser Arg Arg His Pro Pro Thr Pro Pro Glu Pro Ser Gly
           805                 810                 815
   GGC CTG CCC AGG GGA CCC CCT GAG CCC CCC GAC CGG CTT AGC TGT GAT        2673
   Gly Leu Pro Arg Gly Pro Pro Glu Pro Pro Asp Arg Leu Ser Cys Asp
   820                 825                 830                 835
   GGG AGT CGA GTG CAT TTG CTT TAT AAG TGAGGGTAGG GTGAGGGAGG              2720
   Gly Ser Arg Val His Leu Leu Tyr Lys
                   840
   ACAGGCCAGT AGGGGGAGGG AAAGGGAGAG GGGAAGGGCA GGGGACTCAG GAAGCAGGGG       2780
   GTCCCCATCC CCAGCTGGGA AGAACATGCT ATCCAATCTC ATCTCTTGTA AATACATGTC 2840
   CCCCTGTGAG TTCTGGGCTG ATTTGGGTCT CTCATACCTC TGGGAAACAG ACCTTTTTCT 2900
   CTCTTACTGC TTCATGTAAT TTTG 2924
(2) INFORMATION FOR SEQ ID NO: 8:
   (i) SEQUENCE CHARACTERISTICS:
      (A) LENGTH: 844 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Met Gly Pro Gly Ala Pro Phe Ala Arg Val Gly Trp Pro Leu Pro Leu
    1               5                  10                  15
   Leu Val Val Met Ala Ala Gly Val Ala Pro Val Trp Ala Ser His Ser
               20                  25                  30
   Pro His Leu Pro Arg Pro His Ser Arg Val Pro Pro His Pro Ser Ser
           35                  40                  45
   Glu Arg Arg Ala Val Tyr Ile Gly Ala Leu Phe Pro Met Ser Gly Gly
       50                  55                  60
   Trp Pro Gly Gly Gln Ala Cys Gln Pro Ala Val Glu Met Ala Leu Glu
   65                  70                  75                  80
   Asp Val Asn Ser Arg Arg Asp Ile Leu Pro Asp Tyr Glu Leu Lys Leu
                   85                  90                  95
   Ile His His Asp Ser Lys Cys Asp Pro Gly Gln Ala Thr Lys Tyr Leu
   100 105 110
   Tyr Glu Leu Leu Tyr Asn Asp Pro Ile Lys Ile Ile Leu Met Pro Gly
   115 120 125
   Cys Ser Ser Val Ser Thr Leu Val Ala Glu Ala Ala Arg Met Trp Asn
   130 135 140
   Leu Ile Val Leu Ser Tyr Gly Ser Ser Ser Pro Ala Leu Ser Asn Arg
   145 150 155 160
   Gln Arg Phe Pro Thr Phe Phe Arg Thr His Pro Ser Ala Thr Leu His
   165 170 175
   Asn Pro Thr Arg Val Lys Leu Phe Glu Lys Trp Gly Trp Lys Lys Ile
   180 185 190
   Ala Thr Ile Gln Gln Thr Thr Glu Val Phe Thr Ser Thr Leu Asp Asp
   195 200 205
   Leu Glu Glu Arg Val Lys Glu Ala Gly Ile Glu Ile Thr Phe Arg Gln
   210 215 220
   Ser Phe Phe Ser Asp Pro Ala Val Pro Val Lys Asn Leu Lys Arg Gln
   225 230 235 240
   Asp Ala Arg Ile Ile Val Gly Leu Phe Tyr Glu Thr Glu Ala Arg Lys
   245 250 255
   Val Phe Cys Glu Val Tyr Lys Glu Arg Leu Phe Gly Lys Lys Tyr Val
   260 265 270
   Trp Phe Leu Ile Gly Trp Tyr Ala Asp Asn Trp Phe Lys Ile Tyr Asp
   275 280 285
   Pro Ser Ile Asn Cys Thr Val Asp Glu Met Thr Glu Ala Val Glu Gly
   290 295 300
   His Ile Thr Thr Glu Ile Val Met Leu Asn Pro Ala Asn Thr Arg Ser
   305 310 315 320
   Ile Ser Asn Met Thr Ser Gln Glu Phe Val Glu Lys Leu Thr Lys Arg
   325 330 335
   Leu Lys Arg His Pro Glu Glu Thr Gly Gly Phe Gln Glu Ala Pro Leu
   340 345 350
   Ala Tyr Asp Ala Ile Trp Ala Leu Ala Leu Ala Leu Asn Lys Thr Ser
   355 360 365
   Gly Gly Gly Gly Arg Ser Gly Val Arg Leu Glu Asp Phe Asn Tyr Asn
   370 375 380
   Asn Gln Thr Ile Thr Asp Gln Ile Tyr Arg Ala Met Asn Ser Ser Ser
   385 390 395 400
   Phe Glu Gly Val Ser Gly His Val Val Phe Asp Ala Ser Gly Ser Arg
   405 410 415
   Met Ala Trp Thr Leu Ile Glu Gln Leu Gln Gly Gly Ser Tyr Lys Lys
   420 425 430
   Ile Gly Tyr Tyr Asp Ser Thr Lys Asp Asp Leu Ser Trp Ser Lys Thr
   435 440 445
   Asp Lys Trp Ile Gly Gly Ser Pro Pro Ala Asp Gln Thr Leu Val Ile
   450 455 460
   Lys Thr Phe Arg Phe Leu Ser Gln Lys Leu Phe Ile Ser Val Ser Val
   465 470 475 480
   Leu Ser Ser Leu Gly Ile Val Leu Ala Val Val Cys Leu Ser Phe Asn
   485 490 495
   Ile Tyr Asn Ser His Val Arg Tyr Ile Gln Asn Ser Gln Pro Asn Leu
   500 505 510
   Asn Asn Leu Thr Ala Val Gly Cys Ser Leu Ala Leu Ala Ala Val Phe
   515 520 525
   Pro Leu Gly Leu Asp Gly Tyr His Ile Gly Arg Asn Gln Phe Pro Phe
   530 535 540
   Val Cys Gln Ala Arg Leu Trp Leu Leu Gly Leu Gly Phe Ser Leu Gly
   545 550 555 560
   Tyr Gly Ser Met Phe Thr Lys Ile Trp Trp Val His Thr Val Phe Thr
   565 570 575
   Lys Lys Glu Glu Lys Lys Glu Trp Arg Lys Thr Leu Glu Pro Trp Lys
   580 585 590
   Leu Tyr Ala Thr Val Gly Leu Leu Val Gly Met Asp Val Leu Thr Leu
   595 600 605
   Ala Ile Trp Gln Ile Val Asp Pro Leu His Arg Thr Ile Glu Thr Phe
   610 615 620
   Ala Lys Glu Glu Pro Lys Glu Asp Ile Asp Val Ser Ile Leu Pro Gln
   625 630 635 640
   Leu Glu His Cys Ser Ser Arg Lys Met Asn Thr Trp Leu Gly Ile Phe
   645 650 655
   Tyr Gly Tyr Lys Gly Leu Leu Leu Leu Leu Gly Ile Phe Leu Ala Tyr
   660 665 670
   Glu Thr Lys Ser Val Ser Thr Glu Lys Ile Asn Asp His Arg Ala Val
   675 680 685
   Gly Met Ala Ile Tyr Asn Val Ala Val Leu Cys Leu Ile Thr Ala Pro
   690 695 700
   Val Thr Met Ile Leu Ser Ser Gln Gln Asp Ala Ala Phe Ala Phe Ala
   705 710 715 720
   Ser Leu Ala Ile Val Phe Ser Ser Tyr Ile Thr Leu Val Val Leu Phe
   725 730 735
   Val Pro Lys Met Arg Arg Leu Ile Thr Arg Gly Glu Trp Gln Ser Glu
   740 745 750
   Ala Gln Asp Thr Met Lys Thr Gly Ser Ser Thr Asn Asn Asn Glu Glu
   755 760 765
   Glu Lys Ser Arg Leu Leu Glu Lys Glu Asn Arg Glu Leu Glu Lys Ile
   770 775 780
   Ile Ala Glu Lys Glu Glu Arg Val Ser Glu Leu Arg His Gln Leu Gln
   785 790 795 800
   Ser Arg Gln Gln Leu Arg Ser Arg Arg His Pro Pro Thr Pro Pro Glu
   805 810 815
   Pro Ser Gly Gly Leu Pro Arg Gly Pro Pro Glu Pro Pro Asp Arg Leu
   820 825 830
   Ser Cys Asp Gly Ser Arg Val His Leu Leu Tyr Lys
   835 840

## Description

The present invention relates to nucleic acids encoding proteins of the GABA_{B} receptor family, as well as proteins encoded thereby and the use of such proteins for the development of pharmacological agents.

Gamma-aminobutyric acid (GABA) is the major inhibitory neurotransmitter found in the brain and peripheral nervous system. Receptors for GABA have been divided into two subfamilies, the GABA_{A} and GABA_{B} receptors. Of these, GABA_{A} receptors are involved in fast inhibitory signal transmission, whilst GABA_{B} receptors appear to be involved in modulation of neurotransmission. Pre-synaptic GABA_{B} receptors influence the release of neurotransmitters and neuropeptides such as GABA, glutamate, noradrenaline, dopamine, 5-hydroxytryptamine, substance P, cholecystokinin and somatostatin, while post-synaptic GABA_{B} receptors are coupled to potassium channels via G proteins and mediate late inhibitory post-synaptic potentials (IPSPs). The effect of the activation of both subtypes of the GABA_{B} receptor is to modulate synaptic transmission.

GABA_{B} receptors are located throughout the central and peripheral nervous systems (see Ong and Kerr, Life Sciences, (1990) 46, 1489-1501; Bowery *et al.*, Drug Res. (1992) 42(1), 2a, 215-223), and are thus involved in the regulation of a wide variety of neurally-controlled physiological responses, from memory and learning to muscle contraction. This makes the GABA_{B} receptor a target for pharmaceutical agents intended to treat central and peripheral neural disorders, and indeed a variety of GABA_{B} agonists and antagonists are known and have been proposed for use in therapy (Bittiger *et al.*, in *GABA: Receptors, Transporters and Metabolism,* Tanaka, C., and Bowery, N.G. (Eds). Birkhäuser Verlag Basel/Switzerland (1996), 297-305; Bittiger *et al*., Trends Pharmacol. Sci., 14, 391-394, 1993; Froestl *et al.,* J. Med. Chem., 38, 3297-3312, 1995; Froestl *et al*., Ibid., 3313-3331). For example, in Alzheimer's disease and other dementias such as Age Associated Memory Impairment and Multi Infarct Dementia, loss of cognitive function is associated with reduced levels of a number of neurotransmitters in the brain. In particular, a deficit in L-glutamate is expected to cause a major loss of cognitive functions, since L-glutamate appears to be crucially involved in the processes underlying memory formation and learning. GABA acts directly at many synapses to reduce the release of L-glutamate by acting on GABA_{B} heteroreceptors. Thus, GABA_{B} receptor antagonists are indicated for the treatment of dementias, and indeed have been shown to improve cognitive functions in animal studies. In addition, GABA_{B} receptor antagonists are expected to be active in psychiatric and neurological disorders such as depression, anxiety and epilepsy (Bittiger *et al*., 1993, 1996, Op. Cit.; Froestl *et al.,* 1995, Op. Cit.). GABA_{B} receptor agonists are known as antispastic agents, and in peripheral nervous system applications, agonists are expected to be beneficial in bronchial inflammation, asthma and coughing (Bertrand *et al*., Am. J. Resp. Crit. Care Med. 149, A900, 1994). GABA is moreover associated with activity in the intestine, the cardiovascular system, gall and urinary bladders, and a variety of other tissues (Ong and Kerr, Op. Cit.).

GABA action in each of the above cases is known to be mediated by GABA_{B} receptors, making the receptors targets for pharmacological agents designed to treat a number of disorders.

Despite the advanced state of molecular biology and protein purification technology, and the evident desirability of obtaining a purified GABA_{B} receptor for pharmacological studies, the GABA_{B} receptor previously has not been cloned or purified to homogeneity. A previous report of its partial purification (Nakayasu *et al*., J. Biol. Chem., 268, 8658-8664, 1993) appears to have been inaccurate, relating to an 80 kDa protein, which we now know to be too small. In order to be able to clone the GABA_{B} receptor, we have developed a number of GABA_{B} receptor-specific ligands. By expression cloning using one such highly selective GABA_{B} receptor ligand labelled to high specific radioactivity, we have now cloned different GABA_{B} receptors from rat and human sources, sequenced them and expressed the respective recombinant receptors in mammalian cell culture.

### Summary of the Invention

The present invention provides isolated GABA_{B} receptors and GABA_{B} receptor proteins, as well as nucleic acids which encode such proteins. The proteins and nucleic acids of the invention share significant homology with the GABA_{B} receptors and the DNAs encoding them as specifically disclosed herein. In particular, there are provided two GABA_{B} receptor proteins designated GABA_{B}R1a and GABA_{B}R1b which are distinct variants of GABA_{B} isolated from rat. The respective cDNA and derived amino acid sequences are set forth in SEQ ID Nos. 1, 2, and 5, 6, respectively. Furthermore, there are provided two human GABA_{B} receptor clones termed GABA_{B}R1a/b (representing a partial receptor clone) and GABA_{B}R1b (representing a full-length receptor clone) isolated from human sources.
The respective cDNA and derived amino acid sequences are set forth in SEQ ID Nos. 3, 4, and 7, 8, respectively.

The GABA_{B} receptors and GABA_{B} receptor proteins of the invention show specific binding to one or more of the selective GABA_{B} receptor antagonists of Formula I and Formula II: 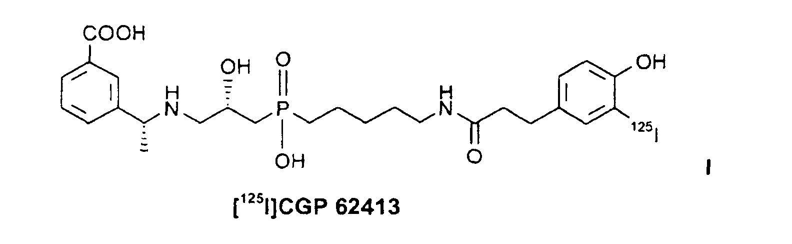 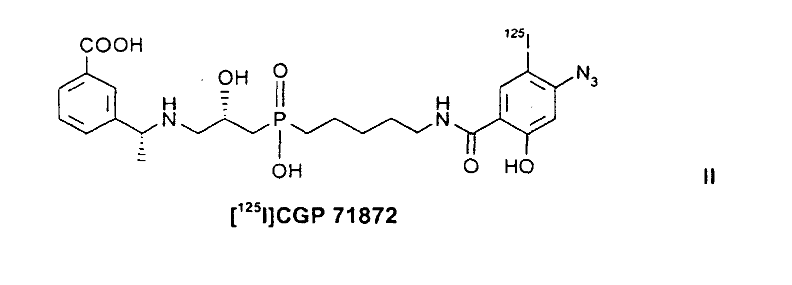 Moreover, binding of the these selective GABA_{B} receptor antagonists may be competed with other selective GABA_{B} receptor agonists or antagonists, such as the compound of Formula III and Formula IV:

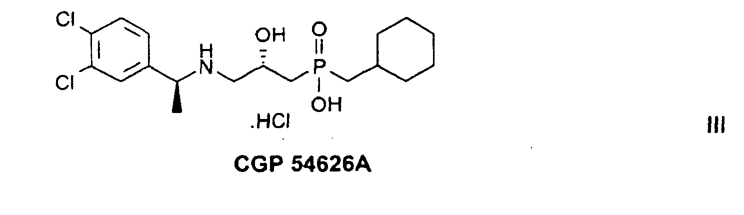

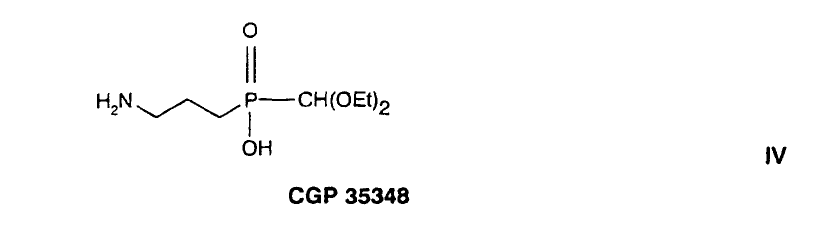

The invention moreover provides methods for isolating other members of the GABA_{B} receptor family using DNA cloning technology and probes derived from the sequences provided herein, as well as novel members of the GABA_{B} receptor family isolated by such methods.

Furthermore, the invention relates to the use of GABA_{B} receptors and GABA_{B} receptor proteins and cells transformed with a gene encoding such a GABA_{B} receptor or receptor protein in a method for identifying and characterising compounds which modulate the activity of the GABA_{B} receptor(s), such as GABA_{B} receptor agonists and antagonists, which may be useful as pharmacological agents for the treatment of disorders associated with the central and peripheral nervous systems. In particular, GABA_{B} receptor antagonists can e.g. be useful as cognition enhancers, nootropics, antidepressants and anxiolytics for the treatment of cerebral insufficiency, depression, anxiety, epilepsy of the petit mal type, schizophrenia and myopia, whereas GABA_{B} receptor agonists can e.g. be useful in the treatment of disorders such as spasticity, trigeminal neuralgia, asthma, cough, emesis, ulcers, urinary incontinence and cocain addiction.

### Brief Description of the Figures

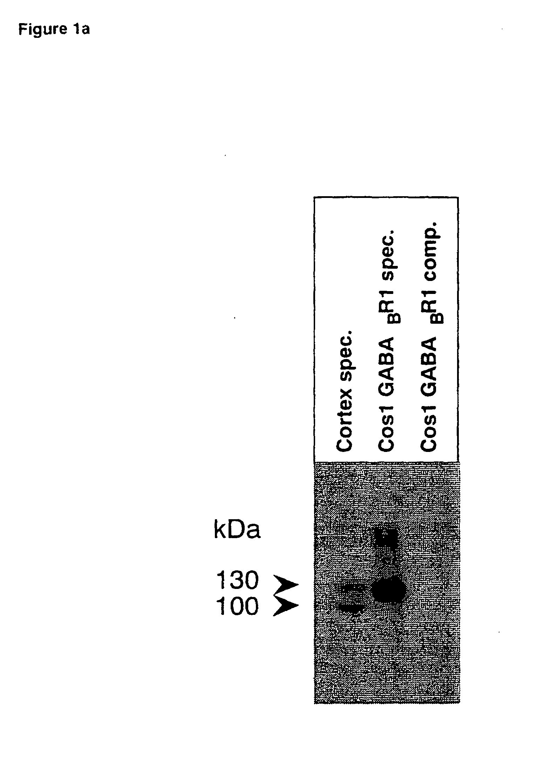

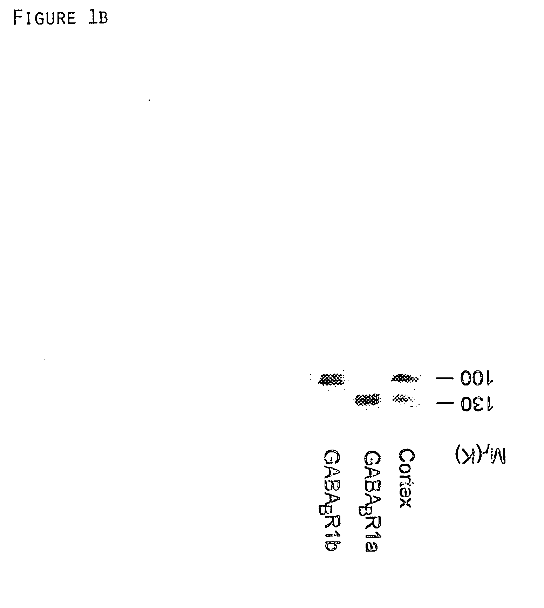

Figure 1 a depicts the expression of the recombinant GABA_{B}R1a receptor in COS1 cells. Membranes from rat cortex membranes (lane 1) and COS1 cells transfected with the GABA_{B}R1a rat-cDNA (lanes 2 and 3) are labelled with the photoaffinity ligand [¹²⁵I]CGP 71872. Autoradiography of a 6% SDS gel with 25µg protein loaded per lane is shown. Lanes 1 and 2: Specific binding with 0.6nM [¹²⁵I]CGP 71872. Lane 3: Control experiment where specific binding with 0.6nM [¹²⁵I]CGP 71872 is competed with 1µM of unlabeled CGP 54626A (an antagonist specific for GABA_{B} receptors). The apparent molecular weight of native and recombinant GABA_{B} receptors are estimated from gel mobilities relative to those of SDS-PAGE standards (BioRad). Figure 1 b additionally shows the results for COS1 cells transfected with the GABA_{B}R1b rat-cDNA (lane 3).

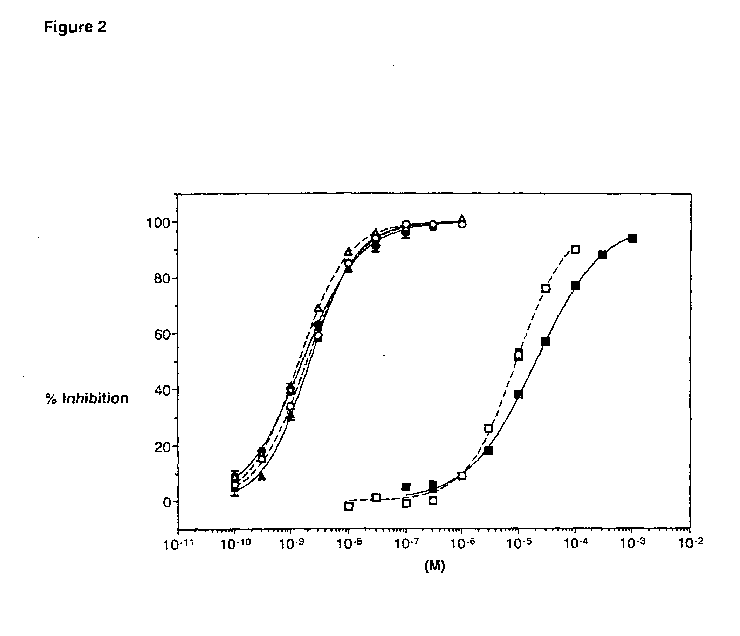

Figure 2 shows the inhibition of [¹²⁵I]CGP 64213 binding to GABA_{B} receptors in membranes from rat cerebral cortex (open symbols) and recombinant GABA_{B}R1a receptors in membranes from COS 1 cells (closed symbols) by the GABA_{B} receptor antagonists CGP 54626A (●), CGP 64213 (▲) and CGP 35348 (■).

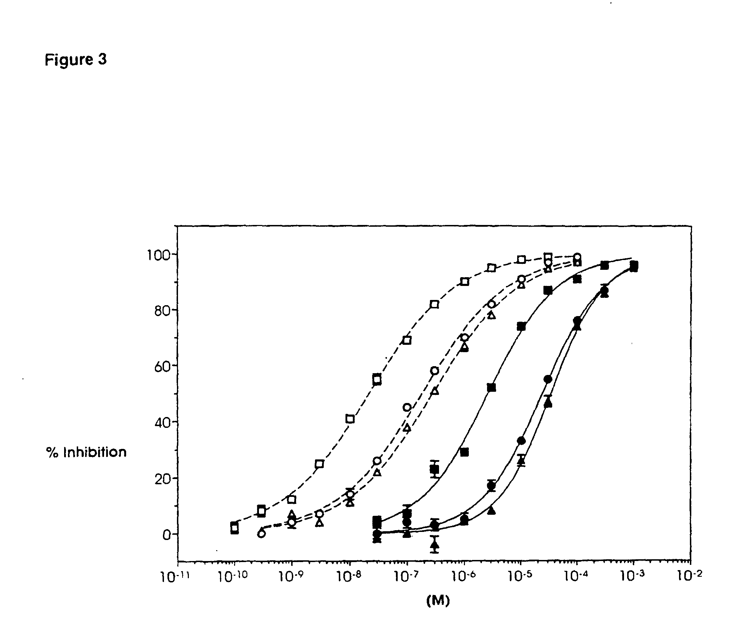

Figure 3 shows the inhibition of [¹²⁵I]CGP 64213 binding to GABA_{B} receptors in membranes from rat cerebral cortex (open symbols) and recombinant GABA_{B}R1a receptors in membranes from COS 1 cells (closed symbols) by the GABA_{B} receptor agonists GABA (●), L-baclofen (▲) and APPA 3-(aminopropyl-phosphinic acid )(■).

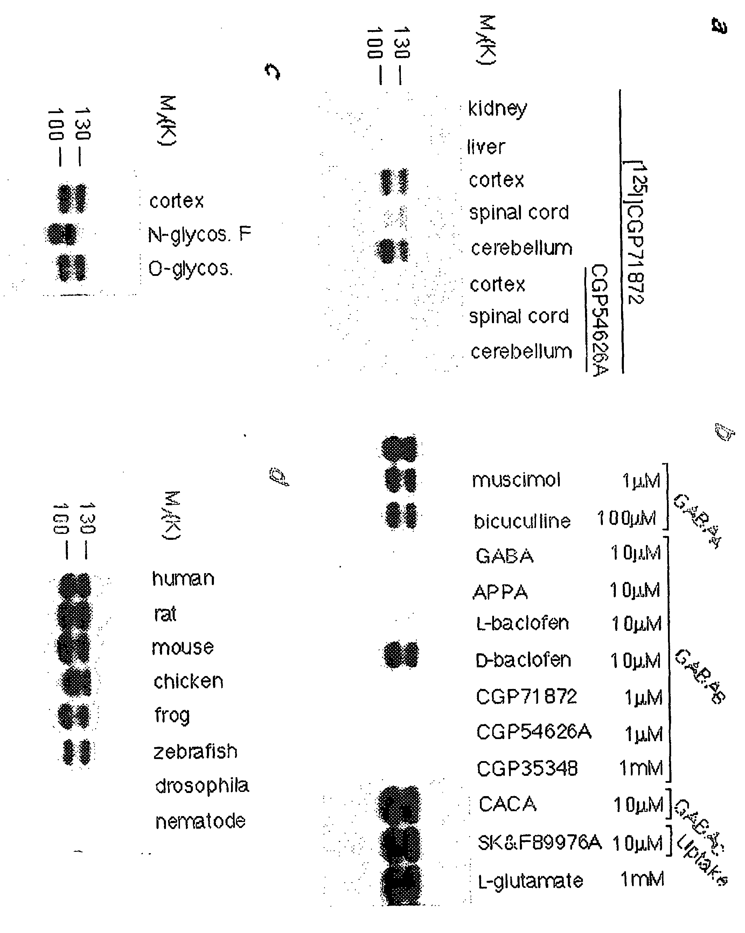

Figure 4 shows photoaffinity crosslinking of GABA_{B} receptor proteins. Cell membranes of the tissues indicated are photoaffinity-labelled with [¹²⁵I]CGP71872 and subjected to SDS-PAGE and autoradiography. *a, b,* Selectivity of the photoaffinity ligand [¹²⁵I]CGP71872. *a*, Differential distribution of GABA_{B} receptor variants of 130K and 100K in tissues of the nervous system. [¹²⁵I]CGP71872 binding is inhibited by addition of 1 µM of CGP54626A, a selective GABA_{B} receptor antagonist. *b*, Competition of [¹²⁵I]CGP71872 labelling by different ligands. Incubation of membrane extracts with the photoaffinity ligand is carried out in the presence of competitor substances at the concentrations indicated, *c*, GABA_{B} receptors are N-glycosylated. Photoaffinity-labelled rat cortex cell membranes are incubated with 0.4 units N-glycosidase F or 0.6 milliunits O-glycosidase (Boehringer Mannheim). *d*, Photolabelling of GABA_{B} receptors from different species. Brain tissues from the species indicated are labelled as described hereinbelow. In the case of *Drosophila melanogaster* and *Haemonchus concortus* whole animals are analysed.

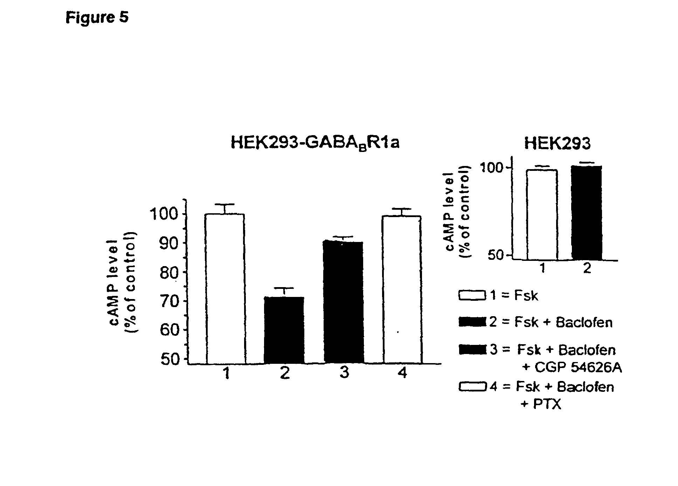

Figure 5 shows the results of assays concerning pharmacological properties of native and recombinant GABA_{B} receptors. GABA_{B}R1a mediates inhibition of adenylate cyclase. HEK293 cells stably expressing GABA_{B}R1a are treated with 20 µM forskolin (Fsk) to stimulate cAMP formation (100%). Fsk induced cAMP accumulation is reduced significantly (2*P* < 0.001; Dunnett's *t*-test) upon simultaneous addition of 300 µM L-baclofen. The effect of L-baclofen is antagonised in the presence of 10 µM CGP54626A. Preincubation of the cells with 10 ng/ml pertussis toxin (PTX) for 15-20 h completely abolishes the effect of L-baclofen. No L-baclofen response is observed in non-transfected HEK293 cells (insert). Bars represent mean values +S.E.M. of at least three independent experiments performed in quadruplicate.

### Detailed Description of the Invention

The invention relates to isolated GABA_{B} receptors and GABA_{B} receptor proteins, nucleic acids coding therefore and various applications thereof. Before the present invention, the GABA_{B} receptor has not been available in purified form, but only as crude membrane preparations. For the first time, the present invention enables the production of different but related GABA_{B} receptors in a substantially purified form, by means of recombinant DNA technology. In general, it is expected that such proteins in glycosylated form will have an observed molecular weight of between 100 and 130 kDa, whereas the unglycosylated forms will have an observed molecular weight of between 90 and 110 kDa, respectively.

GABA_{B} receptors according to the invention are G-protein coupled modulators of neurotransmitter activity which are responsive to GABA. They may be defined by binding to labelled ligands which are selective for GABA_{B} receptors, in particular [¹²⁵I]CGP 62413 and [¹²⁵I]CGP 71872. Functional studies are moreover possible in which a recombinant GABA_{B} receptor is expressed in cell systems containing G-proteins and effectors such as ionic channels which can be activated by GABA and GABA_{B} receptor agonists.

Proteins according to the invention may be defined electrophysiologically in transgenic or knockout animals, for example in terms of their responsiveness in assays for the GABA_{B} receptor(s) which are known in the art, such as the measurement of late IPSPs (inhibitory post-synaptic potentials), paired-pulse inhibition or (-)-baclofen-induced depression of field EPSPs (excitatory post-synaptic potentials). GABA_{B} receptors are responsible for the observation of IPSPs as a result of indirect coupling to potassium channels in neurons, so established agonists and antagonists of GABA_{B} receptors may be used to determine the presence of GABA_{A} receptors in neuronal preparations by assaying for their effect on IPSPs.

Advantageously, however, GABA_{B} receptor proteins according to the invention are assessed by their susceptibility to CGP64213 and CGP71872 as measured by paired-pulse widening of field EPSPs. Both said compounds abolish paired-pulse widening normally associated with GABA_{B} receptors, since they are effective GABA_{B} autoreceptor antagonists. Preferably, therefore, the activation of GABA_{B} receptor proteins according to the invention is specifically inhibited by CGP64213 and CGP71872. Examples of specific inhibition by these compounds are set out hereinbelow.

As used herein, the term "GABA_{B} receptor(s)" refers to the proteins whose sequences are substantially those set forth in SEQ ID Nos. 2 and 8, while the term "GABA_{B} receptor proteins" includes derivatives and variants such as e.g. splice variants thereof which are related structurally and/or functionally to the GABA_{B} receptor(s). Preferred GABA_{B} receptor proteins according to the invention are e.g. those set forth in SEQ ID Nos. 4 and 6, and share at least one common structural determinant with the GABA_{B} receptors having the amino acid sequences set forth in SEQ ID Nos. 2 and 8, respectively. "Common structural determinant" means that the derivative in question comprises at least one structural feature of the GABA_{B} receptors set out in SEQ ID Nos. 2 and 8. Structural features includes possession of an epitope or antigenic site that is capable of cross-reacting with antibodies raised against a naturally occurring or denatured GABA_{B} receptor polypeptide or fragment thereof, possession of amino acid sequence identity with the GABA_{B} receptor(s) and features having common a structure/function relationship. Thus the GABA_{B} receptor proteins as provided by the present invention include amino acid mutants, glycosylation variants and other covalent derivatives of the GABA_{B} receptor(s) which retain the physiological and/or physical properties of the GABA_{B} receptor(s).

Further included within the scope of the term "GABA_{B} receptor proteins" are naturally occurring variants of the GABA_{B} receptor(s) found within a particular species, preferably a mammal. Such a variant may be encoded by a related gene of the same gene family, by an allelic variant of a particular gene, or represent an alternative splicing variant of the GABA_{B} receptor gene. Variants according to the invention have the same basic function as the GABA_{B} receptor(s), but may possess divergent characteristics consistent with their nature as variants. For example, it is expected that the GABA_{B} receptors are members of a family of GABA_{B} receptor proteins, the isolation and characterisation of which is enabled for the first time by the present invention. Different members of the GABA_{B} receptor family may be expected to have different activity profiles, possibly according to differences in their tissue-specific localisation and role in modulating neuronal signalling.

Moreover, the present invention enables the isolation and characterisation of further GABA_{B} receptors, GABA_{B} receptor proteins and GABA_{B} receptor protein-encoding nucleic acids from any species, including man. The provision of sequence data enables the person skilled in the art to apply standard hybridisation methodology, as is known in the art and set out by way of example hereinbelow, to isolate any desired GABA_{B} receptor-encoding nucleic acid.

The invention further comprises derivatives of the GABA_{B} receptor(s), which retain at least one common structural determinant of the GABA_{B} receptor(s). For example, derivatives include molecules wherein the protein of the invention is covalently modified by substitution, chemical, enzymatic, or other appropriate means with a moiety other than a naturally occurring amino acid. Such a moiety may be a detectable moiety such as an enzyme or a radioisotope.

Derivatives which retain common structural determinants can be fragments of the GABA_{B} receptor(s). Fragments of the GABA_{B} receptor(s) comprise individual domains thereof, as well as smaller polypeptides derived from the domains. Preferably, smaller polypeptides derived from the GABA_{B} receptor(s) according to the invention define a single feature which is characteristic of the GABA_{B} receptor(s). Fragments may in theory be almost any size, as long as they retain one feature of the GABA_{B} receptor(s). Preferably, fragments will be between 5 and 600 amino acids in length. Longer fragments are regarded as truncations of the full-length GABA_{B} receptor(s) and generally encompassed by the term "GABA_{B} receptor(s)". Preferably, said fragments retain the functional activity of the GABA_{B} receptor(s). Such fragments may be produced by persons skilled in the art, using conventional techniques, by removing amino acid residues from the GABA_{B} receptor proteins of the invention which are not essential for a particular functional aspect of the GABA_{B} receptor proteins. Determination of functional aspects of a GABA_{B} receptor protein may be made employing pharmacological or electrophysiological assays as herein described, and particularly by assays which monitor the ability of the GABA_{B} receptor protein to bind GABA or a GABA mimic, or to couple to G proteins.

Derivatives of the GABA_{B} receptor(s) also comprise mutants thereof, which may contain amino acid deletions, additions or substitutions, subject to the requirement to maintain at least one feature characteristic of the GABA_{B} receptor(s). Thus, conservative amino acid substitutions may be made substantially without altering the nature of the GABA_{B} receptor(s). Substitutions and further deletions may moreover be made to the fragments of GABA_{B} receptor proteins comprised by the invention. GABA_{B} receptor protein mutants may be produced from a DNA encoding a GABA_{B} receptor protein which has been subjected to *in vitro* mutagenesis resulting e.g. in an addition, exchange and/or deletion of one or more amino acid encoding triplets. For example, substitutional, deletional or insertional variants of the GABA_{B} receptor(s) can be prepared by recombinant methods and screened for immuno- or physiological crossreactivity with the native forms of the GABA_{B} receptor(s).

Mutations may be performed by any method known to those of skill in the art. Preferred, however, is site-directed mutagenesis of a nucleic acid sequence encoding the polypeptide of interest. A number of methods for site-directed mutagenesis are known in the art, from methods employing single-stranded phage such as M13 to PCR-based techniques (see "PCR Protocols: A guide to methods and applications", M.A. Innis, D.H. Gelfand, J.J. Sninsky, T.J. White (eds.). Academic Press, New York, 1990). Preferably, the commercially available Altered Site II Mutagenesis System (Promega) may be employed, according to the directions given by the manufacturer.

The fragments, mutants and other derivatives of the GABA_{B} receptor(s) preferably retain substantial homology with the GABA_{B} receptor(s). As used herein, "homology" means that the two entities share sufficient characteristics for the skilled person to determine that they are similar in origin and function. Preferably, homology is used to refer to sequence identity. Thus, the derivatives of the GABA_{B} receptor(s) preferably retain substantial sequence identity with the sequences set forth in SEQ ID Nos. 2 and 8, respectively.

"Substantial homology", where homology indicates sequence identity, means more than 30% sequence identity, preferably more than 65% sequence identity and most preferably a sequence identity of 80% or more.

According to a further aspect of the present invention, there are provided nucleic acids encoding GABA_{B} receptors and GABA_{B} receptor proteins (SEQ ID Nos. 1,7, and 3,5, respectively). In addition to being useful for the production of recombinant GABA_{B} receptors and receptor proteins, these nucleic acids are also useful as probes, thus readily enabling those skilled in the art to identify and/or isolate nucleic acids encoding further members of the GABA_{B} receptor family and variants thereof as set forth hereinbefore.

In another aspect, the invention provides nucleic acid sequences that are complementary to, or are capable of hybridising to, nucleic acid sequences encoding the GABA_{B} receptors or receptor proteins. Preferably, such nucleic acids are capable of hybridising under high or moderate stringency, as defined hereinbelow.

Furthermore, nucleic acids according to the invention are useful in a method determining the presence of a GABA_{B} receptor- or receptor protein-specific nucleic acid, said method comprising hybridising the DNA (or RNA) encoding (or complementary to) the GABA_{B} receptor or receptor protein to test sample nucleic acid and determining the presence of the GABA_{B} receptor- or receptor protein-specific nucleic acid.

The invention also provides a method for amplifying a nucleic acid test sample comprising priming a nucleic acid polymerase (chain) reaction with nucleic acid (DNA or RNA) encoding a GABA_{B} receptor or receptor protein, or a nucleic acid complementary thereto.

Isolated GABA_{B} receptor- or receptor protein-specific nucleic acids include nucleic acids that are free from at least one contaminant nucleic acid with which they are ordinarily associated in the natural source of GABA_{B} receptor- or receptor protein-specific nucleic acids or in crude nucleic acid preparations, such as DNA libraries and the like. Isolated nucleic acids thus are present in other than in the form or setting in which they are found in nature. However, isolated GABA_{B} receptor and receptor protein encoding nucleic acids include GABA_{B} receptor- and receptor protein-specific nucleic acids in ordinarily GABA_{B} receptor- or receptor protein-expressing cells, where the nucleic acids are in a chromosomal location different from that of natural cells or are otherwise flanked by different DNA sequences than those found in nature.

In accordance with the present invention, there are provided isolated nucleic acids, e.g. DNAs or RNAs, encoding GABA_{B} receptors and GABA_{B} receptor proteins, particularly mammalian GABA_{B} receptors and receptor proteins, such as e.g. human and rat GABA_{B} receptors and receptor proteins, or fragments thereof. In particular, the invention provides DNA molecules encoding human and rat GABA_{B} receptors or receptor proteins, or fragments thereof. By definition, such a DNA comprises a coding single stranded DNA, a double stranded DNA consisting of said coding DNA and complementary DNA thereto, or this complementary (single stranded) DNA itself. Exemplary nucleic acids encoding GABA_{B} receptors and GABA_{B} receptor proteins are represented in SEQ ID Nos. 1, 7, and 3, 5, respectively.

The preferred sequences encoding GABA_{B} receptors and receptor proteins are those having substantially the same nucleotide sequence as the coding sequences in SEQ ID Nos. 1, 3, 5 and 7, with the nucleic acids having the same sequence as the coding sequences in SEQ ID Nos. 1, 3, 5 and 7 being most preferred. As used herein, nucleotide sequences which are substantially the same share at least about 90 % identity. However, in the case of splice variants having e.g. an additional exon sequence homology may be lower.

The nucleic acids of the invention, whether used as probes or otherwise, are preferably substantially homologous to the sequences encoding the GABA_{B} receptors or receptor proteins as shown in SEQ ID No. 1, 3, 5 and 7. The terms "substantially" and "homologous" are used as hereinbefore defined with reference to the GABA_{B} receptor polypeptides.

Preferably, nucleic acids according to the invention are fragments of the GABA_{B} receptor- or receptor protein-encoding sequences, or derivatives thereof as hereinbefore defined in relation to polypeptides. Fragments of the nucleic acid sequences of a few nucleotides in length, preferably 5 to 150 nucleotides in length, are especially useful as probes.

Exemplary nucleic acids can alternatively be characterised as those nucleotide sequences which encode a GABA_{B} receptor or receptor protein as hereinbefore defined and hybridise to the DNA sequences set forth in SEQ ID Nos. 1, 3, 5 and/or 7, or a selected fragment of said DNA sequences. Preferred are such sequences encoding GABA_{B} receptors or receptor proteins which hybridise under high-stringency conditions to the sequences of SEQ ID Nos. 1, 3, 5 and/or 7.

Stringency of hybridisation refers to conditions under which polynucleic acids hybrids are stable. Such conditions are evident to those of ordinary skill in the field. As known to those of skill in the art, the stability of hybrids is reflected in the melting temperature (Tₘ) of the hybrid which decreases approximately by 1 to 1.5°C with every 1% decrease in sequence homology. In general, the stability of a hybrid is a function of sodium ion concentration and temperature. Typically, the hybridisation reaction is performed under conditions of higher stringency, followed by washes of varying stringency.

As used herein, high stringency refers to conditions that permit hybridisation of only those nucleic acid sequences that form stable hybrids in 1 M Na⁺ at 65-68 °C. High stringency conditions can be provided, for example, by hybridisation in an aqueous solution containing 6x SSC, 5x Denhardt's, 1 % SDS (sodium dodecyl sulphate), 0.1 sodium pyrophosphate and 0.1 mg/ml denatured salmon sperm DNA as non specific competitor. Following hybridisation, high stringency washing may be done in several steps, with a final wash (about 30 min) at the hybridisation temperature in 0.2 - 0.1 x SSC, 0.1 % SDS.

Moderate stringency refers to conditions equivalent to hybridisation in the above described solution but at about 60-62°C. In that case the final wash is performed at the hybridisation temperature in 1x SSC, 0.1 % SDS.

Low stringency refers to conditions equivalent to hybridisation in the above described solution at about 50-52°C. In that case, the final wash is performed at the hybridisation temperature in 2x SSC, 0.1 % SDS.

It is understood that these conditions may be adapted and duplicated using a variety of buffers, e.g. formamide-based buffers, and temperatures. Denhardt's solution and SSC are well known to those of skill in the art as are other suitable hybridisation buffers (see, e.g. Sambrook, *et al*., eds. (1989) Molecular Cloning: A Laboratory Manual, Cold Spring Harbor Laboratory Press, New York or Ausubel, *et al*., eds. (1990) Current Protocols in Molecular Biology, John Wiley & Sons, Inc.). In particular, the skilled person will understand that the stringency of hybridisation conditions may be varied by altering a number of parameters, primarily the salt concentration and the temperature, and that the conditions obtained are a result of the combined effect of all such parameters. Optimal hybridisation conditions have to be determined empirically, as the length and the GC content of the probe also play a role.

Nucleic acids according to the invention may moreover be designed to have quite different sequences from those of nucleic acids encoding GABA_{B} receptors or receptor proteins as derived from natural sources, through exploitation of the degeneracy of the amino acid code. In most cases, a plurality of nucleotide triplets may be used to encode a given amino acid. Thus, an almost limitless number of nucleic acids which encode identical GABA_{B} receptors or receptor proteins may be designed. Those which most differ from the sequence of the naturally occurring nucleic acid may be so different as to be unable to hybridise therewith. The invention thus specifically encompasses any nucleic acid which encodes a GABA_{B} receptor or GABA_{B} receptor protein as hereinbefore defined. Preferred are all nucleic acids which encode the sequences of the GABA_{B} receptors and receptor proteins set forth in SEQ ID Nos. 2, 8, and 4, 6, respectively.

Given the guidance provided herein, the nucleic acids of the invention are obtainable according to methods well known in the art. For example, a DNA of the invention is obtainable by chemical synthesis, using polymerase chain reaction (PCR) or by screening a genomic library or a suitable cDNA library prepared from a source believed to possess GABA_{B} receptor or receptor protein and to express it at a detectable level.

Chemical methods for synthesis of a nucleic acid of interest are known in the art and include triester, phosphite, phosphoramidite and H-phosphonate methods, PCR and other autoprimer methods as well as oligonucleotide synthesis on solid supports. These methods may be used if the entire nucleic acid sequence of the nucleic acid is known, or the sequence of the nucleic acid complementary to the coding strand is available. Alternatively, if the target amino acid sequence is known, one may infer potential nucleic acid sequences using known and preferred coding residues for each amino acid residue.

An alternative means to isolate a gene encoding GABA_{B} receptor or receptor protein is to use PCR technology as described e.g. in section 14 of Sambrook et al., 1989. This method requires the use of oligonucleotide probes that will hybridise to a GABA_{B} receptor- or receptor protein-specific nucleic acid.

A nucleic acid encoding a GABA_{B} receptor or receptor protein can be isolated by screening suitable cDNA or genomic libraries under suitable hybridisation conditions with a probe, i.e. a nucleic acid disclosed herein including oligonucleotides derivable from the sequences set forth in SEQ ID Nos. 1, 3, 5 and 7. Suitable libraries are commercially available or can be prepared e.g. from cell lines, tissue samples, and the like. Libraries are screened with probes or analytical tools designed to identify the gene of interest or the protein encoded by it. For cDNA expression libraries suitable means include monoclonal or polyclonal antibodies that recognise and specifically bind to the GABA_{B} receptor or GABA_{B} receptor protein; oligonucleotides of about 20 to 80 bases in length that encode known or suspected GABA_{B} receptor- or receptor protein-specific cDNA from the same or different species; and/or complementary or homologous cDNAs or fragments thereof that encode the same or a hybridising gene. Appropriate probes for screening genomic DNA libraries include, but are not limited to oligonucleotides, cDNAs or fragments thereof that encode the same or hybridising DNA; and/or homologous genomic DNAs or fragments thereof.

Particularly preferred screening techniques include the hybridisation of a test sample of DNA (cDNA or genomic library) with a GABA_{B} receptor- or receptor protein-specific cDNA (SEQ ID Nos. 1, 3, 5, 7) under suitable hybridisation conditions. Either the full length or fragments of the GABA_{B} receptor- or receptor protein-specific cDNA can be used as probes. Such screening is initially carried out under low-stringency conditions. Low stringency conditions are as hereinbefore defined, but may be varied by adjusting the temperature and ionic strength of the hybridisation solution. For example, suitable conditions comprise hybridisation at a temperature between 40°C and 60°C in 0.5M NaH₂PO₄ pH 7.2, 7% sodium dodecyl sulphate (SDS), 1% bovine serum albumin, 1 mM EDTA, with a washing step at 50°C or less in 2 x standard saline citrate (SSC, 20 x SSC contains 3M sodium chloride, 0.3M sodium citrate, pH 7.0), 0.1% SDS. Preferably, hybridisation conditions will be selected which allow the identification of nucleotide sequences having at least 40% sequence homology with respect to the probe. Similar homology screening techniques useful for the identification and isolation of additional cDNAs and genes of the GABA_{B}-receptor gene family are described in United States Patent Number 5,202,257, incorporated herein by reference.

After low stringency hybridisation has been used to identify cDNA or genomic clones having a substantial similarity with the probe sequence, these clones are then subjected to moderate to high stringency conditions in order to identify those clones having particularly high level of homology with respect to the probe sequence. Further examples of high stringency conditions comprise a hybridisation temperature of about 60°C to 68°C using the above mentioned hybridisation solution. Washing conditions comprise 0.5 x SSC, 0.1% SDS or less at a temperature of about 65°C or less.

In view of the identification of GABA_{B} receptor- and receptor protein-specific cDNAs according to the invention, the compiled sequence information can be used to design a set of degenerate oligonucleotide primer sequences from the regions most conserved among members of the gene family. A mixture of such oligonucleotide primers can be used in the polymerase chain reaction (PCR) to amplify cDNAs or genomic segments from genes rotated to the already isolated GABA_{B} receptor- and receptor protein-specific cDNAs.

Subsequently, these segments can serve as probes for identifying further full-length cDNA clones using high stringency hybridisation conditions. Alternatively, antibodies derived against the GABA_{B} receptors or GABA_{B} receptor protein provided by the present invention can be used to purify and sequence related GABA_{B} receptors and receptor proteins also recognised by the antibodies.

Screening of libraries in order to isolate nucleic acids according to the invention may moreover be performed by expression screening. Such methodology is known to those skilled in the art, for example as set out in Sambrook *et al*. (Op. Cit.), but essentially comprises the incorporation of nucleic acid clones into expression vectors which are then screened using a ligand specific for the desired protein product. GABA_{B} receptor- or receptor protein-specific ligands may be antibodies, as described hereinbelow, or specific GABA antagonists or agonists. Especially preferred are compounds such as CGP 64213, described hereinbelow.

As used herein, an oligonucleotide probe is preferably a single-stranded DNA or RNA that has a sequence of nucleotides that includes between 10 and 50, preferably between 15 and 30 and most preferably at least about 20 contiguous bases that are the same as (or the complement of) an equivalent or greater number of contiguous bases as set forth in SEQ ID Nos. 1, 3, 5 and 7. The nucleic acid sequences selected as probes should be of sufficient length and sufficiently unambiguous so that false positive results are minimised. The nucleotide sequences are usually based on conserved or highly homologous nucleotide sequences or regions of the GABA_{B} receptor or receptor protein. The nucleic acids used as probes may be degenerate at one or more positions. The use of degenerate oligonucleotides may be of particular importance where a library is screened from a species in which preferential codon usage in that species is not known.

Preferred regions from which to construct probes include 5' and/or 3' coding sequences, sequences predicted to encode ligand binding sites, and the like. For example, either the full-length cDNA clones disclosed herein or fragments thereof can be used as probes. Preferably, nucleic acid probes of the invention are labelled with suitable label means for ready detection upon hybridisation. For example, a suitable label means is a radiolabel. The preferred method of labelling a DNA fragment is by incorporating α³²P dATP with the Klenow fragment of DNA polymerase in a random priming reaction, as is well known in the art. Oligonucleotides are usually end-labelled with γ³²P-labelled ATP and polynucleotide kinase. However, other methods (e.g. non-radioactive) may also be used to label the fragment or oligonucleotide, including e.g. enzyme labelling, fluorescent labelling with suitable fluorophores and biotinylation.

After screening the library, for example with a portion of DNA including substantially the entire GABA_{B} receptor- or receptor protein-encoding sequence or a suitable oligonucleotide based on a portion of said DNA, positive clones are identified by detecting a hybridisation signal; the identified clones are characterised by restriction enzyme mapping and/or DNA sequence analysis, and then examined, for example by comparison with the sequences set forth herein, to ascertain whether they include DNA encoding a complete GABA_{B} receptor or receptor protein (i.e., if they include translation initiation and termination codons). If the selected clones are incomplete, they may be used to rescreen the same or a different library to obtain overlapping clones. If the library is genomic, then the overlapping clones may include exons and introns. If the library is a cDNA library, then the overlapping clones will include an open reading frame. In both instances, complete clones may be identified by. comparison with the DNAs and deduced amino acid sequences provided herein.

In order to detect any abnormality of endogenous GABA_{B} receptor or receptor protein, genetic screening may be carried out using the nucleotide sequences of the invention as hybridisation probes. Also, based on the nucleic acid sequences provided herein antisense-type therapeutic agents may be designed. In particular reference thereto, it is to be noted that antisense oligonucleotides are based on oligonucleotide probes as hereinbefore defined, and included within the definition thereof. Such oligonucleotides, especially but not only when intended for use as antisense therapeutic agents, may comprise modifications to the oligonucleotide, for example by incorporation of unnatural nucleotide analogues and modifications to natural oligonucleotides. For example, the oligonucleotides may encompass an altered backbone, for example in the form of a phosphorothioate, modifications such as 2'-O-Methyl modifications, or may be in the form of peptide nucleic acids.

It is envisaged that the nucleic acids of the invention can be readily modified by nucleotide substitution, nucleotide deletion, nucleotide insertion or inversion of a nucleotide stretch, and any combination thereof. Such mutants can be used e.g. to produce a GABA_{B} receptor or receptor protein mutant that has an amino acid sequence differing from the GABA_{B} receptor or receptor protein sequences as disclosed herein or as found in nature. Mutagenesis may be predetermined (site-specific) or random. A mutation which is not a silent mutation must not place sequences out of reading frames and preferably will not create complementary regions that could hybridise to produce secondary mRNA structure such as loops or hairpins.

In still another aspect of the invention, the nucleic acids are DNA molecules and further comprise a replicable vector comprising the nucleic acid encoding the GABA_{B} receptor or receptor protein operably linked to control sequences recognised by a host transformed by the vector. As used herein, vector (or plasmid) refers to discrete elements that are used to introduce heterologous DNA into cells for either expression or replication thereof. Selection and use of such vehicles is a routine matter for the person of ordinary skill in the art and is described, for example, in Sambrook *et al*., (1989) Molecular Cloning: A Laboratory Manual, Second Edition, Cold Spring Harbor Laboratory Press. Many vectors are available, and selection of appropriate vector will depend on the intended use of the vector, i.e. whether it is to be used for DNA amplification or for DNA expression, the size of the DNA to be inserted into the vector, and the host cell to be transformed with the vector. Each vector contains various components depending on its function (amplification of DNA or expression of DNA) and the host cell for which it is compatible. The vector components generally include, but are not limited to, one or more of the following: an origin of replication, one or more marker genes, an enhancer element, a promoter, a transcription termination sequence and a signal sequence.

Advantageously, a eukaryotic expression vector encoding a GABA_{B} receptor or receptor protein will comprise a locus control region (LCR). LCRs are capable of directing high-level integration site independent expression of transgenes integrated into host cell chromatin, which is of importance especially where the GABA_{B} receptor or receptor protein gene is to be expressed in the context of a permanently-transfected eukaryotic cell line in which chromosomal integration of the vector has occurred, in vectors designed for gene therapy applications or in transgenic animals.

Suitable vectors for expression in eukaryotic host cells, including yeast, fungi, insect, plant, animal, human, or nucleated cells from other multicellular organisms, will also contain sequences necessary for the termination of transcription and for stabilising the mRNA. Such sequences are commonly available from the 5' and 3' untranslated regions of eukaryotic or viral DNAs or cDNAs.

Furthermore the invention provides host cells transformed with such a vector and a method of using a nucleic acid encoding a GABA_{B} receptor or receptor protein according to the invention to produce such a GABA_{B} receptor or receptor protein, comprising expressing a GABA_{B} receptor- or receptor protein-specific nucleic acid in a culture of the transformed host cells and, if desired, recovering the GABA_{B} receptor or receptor protein from the host cell culture. In accordance with another embodiment of the present invention, there are provided cells containing the above-described nucleic acids. Such host cells such as prokaryote, yeast and higher eukaryote cells may be used for replicating DNA and producing GABA_{B} receptor or receptor protein. Suitable prokaryotes include eubacteria, such as Gram-negative or Gram-positive organisms, such as E. coli, e.g. E. coli K-12 strains DH5a, MC1061/P3 and HB101, or Bacilli. Further hosts suitable for GABA_{B} receptor protein encoding vectors include eukaryotic microbes such as filamentous fungi or yeast, e.g. Saccharomyces cerevisiae. Higher eukaryotic cells include insect and vertebrate cells, particularly mammalian cells. In recent years propagation of vertebrate cells in culture (tissue culture) has become a routine procedure. Examples of useful mammalian host cell lines are epithelial or fibroblastic cell lines such as Chinese hamster ovary (CHO) cells, COS cells, NIH 3T3 cells, HeLa cells or HEK293 cells. The host cells referred to in this disclosure comprise cells in *in vitro* culture as well as cells that are within a host animal.

DNA may be stably incorporated into cells or may be transiently expressed using methods known in the art, such as those detailed in Sambrook *et al*., Op. Cit., or Ausubel *et al*., (1990) Current Protocols in Molecular Biology, John Wiley & Sons, Inc.

The polypeptides according to the invention can advantageously be expressed in insect cell systems, including whole insects. Insect cell lines suitable for use in the method of the invention include, in principle, any lepidopteran cell which is capable of being transformed with an expression vector and expressing heterologous proteins encoded thereby. In particular, use of the Sf cell lines, such as the *Spodoptera frugiperda* cell line IPBL-SF-21 AE (Vaughn *et al*., (1977) In Vitro, **13**, 213-217) is preferred. The derivative cell line Sf9 is particularly preferred. However, other cell lines, such as *Tricoplusia ni* 368 (Kurstack and Marmorosch, (1976) Invertebrate Tissue Culture Applications in Medicine, Biology and Agriculture. Academic Press, New York, USA) may be employed. These cell lines, as well as other insect cell lines suitable for use in the invention, are commercially available (e.g. from Stratagene, La Jolla, CA, USA).

Expression vectors suitable for use in the invention include all vectors which are capable of expressing foreign proteins in insect cell lines. In general, vectors which are useful in mammalian and other eukaryotic cells are also applicable to insect cell culture. Baculovirus vectors, specifically intended for insect cell culture, are especially preferred and are widely obtainable commercially (e.g. from Invitrogen and Clontech). Other virus vectors capable of infecting insect cells are known, such as Sindbis virus (Hahn *et al*., (1992) PNAS (USA) 89, 2679-2683). The baculovirus vector of choice (reviewed by Miller (1988) Ann. Rev. Microbiol. **42**, 177-199) is *Autographa californica* multiple nuclear polyhedrosis virus, AcMNPV.

Nucleic acids and/or proteins according to the invention may be used in methods for screening compounds of mixtures of compounds which are potential modulators of GABA_{B} receptors, and thus potential pharmacological agents. For example, cells transformed with a gene encoding a GABA_{B} receptor or receptor protein can be used in a cell-based screening assay, in which the response of the cell to the agents being tested is monitored. The response may be in the form of the activation of a reporter gene, a measurable pharmacological or electrophysiological change, or the like. Alternatively, purified GABA_{B} receptors or receptor proteins according to the invention can be used in *in vitro* assays to screen for modulators of GABA_{B} receptor activity.

Likewise, compounds which are capable of modulating the expression of the GABA_{B} receptor genes, thus regulating GABA_{B} receptor activity, can be screened for using an expression system in which a test gene (which may be one of the GABA_{B} receptor genes itself) is operably linked to the control sequences normally associated with the GABA_{B} receptor gene.

The invention moreover includes compounds identified by such screening assays and the use of such compounds for the treatment of conditions which are susceptible to treatment by GABA_{B} receptor modulation as exemplified hereinbefore.

In accordance with yet another embodiment of the present invention, there are provided antibodies specifically recognising and binding to one or more of the GABA_{B} receptors or receptor proteins of the invention. For example, such antibodies can be generated against the GABA_{B} receptors having the amino acid sequences set forth in SEQ ID Nos. 2 and 8. Alternatively, GABA_{B} receptor proteins as set forth in SEQ ID Nos. 4 and 6 or GABA_{B} receptor protein fragments (which may also be synthesised by *in vitro* methods) are fused (by recombinant expression or an *in vitro* peptidyl bond) to an immunogenic polypeptide and this fusion polypeptide, in turn, is used to raise antibodies against a GABA_{B} receptor protein epitope.

Anti-GABA_{B} receptor or receptor protein antibodies may be recovered from the serum of immunised animals. Monoclonal antibodies may be prepared from cells from immunised animals in the conventional manner.

The antibodies of the invention are useful for studying GABA_{B} receptor protein localisation, screening of an expression library to identify nucleic acids encoding GABA_{B} receptors or receptor proteins or the structure of functional domains, as well as for the purification of GABA_{B} receptors or receptor proteins, and the like.

Antibodies according to the invention may be whole antibodies of natural classes, such as IgE and IgM antibodies, but are preferably IgG antibodies. Moreover, the invention includes antibody fragments, such as Fab, F(ab')₂, Fv and ScFv. Small fragments, such Fv and ScFv, possess advantageous properties for diagnostic and therapeutic applications on account of their small size and consequent superior tissue distribution.

The antibodies according to the invention may be used in diagnostic and therapeutic applications. Accordingly, they may be altered antibodies comprising an effector protein such as a toxin or a label. Especially preferred are labels which allow the imaging of the distribution of the antibody *in vivo*. Such labels may be radioactive labels or radioopaque labels, such as metal particles, which are readily visualisable within an organism. Moreover, they may be fluorescent labels or other labels which are visualisable on tissue samples removed from organisms.

Recombinant DNA technology may be used to improve the antibodies of the invention. Thus, chimeric antibodies may be constructed in order to decrease the immunogenicity thereof in diagnostic or therapeutic applications. Moreover, immunogenicity may be minimised by humanising the antibodies by CDR grafting [see European Patent Application 0 239 400 (Winter)] and, optionally, framework modification [see EP 0 239 400 and Riechmann *et al*., Nature 332, 323-327, 1988].

Antibodies according to the invention may be obtained from animal serum, or, in the case of monoclonal antibodies or fragments thereof, produced in cell culture. Recombinant DNA technology may be used to produce the antibodies according to established procedure, in bacterial or preferably mammalian cell culture. The selected cell culture system preferably secretes the antibody product.

Therefore, the present invention includes a process for the production of an antibody according to the invention comprising culturing a host, e.g. *E. coli* or a mammalian cell, which has been transformed with a hybrid vector comprising an expression cassette comprising a promoter operably linked to a first DNA sequence encoding a signal peptide linked in the proper reading frame to a second DNA sequence encoding said protein, and isolating said protein.

The invention further concerns hybridoma cells secreting the monoclonal antibodies of the invention. The preferred hybridoma cells of the invention are genetically stable, secrete monoclonal antibodies of the invention of the desired specificity and can be activated from deep-frozen cultures by thawing and recloning.

The invention also concerns a process for the preparation of a hybridoma cell line secreting monoclonal antibodies directed to a GABA_{B} receptor or receptor protein, characterised in that a suitable mammal, for example a Balb/c mouse, is immunised with purified GABA_{B} receptor or receptor protein, an antigenic carrier containing purified GABA_{B} receptor or receptor protein or with cells bearing GABA_{B} receptor or receptor protein, antibody-producing cells of the immunised mammal are fused with cells of a suitable myeloma cell line, the hybrid cells obtained in the fusion are cloned, and cell clones secreting the desired antibodies are selected. For example spleen cells of Balb/c mice immunised with cells bearing GABA_{B} receptor or receptor protein are fused with cells of the myeloma cell line PAl or the myeloma cell line Sp2/0-Ag14, the obtained hybrid cells are screened for secretion of the desired antibodies, and positive hybridoma cells are cloned.

The invention also concerns recombinant DNAs comprising an insert coding for a heavy chain variable domain and/or for a light chain variable domain of antibodies directed to the extracellular domain of GABA_{B} receptor or receptor protein as described hereinbefore. By definition such DNAs comprise coding single stranded DNAs, double stranded DNAs consisting of said coding DNAs and of complementary DNAs thereto, or these complementary (single stranded) DNAs themselves.

The invention also provides a transgenic non-human mammal which has been modified to modulate the expression of endogenous GABA_{B} receptor or receptor protein. Preferably, the transgenic non-human mammal is a transgenic mouse. For example, therefore, a transgenic mouse may be designed in which GABA_{B} receptor or receptor protein production is greatly reduced or eliminated, according to procedures established in the art (Mansour et *al*., Nature 336, 348-352, 1988). Alternatively, the transgenic mouse of the invention may express elevated levels of GABA_{B} receptor or receptor protein, or may be subject to regulation of GABA_{B} receptor or receptor protein expression in a developmentally or tissue-specific manner, or via control by exogenous agents. Study of such an animal provides insights into the importance of the GABA_{B} receptors and receptor proteins *in vivo.*

The invention is further described hereinbelow, for the purposes of illustration only, in the following Examples.

### Reference Example 1

### Synthesis of ligand CGP64213

The radioligand **[**^{**125**}**I]CGP 64213,** which is used to visualise GABA_{B} receptors expressed in COS cells, is synthesised according to Scheme 1, using the following reagents and conditions:
(1) NaH, THF, rt, 3 h; 5-bromovaleronitrile, rt, 16 h; (2) Raney nickel, 4% NH₃ in EtOH, 45° C, 16 h; (3) *N*-ethoxy-carbonylphtalimide, Na₂CO₃, H₂O, CH₂Cl₂, rt, 5h; (4) Me₃SiCl, EtOH, CH₂Cl₂ (1:9), rt, 17 h; (5) Me₃SiCl, Et₃N, THF, rt, 17 h; (*R*)-epichlorohydrin, 10 mol% ZnCl₂ THF, 80° C, 17 h; HOAC, MeOH, rt, 17 h; (6) *i*-Pr₂EtN, EtOH, 80° C, 7 d; (7) LiOH, EtOH, H₂O (1:1), 100° C, 17 h; MeOH, H₃PO₄; (8) conc. HCl, 100° C, 17 h; (9) *i*-Pr₂EtN, DMF, rt, 72 h; (10) Na¹²⁵I, phosphate buffer pH 7.4, H₂O₂, cat. lactoperoxidase, 30 min, RP-HPLC.

Ethyl (1,1-diethoxyethyl)phosphinate 1, prepared according to Froestl, W., *et al. J. Med. Chem*. (1995), **38**, 3297-3312, from phosphinic acid and triethylorthoacetate under catalysed by boron trifluoride diethyl etherate, is condensed with 5-bromovaleronitrile to give the oily cyano-derivative **2** (bp 164° C at 0.13 mbar), which is hydrogenated over Raney nickel in ethanol containing 4% of ammonia to give primary amine **3** (bp 150-160° C at 10⁻⁴ mbar; Kugelrohr bath temperature). The amino-group in **3** is protected as pthalimide to give **4**, which is now deprotected at the phosphinic acid moiety under very mild conditions to give monosubstituted phosphinic acid ester 5. On reaction with trimethylchlorosilane the penta-valent phosphinate ester 5 is converted into a very reactive silyated phosphonite, which reacts readily with (*R*)-epichlorohydrin under zinc chloride catalysis to produce chlorohydrin **7**. Condensation with 1-(*R*)-(+)-(3-cyanophenyl)-ethylamine **8**, which itself is prepared via resolution of racemic (3-cyano-phenyl)-ethylamine with *N*-acetly-*L*-leucine to separate 1-(*S*)-(+)-(3-cyanophenyl)-ethylamine (according to Pickard et al., *J. Amer. Chem. Soc.* (1990) **112,** 5741-5747) and treatment of the remaining mother liquors with (-)-camphanic acid followed by three crystallisations, gives the aromatic nitrile-ester **9**, which is hydrolysed to the meta-benzoic acid derivative 10 with lithium hydroxide. Concomitant hydrolysis of the ethyl phophinate ester occurs. The pthalimide protecting group is removed by boiling with concentrated hydrochlorid acid overnight to give the key intermediate **CGP 57604A**([3-[1-(*R*)-[[3-(5-aminopentyl)-hydroxyphosphinyl]-2-(*S*)-hydroxypropyl]amino]-ethyl]-benzoic acid hydrochloride). This is reacted with commercially available *N*-hydroxysuccinimidyl-3-(4-hydroxyphenyl)-propionate **11** in DMF using Hünig's base to give intermediate **12,** which is iodinated with sodium iodide (125 isotope) using hydroperoxide and catalytic amounts of lactoperoxidase to give the radioactive ligand **[**^{**125**}**I]CGP 64213.**

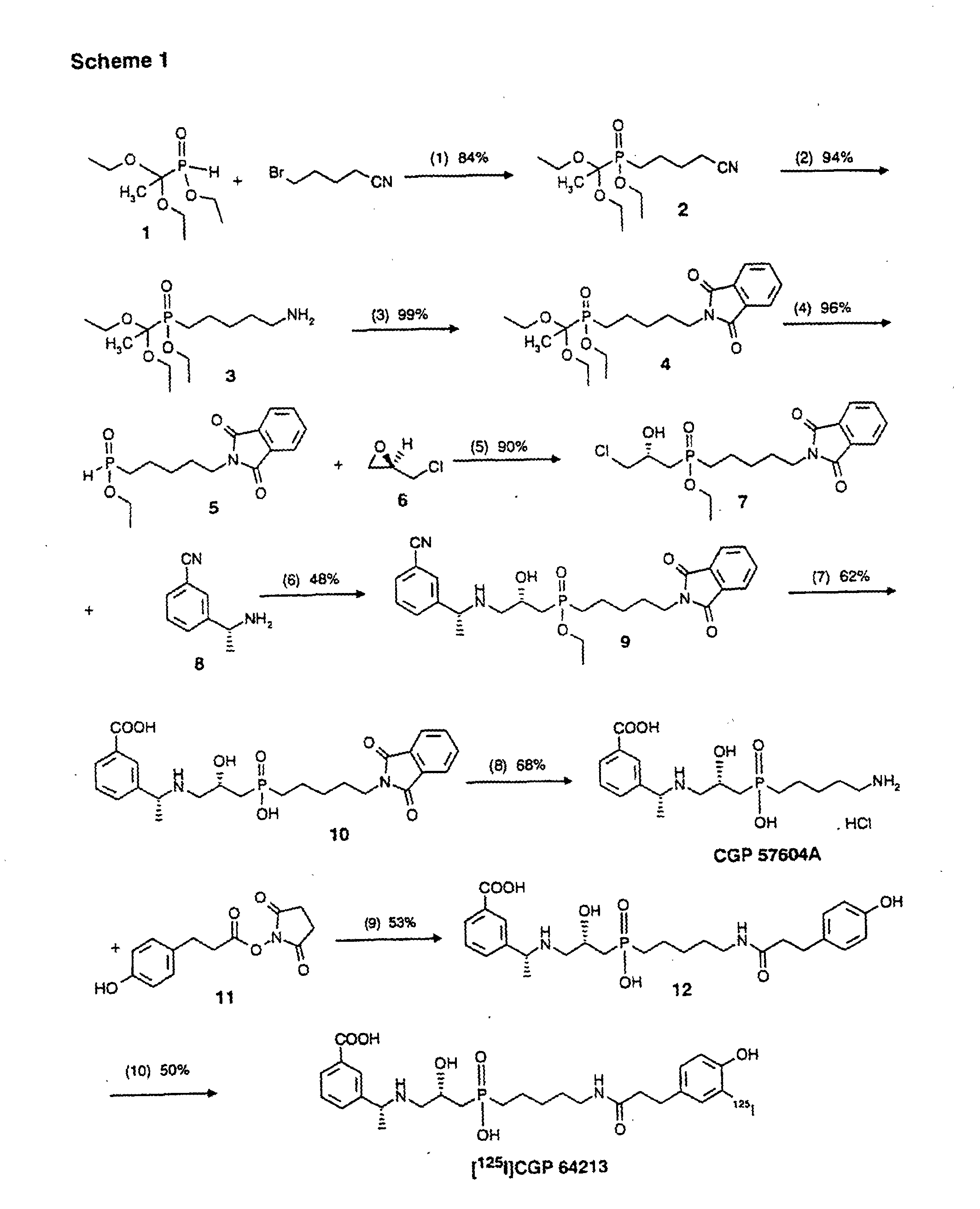

Unlabelled **CGP 64213** is prepared in a slightly different way: 3-(4-hydroxy-5-iodophenyl propionic acid **13** is prepared by iodination of 3-(4-hydroxy-phenyl)propionic acid according to Runeberg, J., *Acta Chem. Scand.* (1958), **12,** 188-91. *N*-hydroxy-succinimidyl-3-(4-hydroxy-5-iodophenyl)propionate **14** (mp: 191-4° C) is prepared according to Scheme 2 in 73% yield. Condensation of **CGP 57604A** (Scheme 1) with **14** using Hünig's base in DMF at room temperature for 72 hours proceeded as reaction 9 of Scheme 1 to give non radioactive **CGP 64213** (mp: 170-5° C, crystallised from acetone) in a yield of 53%.

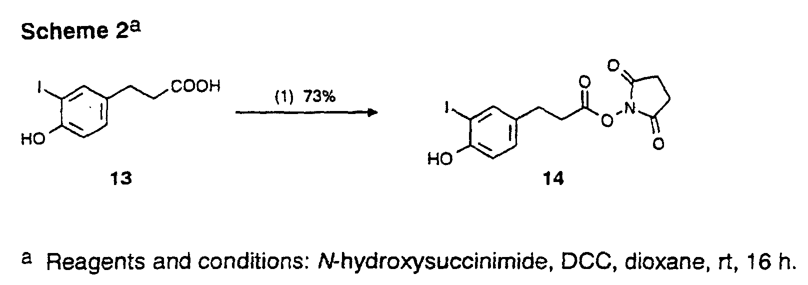

### Characterisation of radioligand [¹²⁵I]CGP 64213:

### Preparation of synaptic membranes from rat cerebral cortex

Twenty male rats [Tif: RAI f (SPF)] of about 200 g body weight are used. The animals are decapitated, the brains removed, the cerebral cortices dissected and homogenised in 10 volumes of ice-cold 0.32 M sucrose, containing MgCl₂ (1 mM) and K₂HPO₄ (1 mM), with a glass/Teflon homogeniser. The membranes are centrifuged at 1000 x g for 15 min, the pellet resuspended and the centrifugation repeated. The supernatants are pooled and centrifuged at 20000 x g for 15 min. The pellet is osmotically shocked by resuspension in 10 volumes H₂O and kept on ice for 30 min. The suspension is centrifuged at 39000 x g, resuspended in Krebs-Henseleit buffer (20mM Tris, pH 7.4, 118mM NaCl, 5.6mM glucose, 1.2mM KH₂PO₄, 1.2mM MgSO₄, 4.7mM KCI, 1.8mM CaCl₂), and kept for 2 days at -20°C. The membranes are thawed at room temperature, washed three times with Krebs-Henseleit buffer by centrifugation at 20000 x g for 15 min, left overnight at 4°C and washed again three times. The final pellet is resuspended with a glass/Teflon homogenise in 20 ml of the same buffer. 2 ml aliquots are frozen and stored in liquid nitrogen. Just before use membranes are thawed quickly in a water bath at 37°C and again washed by centrifugation at 20000 x g for 15 min with the same buffer three times.

### Binding assay and characterisation of radioligand

Incubation with [¹²⁵I]CGP 64213, specific radioactivity for fresh material 2000 Ci/mmol, is performed in 0.2 ml Krebs-Henseleit-Tris buffer, pH 7.4, at 20°C for 90 min with 50µg cortex membrane protein as substrate. The incubation is terminated by filtration through GF/B Whatman glass fibre filters. Nonspecific binding is defined by 10⁻⁶ M CGP 54626A and is 5% of total binding at a concentration of 2 nM. In saturation experiments with increasing concentrations of [¹²⁵I]CGP 64213 and with nonlinear least square fitting a dissociation constant K_{D} of 2.66 nM is determined. In inhibition studies at a concentration of 0.1 nM [¹²⁵I]CGP 64213, L-baclofen showed an inhibition constant Kᵢ of 442 nM and the antagonist CGP 54626 A a Kᵢ of 2.5 nM in good agreement with Kᵢ's obtained with other GABA_{B} receptor antagonist radioligands. Unlabelled CGP 64213 is found to be inactive at a concentration of 1 µM in assays for GABA_{A}, benzodiazepine, kainate, AMPA, NMDA receptors, for the strychnine independent binding site at NMDA receptors, muscarinic cholinergic, α₁- and α₂- adrenergic, β-adrenergic, 5HT₁, 5HT₂, 5HT₃, histamine₁, histamine₂, adenosine₁ µ- opiate and substance P receptors. The compound is therefore selective for GABA_{B} receptors. At a concentration of 0.1 nM of [¹²⁵I]CGP 64213 association and dissociation kinetics are measured. The halftime of association is 20 min at 20°C and the halftime of dissociation 40 min. The halftime of dissociation is increased to 4 hours by reduction of the temperature to 4°C. This slow off rate and the high specific radioactivity of [¹²⁵I]CGP 64213 allows autoradiographic studies of receptor binding in COS cells as expression systems for GABA_{B} receptors.

### Reference Example 2

### Preparation of photoaffinity ligand

The photoaffinity ligand **[**^{**125**}**I]CGP 71872,** which is used to tag GABA_{B} receptors from rat cortex membranes and recombinant GABA_{B} receptors expressed in COS cells is synthesised according to Scheme 3: Commercially available *N*-hydroxy-succinimidyl-4-azidosalicylate **15** is condensed with **CGP 57604A** to give intermediate **16,** which is iodinated with sodium iodide 125 isotope using chloramine T to give an approximately 1:1 mixture of the 5-iodo derivative **[**^{**125**}**I]CGP 71872** and the 3-iodo-derivative **[**^{**125**}**I]CGP 72565.** They are separated via reverse phase HPLC on a Vydac 218TP54 column (retention times: 16.4 and 17.4 minutes, respectively). Reagents and conditions are as follows:
(1) **CGP 57604A** (Scheme 1), *i*-Pr₂EtN, DMF, rt, 70 h; (2) Na¹²⁵I, chloramine T, 0.01 N NaOH, rt, 1 h; RP-HPLC.

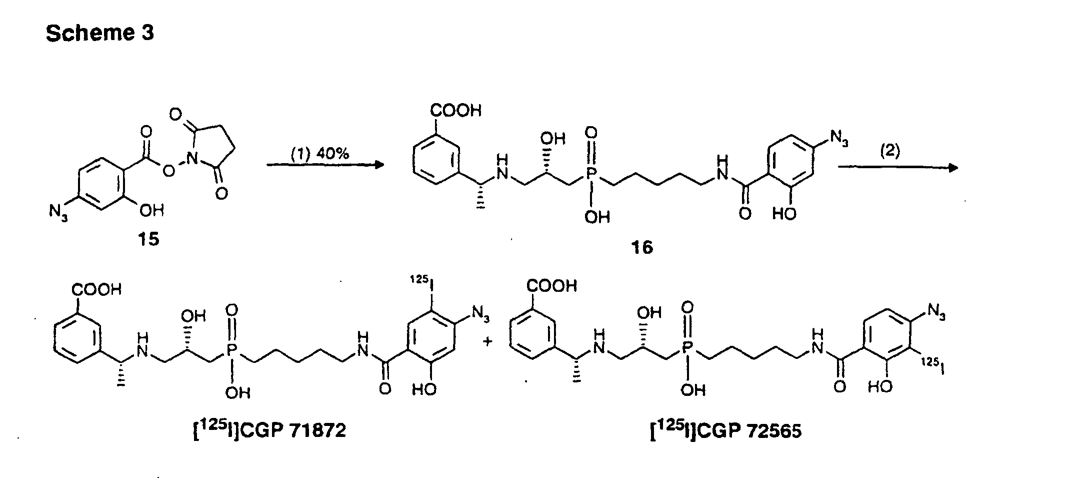

Unlabelled **CGP 71872** is prepared in a different way: N-hydroxy-succinimidyl-4-azido-5-iodo-salicylate **17** is prepared via iodination of 4-azidosalicylic acid and subsequent condensation with *N*-hydroxy-succinimide (Scheme 4). Condensation of **17** with **CGP 57604A** (see Scheme 1, reaction 9) proceeded in 57 % yield to give non radioactive **CGP 71872** (mp: >190° C dec.).

Reagents and conditions as follows: (1) (1) Nal, 2N NaOH, chloramine T, rt, 88 h; (2) *N*-hydroxysuccinimide, DCC, dioxane, rt, 16 h;

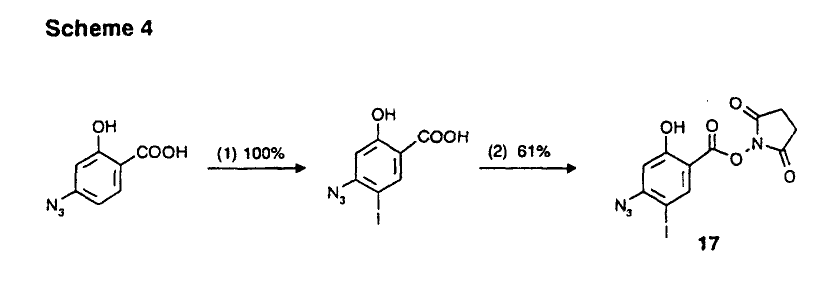

### Characterisation of photoaffinity ligand [¹²⁵I]CGP 71872:

### Binding assay and characterisation of ligand

Rat cortex membranes as described for the [¹²⁵I]CGP 64213 assay are used as substrates. Incubation with [¹²⁵I]CGP 71872, specific radioactivity of fresh material 2000Ci/mmol, is performed in 0.2 ml Krebs-Henseleit buffer, pH 7.4, at 20°C for 90 min with 50 µg membrane protein as substrate The incubation is terminated by filtration through GF/C Whatman glass fibre filters. Nonspecific binding is defined by 10⁻⁶ M CGP 54626 A and is 5% of total binding at a concentration of 2 nM of [¹²⁵I]CGP 71872, In saturation experiments with increasing concentrations of [¹²⁵I]CGP 71872, and nonlinear last square fitting a dissociation constant K_{D} of 3.1 nM is calculated. L-baclofen showed in inhibition experiments a Kᵢ of 340 nM and the antagonist CGP 54 626 A showed a Kᵢ of 3.1 nM. Unlabelled CGP 64213 is found to be inactive at a concentration of 1µM in the same receptor assays as described for [¹²⁵I]CGP 64213 and is, therefore, also selective for GABA_{B} receptors. At a concentration of 2 nM and at 20°C, the halftime for association is 5 min, the halftime of dissociation 10 min. The dissociation time at 8°C is much longer. Only 25% of radioligand dissociates after 120 min.

### Photoaffinity labelling of membranes

Membranes from rat cerebral cortex and from COS1 cells transiently transfected with GABA_{B}R1a and GABA_{B}R1b rat-cDNA, respectively, suspended in Krebs-Henseleit-Tris buffer, pH 7.3, at a concentration of 4 mg protein/ml, are incubated in the dark with 0.6 nM [¹²⁵I] CGP 71872 for one hour at room temperature. The incubation is terminated by centrifugation at 20 000 x g for 10 min at 4°C. This step removed free unbound photoaffinity label. Under these conditions about 50% of the total radioactivity used bound to the receptors. The pellet is resuspended at a concentration of 4mg protein/ml in a polyethylene vial and illuminated with UV light (365 nm) for 3 min (24 W). The suspension is centrifuged at 20 000 x g for 10 min and resuspended at a concentration of 8mg/ml protein in buffer. When the labelling is performed in the presence of excess unlabelled GABA_{B} receptor antagonist (10⁻⁶ M CGP 54626A), no radioactivity is bound to the membranes. The labelled membranes could be stored at -80°C. The results are shown in Figures 1a and 1b.

Additionally, [¹²⁵I]CGP71872 photoaffinity labelling of cortex, cerebellum and spinal cord cell membranes is analysed as outlined above and reveals that the two GABA_{B} protein variants R1a and R1b are differentially expressed in the nervous system. In cerebellum the 100K protein is predominant over the 130K protein, whereas in spinal cord the 130K protein is more prevalent. In cortex tissue both proteins appear equally abundant. No proteins are labelled in tissues such as liver and kidney which are expected to lack GABA_{B} receptors and therefore have been used as controls (see Figure 4a).

Furthermore, native GABA_{B} receptors are photoaffinity-labelled in the presence of various competitor substances indicated in Figure 4b. Neither the GABA_{A} selective ligands muscimol and bicuculline nor the GABA_{C} receptor agonist cis-aminocrotonic acid (CACA) or the inhibitor of the GABA uptake system, SK&F89976A (Zuiderwijk, M., Veenstra, E., Lopes Da Silva, F. H. & Ghijsen, W. E. J. M. Effects of uptake carrier blockers SK&F89976-A and L-*trans*-PDC on in vivo release of amino acids in rat hippocampus. *Eur. J. Pharmacol. 307,* 275-282 (1996)), compete significantly for radioligand binding. In contrast, the GABA_{B} receptor agonists GABA, APPA (3-aminopropyl-phosphinic acid) and L-baclofen compete with [¹²⁵I]CGP71872 for binding. As another known criterion, L-baclofen competes more potently than D-baclofen. The GABA_{B} receptor antagonists CGP54626A, CGP35348 and the non-radioactive photoaffinity ligand are also effective displacers of [¹²⁵I]CGP71872 at native receptors. For all ligands tested, there is no visible difference in the displacement of [¹²⁵I]CGP71872 at the 130K and 100K proteins, indicating a qualitatively similar binding pharmacology for the two receptors.

Native GABA_{B} receptors are N-glycosylated, as shown by the reduction in molecular weight to 110K and 90K, respectively, after cleavage with N-glycosidase F (Fig. 4c). No significant shift in molecular weight is detected after enzymatic treatment with O-glycosidase (Fig. 4c). Photoaffinity-labelled proteins of 130K and 100K are detectable in tissues from all vertebrate species analysed, including zebrafish (Fig. 4d), indicating that the two proteins and their antagonist binding site are highly conserved. The avian GABA_{B} receptor proteins exhibit molecular weights slightly higher than in other species, possibly reflecting differences in glycosylation and/or RNA splicing. No binding of the photoaffinity ligand to any protein can be detected in the fruitfly *Drosophila melanogaster* and the nematode *Haemonchus concortus*.

### Reference Example 3

### Synthesis of the GABA_{B} antagonist ligand CGP 54626A:

The ligand used for displacement experiments, **CGP 54626A,** is synthesised according to Scheme 5:

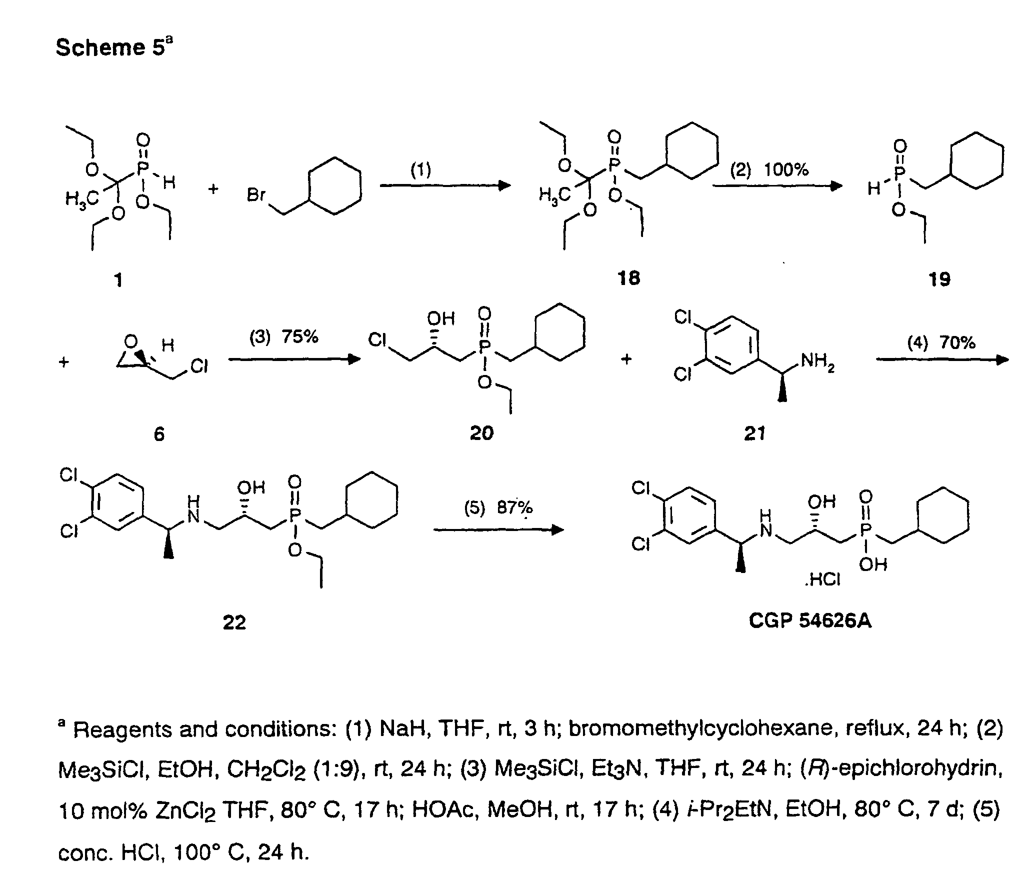

Ethyl (1,1-diethoxyethyl)phosphinate **1,** prepared according to Froestl et al., *J*. *Med. Chem*. (1995), **38,** 3297-3312, from phosphinic acid and triethylorthoacetate catalysed by boron trifluoride diethyletherate, is condensed with bromomethytcyclohexane to give the oily derivative **18** (bp 85° C at 6 × 10⁻⁴ mbar), which is deprotected at the phosphinic acid moiety under very mild conditions to give monosubstituted phosphinic acid ester **19** (bp 50° C at 3 x 10⁻⁴ mbar). On reaction with trimethylchlorosilane the penta-valent phosphinate ester **19** is converted into a very reactive trivalent ethyl phosphonite, which reacted rapidly with (*R*)-epichlorohydrin **6** when catalysed by zinc chloride to produce chlorohydrin **20.** Condensation with 1-(*S*)-(-)-(3,4-dichlorophenyl)-ethylamine **21,** prepared via resolution of racemic 1-(3,4-dichlorophenyl)-ethylamine with (+)-mandelic acid according to Mickel, EP 543780 A2, gave the corresponding secondary amine 22 as a 1:1 mixture of diastereoisomers, which is hydrolysed by boiling with concentrated hydrochloric acid to give **CGP 54626A.**

**[**^{**3**}**H]CGP54626A** is prepared in an analogous way (Scheme 6) by condensation of ethyl (1,1-diethoxyethyl)phosphinate 1 with 3,4-dehydro-cylohexylmethylbromide (prepared according to Yadav and Fallis, (1991) *Can. J. Chem*. **69**, 779-789), preparation of the corresponding 3,4-dehydroderivative of **CGP 54626A,** i.e. **CGP 54951A,** which is tritiated under very carefully controlled conditions to yield **[**^{**3**}**H]CGP54626A.** The compound is the first GABA_{B} receptor antagonist radioligand which was characterised by Bittiger *et al*., *Pharmacol. Commun.* (1992), **2**, 23.

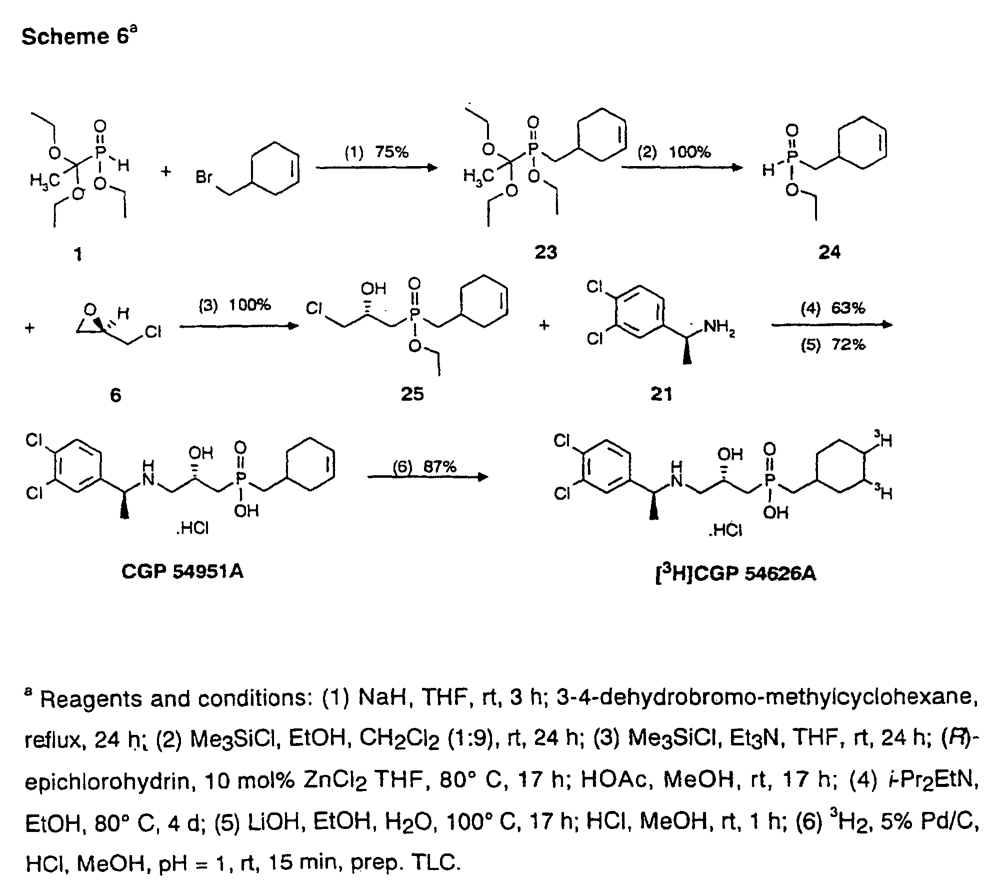

### Example 4

### Proof of functional activity of CGP 64213 and CGP 71872 as GABA_{B} receptor antagonists by in vitro electrophysiological measurements.

Experiments are performed on 400 µm thick hippocampal slices obtained either from female Wistar COB rats (3-4 weeks old) or male rats Tif: RAI f (SPF) using standard techniques. In brief, rats are cervically dislocated prior to decapitation. The brain minus cerebellum is removed rapidly and placed in ice-cold artificial cerebrospinal fluid (ACSF). The hippocampus is carefully isolated and, using either a tissue chopper (Sorvall®) or a vibroslicer (Campden), transverse 400 µm thick slices are cut. The CA3 region of each slice is removed via a scalpel cut. This procedure is performed to eliminate changes in network function that can occur due to epileptiform bursting in area CA3. The resultant CA3-ectomized slices are placed on a nylon mesh at the interface of a warmed (32°C), perfusing (1-2 ml.min⁻¹) ACSF and an oxygen-enriched (95% 0₂, 5% CO₂), humidified atmosphere. The standard perfusion medium comprised (mM): NaCl, 124; KCl, 3; NaHCO₃, 26; NaH₂PO₄, 1.25; CaCl₂, 2; MgSO₄, 1; D-glucose, 10; and is bubbled with 95% O₂, 5% CO₂. An Axoprobe or an Axoclamp-2 amplifier (Axon Instruments, Foster City, CA, USA) is used in bridge mode to make extracellular recordings from either *stratum radiatum* or *stratum oriens* using 4 M NaCl-filled microlectrodes (2 - 5 MΩ). Intracellular recordings are made using 2 M potassium methylsulphate filled microelectrodes (60-100 M Ω). Digitised records are stored on the hard disk of an IBM-compatible PC for off-line analysis. Bipolar stimulating electrodes, made from 55 µm diameter insulated nickel-chromium wire, are positioned in *stratum radiatum* close to the recording electrode placed in either *stratum radiatum* or *stratum oriens,* to provide orthodromic monosynaptic activation of CA1 neurones (Davies *et al*. (1990) *Journal of Physiology* **424:** 513). In every experiment stimuli comprise square-wave pulses (20-200 µs; 5-30 V) delivered homosynaptically at a fixed intensity. All drugs are administered via the perfusion medium. Data are presented as means ± standard error of the mean (S.E.M.) and statistical significance is assessed using Students *t*-tests. n values refer to the number of times a particular experiment is performed, each in a different slice taken from a different rat.

### GABA_{B} autoreceptors

Paired-pulse widening of field EPSPs is used to monitor the effects of CGP 71872 and CGP 64213 on GABA_{B} autoreceptors. Paired-pulse widening occurs when two stimuli are delivered at 5-10 Hz (interstimulus interval 100 - 200 ms); a stimulation protocol that does not release sufficient GABA to activate GABA_{B} heteroreceptors which would, in any case, cause a depression rather than a facilitation of the second field EPSP. This phenomenon is also independent of postsynaptic GABA_{B} receptors (Nathan *et al*. (1991) *Exp*. *Brain Res.* **84(3)** 529-537). It is, however, occluded by blocking GABA_{A} receptor-mediated IPSPs and is inhibited by GABA_{B} receptor antagonists at concentrations that are required to block GABA_{B} autoreceptors (Nathan *et al*. (1990), *Brain Research* **531:** 55-65). (Note that these concentrations are 3-10 fold higher than those necessary to block postsynaptic GABA_{B} receptors on both pyramidal neurones and inhibitory interneurones so ruling out an effect at these receptors). Paired-pulse widening of field EPSPs (fEPSPs) is a sensitive measure of GABA_{B} autoreceptor activity. There is no precedent for any compound being effective in this test system and not in other assays of GABA_{B} autoreceptor activity e.g., paired-pulse or (-)-baclofen-induced depression of IPSCs.

Paired-pulse stimulation at an interstimulus interval of 200 ms caused a consistent widening of the second EPSP relative to the first EPSP. Thus, the area under the curve of the second fEPSP is 247 ± 17 % (in the CGP 64213 series of experiments) and 241 ± 21 % (in the CGP 71872 series of experiments) of the first fEPSP, respectively. In the presence of CGP 64213 (0.3 µM; n = 5) and CGP 71872 (1 µM; n = 3) this paired-pulse widening of EPSPs is abolished indicating the effectiveness of these compounds as antagonists of GABA_{B} autoreceptors.

### GABA_{B} heteroreceptors

The effect of CGP 71872 on the depression of field EPSPs induced by bath application of (-)-baclofen is used as an assay for the effect of this compound on GABA_{B} heteroreceptors located on glutamate afferent terminals. Although, under these conditions, (-)-baclofen will activate other populations of GABA_{B} receptors (e.g., GABA_{B} autoreceptors and postsynaptic GABA_{B} receptors), in addition to GABA_{B} heteroreceptors, activation of these receptors would tend to increase the size of the field EPSP rather than decrease it. As such, this method is a reasonable measure of activity at GABA_{B} heteroreceptors. This method provides a more reliable and a quantitatively more repeatable method for activating GABA_{B} heteroreceptors than that used by lsaacson *et al*. (1993) *Neuron* **332**: 156-158, as it does not rely on physiologically released GABA to activate the heteroreceptors. This latter method is inherently variable due to the different concentrations of synaptically released GABA to which heteroreceptors are exposed in different preparations; a parameter that depends upon the level of GABA released, the distance between the release site and heteroreceptor, and the efficiency of GABA uptake sites. It is important to note, however, that, to date, no discrepancy between the results obtained using these two methods to study GABA_{B} heteroreceptors has been documented for any compound tested.

(-)-Baclofen (10 µM) had no significant effect on the presynaptic fibre volley of the field EPSP (100 ± 1 % of control; *P*>0.05), recorded in *stratum radiatum*, but depressed the field EPSP slope and peak amplitude by 65 ± 6% and 76 ± 9%, respectively (*n* = 10). Maximum depression is obtained after a 5-10 min perfusion and persisted at this level for the duration of the agonist application. Addition of CGP 71872 (1 µM) to the perfusion medium reversed the depression in every experiment in which it is tested (n = 6; P<0.05). Similar results are obtained for field EPSPs recorded in stratum oriens (*n* = 3). In brain slices CGP 71872 had no significant effect on the peak amplitude, slope or presynaptic fibre volley of field EPSPs recorded in *stratum radiatum (n =* 4; *P*>0.05) or *oriens (n* = 3).

### Postsynaptic GABA_{B} receptors

The effect of CGP 71872 on the pharmacologically isolated late IPSP is used as a test system to evaluate the effect of CGP 71872 on postsynaptic GABA_{B} receptors located on CA1 pyramidal neurones. There is a substantial literature (Froestl et al. (1995) Op. Cit.; Jarolimek *et al*. (1993) *Neurosci*. *Lett*. **154:** 31-34; Olpe *et al*. (1990) *Clin. Neuropharmacol*. **13 Suppl. 2,:** 396; McCormick, (1990) *J.Neurophysiol*. **62/5:** 1018; Lambert *et al*., (1989) *Neurosci. Lett.* **107:** 125-128; Soltesz *et al.,* (1989) *Brain Research* **479:** 49-55; Mueller and Misgeld, (1989) *Neurosci. Lett*. **102:** 229-234; Dutar and Nicoll, (1988) *Nature* **322:** 156-8; Karlsson, Pozza and Olpe, (1988) *Eur. J. Pharmacol*. **148:** 485-486) which indicates that this IPSP is mediated by the synaptic activation of GABAs receptors. In addition, this method has been used many times in the past and the data generated have always been consistent with that generated for antagonism of (-)-baclofen-induced hyperpolarisations; an approach that has also been adopted as an assay for activity at postsynaptic GABA_{B} receptors.

The effect of CGP 71872 is tested on a monosynaptically activated GABA_{B} receptor-mediated late IPSP isolated using a combination of the ionotropic excitatory amino acid antagonists D-2-amino-5-phosphonopentanoate (AP5; 50µM) and 6-cyano-7-nitroquinoxaline-2,3-dione (CNQX; 20 µM) and the GABA_{A} receptor antagonist picrotoxin (50µM). In all neurones tested CGP 71872 (1 µM) abolished the late IPSP (*n* = 6) indicating that this compound is an antagonist of postsynaptic GABA_{B} receptors.

### Example 5

### cDNA library construction

RNA is purified from cortex and cerebellum of 7 day old rats according to Chomczynski, P. & Sacchi, N. (1987) *Anal. Biochem*. **162**, 156-159. Poly A(+) RNA is enriched by two passages over an oligo (dT) column (Boehringer Mannheim) as described (Maniatis, T., Fritsch, E.F. & Sambrook, J. (1982) *Molecular cloning: A laboratory manual* (Cold Spring Harbor Lab., Cold Spring Harbor, NY). Oligo (dT) primed double stranded cDNA is synthesised from 5 µg of poly A(+) RNA using a commercial cDNA synthesis system (Amersham). The reverse transcriptase supplied with the kit is replaced by the RNAseH(-) Superscript II reverse transcriptase (Gibco BRL). The cDNA solution is concentrated on Centricon-100 devices (Amicon), preabsorbed with tRNA, to a final volume of 100µl. Small cDNAs are removed by passage through a Chromaspin-1000 column (Clontech). BstXI adaptors (Invitrogen) are added using T4 DNA ligase (Boehringer Mannheim) and the cDNAs are size-fractionated on an agarose gel. cDNAs with sizes larger than 2kb are purified (Qiaex, Qiagen) and ligated into the BstXI sites of the expression vector pcDNAI (Invitrogen). An aliquot of the ligation mixture is transformed (BioRad Gene Pulser II) into electrocompetent MC1061/P3 E.coli cells. The complexity of the library is estimated to be 2 x 10⁶ independent clones. The average insert size deduced from the analysis of 48 clones is 2.9kb (sizes ranging from 2.0kb to 6.6kb).

Plasmids for the transfections of COS1 cells are isolated from bacterial colonies obtained after the initial round of cDNA transformation. Briefly, an aliquot of the cDNA library is transformed into electrocompetent MC1061/P3 E.coli cells and titrated by plating on agar plates. The cDNA library is divided into pools of approximately 2'000 colonies that are plated on 9cm agar plates and grown overnight at 37°C. The bacteria are scraped off the plates and plasmid DNA is prepared using ion exchange columns (Qiawell, Qiagen).

### Example 6

### Transfection of COS cells with cDNA

COS1 cells are obtained from the American Type Culture Collection (ATCC) and grown in Dulbecco's modified Eagle medium (DMEM) supplemented with 10% fetal calf serum (FCS) and 15µg/ml gentamycin (Gibco BRL) in a humidified atmosphere with 5% CO₂.

Plasmid DNA from pools of independent bacterial colonies are introduced into COS1 cells using a modification of the standard DEAE-dextran transfection procedure. Briefly, one day before transfection 7.5 × 10⁵ cells are seeded per 9cm dish. The next day the medium is removed and the cells are incubated 15 min in 10ml of phosphate buffered saline (PBS tablets, Gibco BRL). Afterwards, PBS is removed and 4ml of 1mg/ml DEAE-dextran (Pharmacia) in PBS is added to the dish. After 9 min incubation at room temperature the cells are washed twice with 5ml of PBS each. The PBS is aspirated and 4µg plasmid DNA (derived from pools of 2'000 independent bacterial colonies) in 540µl PBS is added to the dish and the cells incubated with the DNA for 30 min at 37°C with occasional rocking. Subsequently 4ml of DMEM medium containing 10% NU-serum (Collaborative Research) and 80µM chloroquine (Sigma) is added. After 4 hrs incubation at 37°C the medium is removed and the cells are incubated 2 min in 10% (vol/vol) dimethyl sulfoxide (Merck) in PBS. The cells are rinsed with PBS, cell culture medium is added to the culture dishes and the cells are grown for an additional 2 to 3 days.

### Example 7

### Identification of GABA_{B} receptor clone by ligand binding assay

Pools of cDNAs (2000 independent clones each) are analysed for GABA_{B} receptor expression, after transient transfection into COS1 cells, using a radioligand binding assay with iodinated CGP64213 (specific activity 2'000 Ci/mmol).

Culture dishes with transfected COS1 cells are placed on ice and washed twice with 5ml each of ice-cold Krebs-Henseleit-Tris buffer (20mM Tris-Cl pH 7.4, 118mM NaCl, 5.8mM glucose, 1.2mM KH₂PO₄, 1.2mM MgSO₄, 4.7mM KCI, 1.8mM CaCl₂). Afterwards the cells are incubated with 0.2nM of ¹²⁵I-CGP 64213 in Krebs-Tris buffer (1ml solution per 9cm dish). After 80 min incubation at room temperature the dishes are cooled on ice and washed twice for 5 min with 5ml of ice-cold Krebs-Tris buffer. Subsequently the dishes are air dried using a fan and the walls of the plates are removed. For autoradiography, the bottom of the plates are exposed, together with intensifying screens, to Kodak X-OMAT AR films for 2 to 3 weeks at -80°C.

A total of 640,000 independent clones (320 individual pools) from the above mentioned cDNA library are screened. One pool yields a positive signal in the ligand binding assay. The plasmid DNA from this pool is re-transformed into electrocompetent MC1061/P3 cells. 10 plasmid pools from 500 colonies each are prepared, two of which rescreened positive in the binding assay. After 4 subsequent rounds of subdivisions of one of the two pools (SIB selection; McCormick, M. (1987) *Methods Enzymol.* **151,** 445-449) a single cDNA clone containing a 4376bp insert is identified. This first cDNA clone identified, originally referred to as F4, is designated GABA_{B}R1a (SEQ ID No. 1). This cDNA clone encompasses a large open reading frame coding for a putative protein of 960 amino acids with a calculated molecular weight of 108kDa (SEQ ID No.2). According to von Heijne (von Heijne, G. (1986) *Nucl. Acids. Res.* **14**, 4683-4691) the first 16 amino acids encode with high probability a signal peptide that is absent in the mature protein. The calculated molecular weight of the predicted mature protein is 106kDa. Hydrophobicity analysis of the putative protein with the algorithm of Kyte and Dolittle (1982) *J. Mol. Biol*. **157**, 105-132, using sequence analysis programs from the University of Wisconsin Genetics Computer Group (Devereux, *et al*., (1984) *Nucl. Acids. Res.* **12**, 387-395) predicts, as expected for a cell surface receptor coupled to G-proteins, several membrane spanning regions. Putative N-glycosylation sites are found at amino acid positions 7, 67, 392, 423, 465, 485, 497 and 614 of the predicted mature protein as set forth in SEQ ID No. 2.

### Example 8

### Assay of cloned GABA_{B} receptor

In order to isolate membranes containing the cloned GABA_{B} receptor, culture dishes containing GABA_{B} receptor-expressing COS cells are washed twice with Krebs-Henseleit-Tris buffer. Afterwards the cells are scraped off the dishes, homogenised in a glass-glass homogeniser and centrifuged for 30 min at 4°C at 40'000 g. The homogenisation and centrifugation step is repeated once. The pellet is resuspended in buffer and stored in liquid nitrogen until further analysis.

Membranes from COS1 cells transfected with the GABA_{B} receptor cDNA (membranes derived in a similar manner from brain tissue are used for reference) are suspended in Krebs-Henseteit-Tris buffer at a concentration of approximately 1 mg/ml. The membranes are then incubated in the dark with 0.6nM ¹²⁵I-CGP 71872 for one hour at room temperature. In control experiments 1µM of unlabeled CGP 54626A, a GABA_{B} receptor specific antagonist, is included. The incubation is terminated by centrifugation at 20'000 g for 10 min at 4°C. The pellet is washed once in buffer to remove unbound from bound photoaffinity label. The pellet is resuspended in buffer and illuminated with UV light (365nm, 24W) for 3 min. The suspension is again centrifuged (20 min, 40'000 g). The pellet is washed in buffer, dissolved in SDS sample buffer and separated on a 6% SDS gel according to Laemmli, U.K (1970) *Nature* 227, 680-685. The gel is dried and, together with intensifying screens, exposed to Dupont Reflection NEF-495 X-ray films overnight. The protein expressed from the 4'376bp cDNA clone has an apparent molecular mass of about 120kDa (Figure 1). The apparent molecular weight of the recombinant GABA_{B} receptor is estimated from gel mobility relative to those of SDS-PAGE standards (BioRad).

The binding pharmacology of the GABA_{B}R1a receptor expressed in COS1 cells is compared with the binding pharmacology of native GABA_{B} receptors in rat cerebral cortex membranes. To that aim, the binding characteristics of the radioligand [¹²⁵I]CGP 64213 and the inhibition of this binding by selected GABA_{B} receptor antagonists and agonists are compared. The dissociation constant K_{D} for the GABA_{B}R1a receptor expressed in COS cells is determined to be 1.85 nM. The K_{D} of GABA_{B} receptors expressed in cortex membranes is determined to be 2.7 nM and thus is similar to the value obtained for the recombinant receptor. The IC₅₀ values (Table 1) and the slopes of the inhibition curves (Figure 2) for the GABA_{B} receptor antagonists CGP 54626A (Froestl *et al*., (1992) *Pharmacol*. *Communications* **2**, 52-56), CGP 71872, CGP 64213 and CGP 35348 (Froestl *et al*., 1992) are very similar for recombinant and native receptors. The rank order of affinity for the agonists GABA, L-baclofen and CGP 27492 (aminophosphinic acid, APPA) is identical at recombinant and native receptors, however the agonist affinity is always significantly lower at the recombinant GABA_{B}R1a receptor (Figure 3, Table 1). It is known that GTP or its stable analogue Gpp(NH)p reduce the affinity of agonists at native GABA_{B} receptors by decoupling the receptors from their G-proteins (Hill *et al*., (1984) *J.Neurochem.* **42,** 652-657). Therefore, the lower affinity of agonists at the recombinant receptor may reflect the fact that in COS cells the G-proteins that normally couple to GABA_{B} receptors in brain cells are not available. We have determined that for rat cortex GABA_{B} receptors the IC₅₀ value of L-baclofen is shifted from 170 nM to 10 µM in the presence of 300 µM Gpp(NH)p. Thus decoupling G-proteins from native GABA_{B} receptors results in an IC₅₀ value comparable to the 34 µM obtained for the recombinant GABA_{B}R1a receptor expressed in COS cells. In conclusion, the recombinant GABA_{B}R1a receptor shows similar binding pharmacology as native GABA_{B} receptors from rat cortex.

**Table 1.**

| BINDING PHARMACOLOGY OF NATIVE AND RECOMBINANT GABA_{B} RECEPTORS | | |
|---|---|---|
| inhibition of [¹²⁵I]CGP 64213 binding by GABA_{B} receptor antagonists and agonists | | |
| *ANTAGONISTS* | *Rat cerebral cortex* IC₅₀ (µM) | *COS 1 cells* IC₅₀ (µM) |
| CGP 54626A | 0.0019 | 0.0016 |
| CGP 64213 | 0.0014 | 0.0022 |
| CGP 71872 | 0.0021 | 0.0038 |
| CGP 35348 | 9.3 | 20.0 |

| *AGONISTS* | | |
|---|---|---|
| GABA | 0.13 | 23.9 |
| L-baclofen | 0.17 | 34.0 |
| CGP 27492 (APPA) | 0.018 | 2.6 |
| CGP 47656 (partial agonist) | 0.28 | 12.3 |

### Example 9

### Use of the GABA_{B}R1a receptor cDNA to clone related genes

The rat GABA_{B}R1a-receptor cDNA isolated (SEQ ID No. 1) is useful as a probe to identify and isolate additional cDNAs, genes and proteins of the GABA_{B}-receptor gene family. It is also useful to identify and isolate cDNAs, genes and proteins of the GABA_{B}-receptor gene family in other species, such as for example humans.

In order to isolate a further rat clone (referred to as GABA_{B}R1b) and human GABA_{B} receptor clones, the abovementioned rat library and a human fetal brain cDNA library (Clontech, Palo Alto, cat. No. HL3025s) are cross-hybridised with the GABA_{B}R1a cDNA under suitable hybridisation conditions. The human library is an unidirectional oligo (dT)-primed library consisting of 1.2 × 10⁶ independent cDNA clones inserted into the expression vector pcDNAI. The method of screening a plasmid library by colony hybridisation is described in Sambrook et al. (1989). The hybridisation probe used is a ³²P-labelled 1.3kb Pvull/Scal fragment corresponding to bases 1931 to 3264 of the GABA_{B}R1a cDNA (SEQ ID No. 1). Hybridisation is in 0.5M NaH₂PO₄ (pH 7.2), 7% SDS, 1 mM EDTA at 60°C overnight. Subsequent wash steps are for one hour at a final stringency of 0.5 x SSC, 0.1 % SDS at 55 °C (rat library) or 2 x SSC, 0.1% SDS at 50°C (human library). Kodak X OMAT AR films are exposed to the membranes overnight at -80°C with intensifying screens. The X-ray films are aligned to the agar plates with the bacterial colonies and colonies containing cross-hybridising cDNA clones are isolated. The bacteria are replated on agar dishes and the colony hybridisation screen is repeated twice. The individual colonies obtained are further analysed by Southern blot hybridisation. Selected cDNA clones are analysed by sequencing and a 2,9 kb cDNA for rat GABA_{B}R1b characterised (see SEQ ID No. 5). This cDNA encodes a protein of 844 amino acids (see SEQ ID No. 6). The mature GABA_{B}R1b differs from the former GABA_{B}R1a in that the N-terminal 147 amino acid residues are replaced by 18 different residues. Presumably, these two GABA_{B} receptor variants are derived from the same gene by alternative splicing. Those clones which are positive in screening the human library are also analysed by sequencing and reveal one clone termed GABA_{B}R1a/b (see SEQ ID No. 3) with a partial sequence encoding a receptor protein of 793 amino acid residues (see SEQ ID No. 4), as well as another clone termed GABA_{B}R1b human (see SEQ ID No. 7) which represents a full-length cDNA encoding a human GABA_{B} receptor having 844 amino acids (see SEQ ID No. 8).

### Example 10

### GABA_{B} receptors stably expressed in HEK293 cells negatively couple to adenylate cyclase

GABA_{B} receptors are described to inhibit adenylate cyclase activity, stimulate phospholipase A₂, activate K⁺-channels, inactivate voltage-dependent Ca²⁺-channels and to modulate inositol phospholipid hydrolysis. As GABA_{B}R1a and -b have identical sequence in all domains predicted to be intracellular they are expected to be able to couple to the same effector systems. Using rat cortical slice preparations, L-baclofen has been shown to reduce forskolin-stimulated CAMP accumulation by about 40 percent. The ability of GABA_{B}R1a stably expressed in HEK293 cells to reduce forskolin-stimulated CAMP accumulation is analysed (Fig. 5). We chose concentrations of forskolin and L-baclofen that should produce a maximal effect. Forskolin stimulates CAMP levels in HEK293 cells to more than ten times over the basal level. Stimulation of recombinantly expressed GABA_{B} receptors by co-addition of 300 µM L-baclofen reduces forskolin stimulated cAMP accumulation by approximately 30 percent. This inhibition is antagonised by CGP54626A, a GABA_{B} receptor antagonist. The modulation of adenylate cyclase activity by GABA_{B}R1a is sensitive to pertussis toxin, indicating that in HEK293 cells, which are deficient in Gₒ, GABA_{B}R1a couples to Gᵢ. As a control, L-baclofen does not inhibit forskolin-stimulated cAMP formation in untransfected HEK293 cells (Fig. 5).

### Deposition Data

The GABA_{B} receptor clone GABA_{B}R1a derived from rat was deposited under the Budapest Treaty at the Deutsche Sammlung von Microorganismen und Zellkulturen GmbH (DSMZ), Mascheroder Weg 1b, D-38124 Braunschweig, Germany, with an effective deposition date of 17th May 1996 under the accession number DSM 10689.

The GABA_{B} receptor clones GABA_{B}R1b derived from rat as well as GABA_{B}R1b derived from human sources were deposited under the Budapest Treaty at the Deutsche Sammlung von Microorganismen und Zellkulturen GmbH (DSMZ), Mascheroder Weg 1b, D-38124 Braunschweig, Germany, with an effective deposition date of 21th February 1997 under the accession numbers DSM 11422 and 11421, respectively.

## Claims

1. An isolated GABA_{B} receptor or receptor protein encoded
(a) by any of the nucleic acid sequences comprising a nucleic acid sequence as set forth in nucleotides 182 to 3061 of SEQ ID NO:1; as set forth in nucleotides 1 to 2379 of SEQ ID NO:3; as set forth in nucleotides 228 to 2759 of SEQ ID NO:5; or as set forth in nucleotides 169 to 2700 of SEQ ID NO:7;
(b) by any of the nucleic acid clones selected from the group consisting of clones deposited at the DSMZ under accession number DSM 10689, DSM 11421 and DSM 11422; or
(c) by any of the nucleic acid sequences which hybridize under moderate stringent conditions to polynucleotides of SEQ ID NO:1, SEQ ID NO:3, SEQ ID NO:5 or SEQ ID NO:7 whereby the moderate stringent conditions are completed with a wash step of about 60-62 °C, 1x SSC and 0.1% SDS.

2. The GABA_{B} receptor or receptor protein of claim 1 wherein said GABA_{B} receptor or receptor protein is capable of specifically binding to at least one of the selective GABA_{B} receptor antagonists of Formulae I or II:

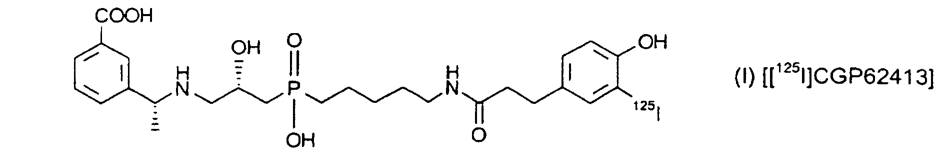

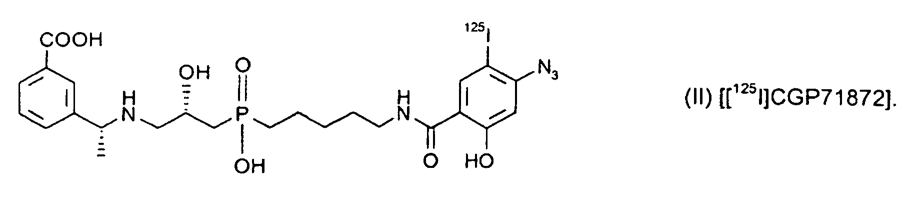

3. The isolated GABA_{B} receptor or receptor protein according to claim 1, wherein the isolated GABA_{B} receptor or receptor protein is a human GABA_{B} receptor or receptor protein.

4. The GABA_{B} receptor or receptor protein according to claim 1, wherein the amino acid sequence comprises an amino acid sequence having more than 30% identity with SEQ ID NO:2, SEQ ID NO:4, SEQ ID NO:6, or SEQ ID NO:8.

5. The GABA_{B} receptor or receptor protein according to claim 1, wherein the amino acid sequence comprises an amino acid sequence as set forth in SEQ ID NO:2, SEQ ID NO:4, SEO ID NO:6, or SEQ ID NO:8.

6. An isolated nucleic acid encoding a GABA_{B} receptor or receptor protein comprising an amino acid sequence as set forth in SEQ ID NO:1; SEO ID NO:3; SEO ID NO:5; or SEQ ID NO:7.

7. The isolated nucleic acid according to claim 6, wherein the nucleic acid sequence comprises the nucleotide sequence as set forth in nucleotides 182 to 3061 of SEQ ID NO:1; as set forth in nucleotides 1 to 2379 of SEQ ID NO:3; as set forth in nucleotides 228 to 2759 of SEO ID NO:5; or as set forth in nucleotides 169 to 2700 of SEQ ID NO:7.

8. The isolated nucleic acid according to claim 6, wherein the nucleic acid sequence is encoded by a nucleic acid clone selected from the group consisting of clones deposited at the DSMZ under accession number DSM 10689, DSM 11421 and DSM 11422.

9. A nucleic acid probe which is capable of hybridising to any one of the nucleic acid sequences comprising SEQ ID NO:1, SEQ ID NO:3, SEQ ID NO:5 or SEQ ID NO:7 under conditions of moderate or high stringency, whereby the moderate stringent conditions are completed with a wash step of about 60-62°C, 1 x SSC, 0.1 % SDS and the high stringent conditions are completed with a wash step of about 65-68 °C, 0.2 - 0.1x SSC, 0.1 % SDS, and with the proviso that the nucleic acid probe with the EMBL Accession Number X90542 is excluded.

10. The nucleic acid probe according to claim 9, wherein the nucleic acid probe has an antisense nucleic acid sequence in relation to the nucleic acid sequences as set forth in claim 9.

11. An antibody which is generated against a receptor or receptor protein as set forth in SEQ ID NO:2, SEQ ID NO:4, SEQ ID NO:6, or SEQ ID NO:8.

12. The antibody of claim 11, wherein the antibody is a monoclonal, polyclonal, or chimeric antibody.

13. The antibody of claim 11, wherein the antibody is a Fab, F(ab')₂, Fv or ScFv fragment.

14. A replicable nucleic acid vector comprising a coding sequence consisting of a nucleic acid according to claim 6 operably linked to control sequences.

15. A host cell transformed with a vector according to claim 14.

16. A transgenic non-human animal cell comprising a GABA_{B} receptor or receptor protein according to claim 1 provided that said transgenic non-human animal cell does not endogenously express a GABA_{B} receptor or receptor protein according to claim 1.

17. A method for identifying a nucleic acid encoding a GABA_{B} receptor or receptor protein, comprising the steps of:
preparing an expression library encoding cDNA molecules which potentially encode a GABA_{B} receptor or receptor protein;
screening the expression library with a specific ligand capable of binding to a GABA_{B} receptor or receptor protein, wherein the specific ligand is the compound of Formula I 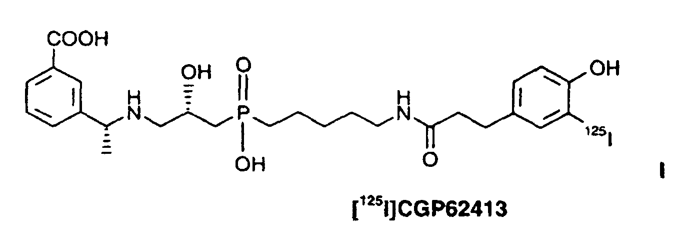 or the compound of Formula II;

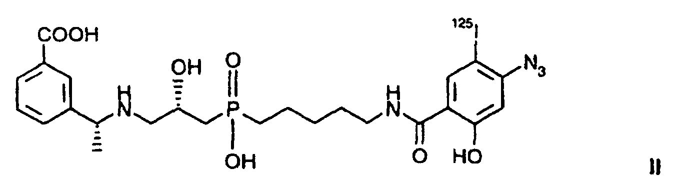

18. A method for identifying a nucleic acid encoding a GABA_{B} receptor or receptor protein, comprising the steps of:
(a) preparing a library encoding cDNA or genomic DNA molecules which potentially encode a GABA_{B} receptor or receptor protein;
(b) screening the library by hybridisation with a nucleic acid probe according to claim 10; and
(c) identifying the nucleic acid molecules which hybridise to the probe.

19. A method for screening compounds or mixtures of compounds which are potential modulators of GABA_{B} receptor activity, comprising the steps of:
preparing a test system comprising a recombinant GABA_{B} receptor or receptor protein; exposing the test system to the compound or mixture of compounds;
identifying the compound or mixture of compounds which causes modulation of GABA_{B} receptor activity as measured by the test system.

20. A method for screening compounds or mixtures of compounds which are potential modulators of GABA_{B} receptor expression, comprising the steps of:
providing an expression system comprising a test gene operably linked to control sequences normally associated with a gene encoding a GABA_{B} receptor or receptor protein;
identifying the compounds which cause a change in the level of expression of the test gene.

21. The selective GABA_{B} receptor antagonist of Formula I or II according to claim 2.

## Patentansprüche

1. Isolierter GABA_{B} Rezeptor oder isoliertes GABA_{B} Rezeptorprotein, das kodiert ist
(a) durch eine der Nukleinsäuresequenzen, die eine Nukleinsäuresequenz umfassen; wie sie durch die Nukleotide 182 bis 3061 der SEQ ID Nr. 1 angegeben ist, wie sie durch die Nukleotide 1 bis 2379 der SEQ ID Nr. 3 angegeben ist, wie sie durch die Nukleotide 228 bis 2759 der SEQ ID Nr. 5 angegeben ist oder wie sie durch die Nukleotide 169 bis 2700 der SEQ ID Nr. 7 angegeben ist,
(b) durch einen der Nukleinsäureklone, die aus der Gruppe ausgewählt sind, die aus den bei der DSMZ unter der Hinterlegungsnummer DSM 10689, DSM 11421 und DSM 11422 hinterlegten Klonen besteht, oder
(c) durch eine der Nukleinsäuresequenzen, die unter moderat stringenten Bedingungen an Nukleotide der SEQ ID Nr. 1, SEQ ID Nr. 3, SEQ ID Nr. 5 oder SEQ ID Nr. 7 hybridisieren, wobei die moderat stringenten Bedingungen mit einem Waschschritt mit etwa 60-62°C, 1x SSC und 0,1 % SDS abgeschlossen werden.

2. GABA_{B} Rezeptor oder Rezeptorprotein nach Anspruch 1, worin der GABA_{B} Rezeptor oder das GABA_{B} Rezeptorprotein zur spezifischen Bindung an zumindest einen der selektiven GABA_{B} Rezeptorantagonisten der Formeln I oder II fähig ist

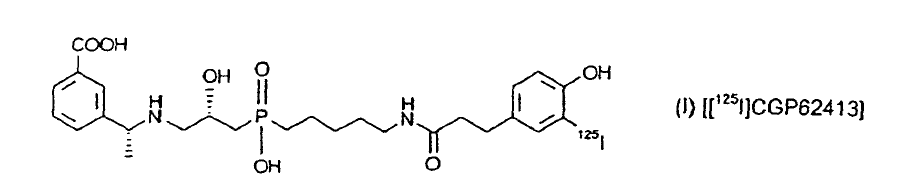

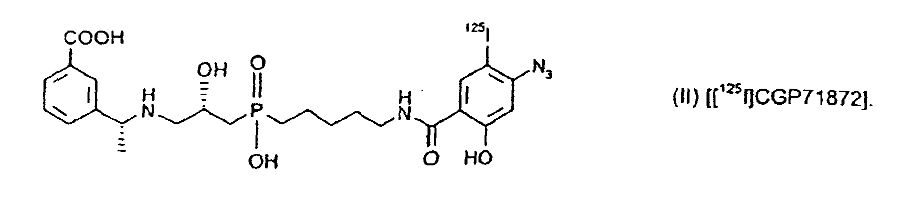

3. Isolierter GABA_{B} Rezeptor oder isoliertes Rezeptorprotein nach Anspruch 1, worin der isolierte GABA_{B} Rezeptor oder das Rezeptorprotein ein humaner GABA_{B} Rezeptor oder ein humanes GABA_{B} Rezeptorprotein ist.

4. GABA_{B} Rezeptor oder Rezeptorprotein nach Anspruch 1, worin die Aminosäuresequenz eine Aminosäuresequenz umfasst, die mehr als 30 % Identität mit der SEQ ID Nr. 2, SEQ ID Nr. 4, SEQ ID Nr. 6 oder SEQ ID Nr. 8 aufweist.

5. GABA_{B} Rezeptor oder Rezeptorprotein nach Anspruch 1, worin die Aminosäuresequenz eine Aminosäuresequenz umfasst, wie die in SEQ ID Nr. 2, SEQ ID Nr. 4, SEQ ID Nr. 6 oder SEQ ID Nr. 8 angegeben ist.

6. Isolierte Nukleinsäure, die für einen GABA_{B} Rezeptor oder ein GABA_{B} Rezeptorprotein kodiert, die eine Aminosäuresequenz umfasst, wie sie in SEQ ID Nr. 1, SEQ ID Nr. 3, SEQ ID Nr. 5 oder SEQ ID Nr. 7 angegeben ist.

7. Isolierte Nukleinsäure nach Anspruch 6, worin die Nukleinsäuresequenz die Nukleotidsequenz umfasst, wie sie in den Nukleotiden 182 bis 3061 der SEQ ID Nr. I angegeben ist, wie sie in den Nukleotiden 1 bis 2379 der SEQ ID Nr. 3 angegeben ist, wie sie in den Nukeotiden 228 bis 2759 der SEQ ID Nr. 5 angegeben ist oder wie sie in den Nukleotiden 169 bis 2700 der SEQ ID Nr. 7 angegeben ist.

8. Isolierte Nukleinsäure nach Anspruch 6, worin die Nukleinsäuresequenz durch einen Nukleinsäureklon kodiert ist, der aus der Gruppe ausgewählt ist, die aus Klonen besteht, die bei der DSMZ unter der Hinterlegungsnummer DSM 10689, DSM 11421 und DSM 11422 hinterlegt sind.

9. Nukleinsäuresonde, die zur Hybridisierung mit einer der Nukleinsäuresequenzen, die die SEQ ID Nr. 1, SEQ ID Nr. 3, SEQ ID Nr. 5 oder SEQ ID Nr. 7 umfassen, unter Bedingungen einer moderaten oder hohen Stringenz fähig ist, wobei die moderat stringenten Bedingungen mit einem Waschschritt von etwa 60-62°C, 1x SSC und 0,1 % SDS abgeschlossen werden und die hochstringenten Bedingungen mit einem Waschschritt von etwa 65-68°C, 0,2 - 0,1x SSC und 0,1 % SDS abgeschlossen werden und mit der Maßgabe, dass die Nukleinsäuresonde mit der EMBL Hinterlegungsnummer X90542 ausgeschlossen ist.

10. Nukleinsäure nach Anspruch 9, worin die Nukleinsäuresonde eine Antisensenukleinsäuresequenz in Bezug auf die Nukleinsäuresequenzen aufweist, wie sie in Anspruch 9 aufgeführt sind.

11. Antikörper, der gegen einen Rezeptor oder ein Rezeptorprotein erzeugt wurde, wie dies in SEQ ID Nr. 2, SEQ ID Nr. 4, SEQ ID 6 oder SEQ ID Nr. 8 angegeben ist.

12. Antikörper nach Anspruch 11, worin der Antikörper ein monoklonaler, polyklonaler oder chimärer Antikörper ist.

13. Antikörper nach Anspruch 11, worin der Antikörper ein Fab, F(ab')₂, Fv oder ScFv Fragment ist.

14. Replizierbarer Nukleinsäurevektor, der eine kodierende Sequenz umfasst, die aus einer Nukleinsäure nach Anspruch 6 besteht, die operativ an Kontrollsequenzen gebunden ist.

15. Wirtszelle, die mit einem Vektor nach Anspruch 14 transformiert ist.

16. Transgene nicht-humane Tierzelle, de einen GABA_{B} Rezeptor oder ein GABA_{B} Rezeptorprotein nach Anspruch 1 umfasst, mit der Maßgabe, dass die transgene, nicht-humane Tierzelle endogen keinen GABA_{B} Rezeptor oder kein GABA_{B} Rezeptorprotein nach Anspruch 1 exprimiert.

17. Verfahren zur Identifizierung einer Nukleinsäure, die einen GABA_{B} Rezeptor oder ein GABA_{B} Rezeptorprotein kodiert, das **gekennzeichnet ist durch** folgende Schritte:
Herstellung einer Expressionsbank, die cDNA Moleküle kodiert, die potentiell für einen GABA_{B} Rezeptor oder ein GABA_{B} Rezeptorprotein kodieren,
Screening der Expressionsbank mit einem spezifischen Liganden, der zur Bindung an einen GABA_{B} Rezeptor oder ein GABA_{B} Rezeptorprotein fähig ist, worin der spezifische Ligand die Verbindung der Formel I ist 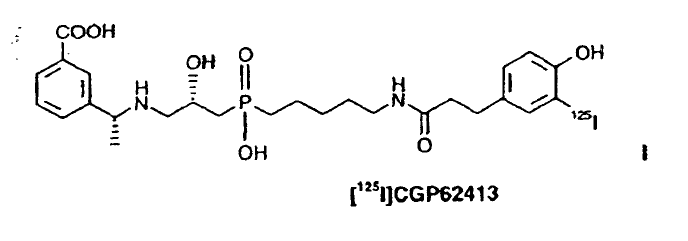 oder die Verbindung der Formel II 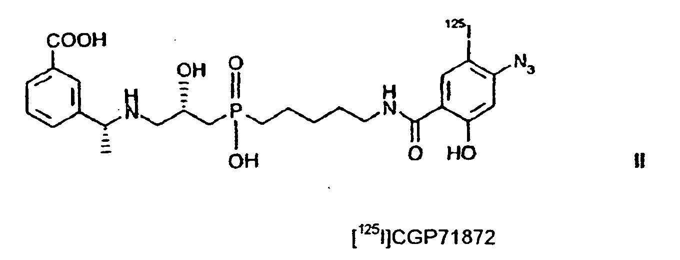 und Isolierung des cDNA Klons, der für einen GABA_{B} Rezeptor oder ein GABA_{B} Rezeptorprotein kodiert.

18. Verfahren zur Identifizierung einer Nukleinsäure, die für einen GABA_{B} Rezeptor oder ein GABA_{B} Rezeptorportein kodiert, das die folgenden Schritte umfasst:
(a) Herstellung einer für cDNA oder genomische DNA Moleküle kodierenden Bank, die potentiell für einen GABA_{B} Rezeptor oder ein GABA_{B} Rezeptorprotein kodieren,
(b) Screening der Bank durch Hybridisierung mit einer Nukleinsäuresonde nach Anspruch 10, und
(c) Identifizierung der Nukleinsäuremoleküte, die mit der Sonde hybridisieren.

19. Verfahren zum Screenen von Verbindungen oder Verbindungsgemischen, die potentielle Modulatoren der GABA_{B} Rezeptoraktivität sind, das die folgenden Schritte umfasst:
Herstellung eines Testsystems, das einen rekombinanten GABA_{B} Rezeptor oder ein rekombinantes GABA_{B} Rezeptorprotein umfasst,
Exposition des Testsystems gegenüber der Verbindung oder dem Verbindungsgemisch, und Identifizierung der Verbindung oder des Verbindungsgemisches, das eine Modulation der GABA_{B} Rezeptoraktivität verursacht, wie dies durch das Testsystem gemessen wird.

20. Verfahren zum Screening von Verbindungen oder Verbindungsgemischen, die potentielle Modulatoren der GABA_{B} Rezeptorexpression sind, das die folgenden Schritte umfasst:
Bereitstellung eines Expressionssystems, das ein Testgen umfasst, welches funktionsfähig an Kontrollsequenzen gebunden ist, die normalerweise mit einem Gen assoziiert sind, das für einen GABA_{B} Rezeptor oder ein GABA_{B} Rezeptorprotein kodiert, und
Identifizierung der Verbindungen, die eine Veränderung in der Expressionsmenge des Testgens verursachen.

21. Selektiver GABA_{B} Rezeptorantagonist der Formel I oder II nach Anspruch 2.

## Revendications

1. Récepteur de GABA_{B} ou protéine de récepteur de GABA_{B} isolé(e) codé(e)
(a) par l'une quelconque des séquences d'acides nucléiques comprenant une séquence d'acides nucléiques telle que représentée dans les nucléotides 182 à 3061 de la SEQ ID N°1 ; telle que représentée dans les nucléotides 1 à 2379 de la SEQ ID N°3 ; telle que représentée dans les nucléotides 228 à 2759 de la SEQ ID N°5 ; ou telle que représentée dans les nucléotides 169 à 2700 de la SEQ ID N°7 ;
(b) par l'un quelconque des clones d'acides nucléiques choisis dans le groupe constitué par les clones déposés au DSMZ sous les numéros d'accès DSM 10689, DSM 11421 et DSM 11422 ; ou
(c) par l'une quelconque des séquences d'acides nucléiques qui s'hybrident dans des conditions de stringence modérée aux polynucléotides de la SEQ ID N°1, de la SEQ ID N°3, de la SEQ ID N°5 ou de la SEQ ID N°7, les conditions de stringence modérée étant complétées par une étape de lavage à environ 60 - 62 °C, 1 × SSC et SDS à 0,1 %.

2. Récepteur de GABA_{B} ou protéine de récepteur de GABA_{B} selon la revendication 1, ledit récepteur ou ladite protéine de récepteur de GABA_{B} étant capable de se lier spécifiquement à au moins l'un des antagonistes sélectifs du récepteur de GABA_{B} de formule I ou II :

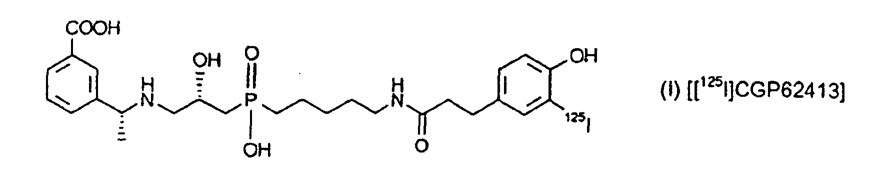

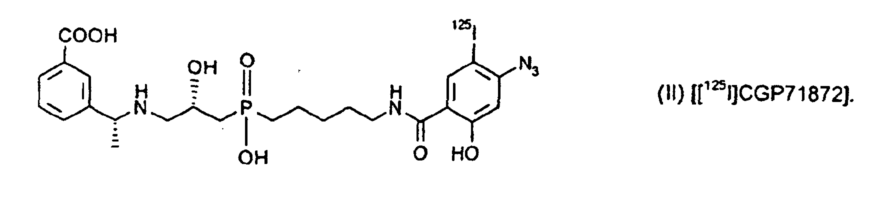

3. Récepteur de GABA_{B} ou protéine de récepteur de GABA_{B} isolé(e) selon la revendication 1, le récepteur de GABA_{B} ou la protéine de récepteur de GABA_{B} isolé(e) étant un récepteur de GABA_{B} ou une protéine de récepteur de GABA_{B} humain(e).

4. Récepteur de GABA_{B} ou protéine de récepteur de GABA_{B} selon la revendication 1, la séquence d'acides aminés comprenant une séquence d'acides aminés ayant plus de 30 % d'identité avec la SEQ ID N°2, la SEQ ID N°4, la SEQ ID N°6 ou la SEQ ID N°8.

5. Récepteur de GABA_{B} ou protéine de récepteur de GABA_{B} selon la revendication 1, la séquence d'acides aminés comprenant une séquence d'acides aminés telle que représentée dans la SEQ ID N°2, la SEQ ID N°4, la SEQ ID N°6 ou la SEQ ID N°8.

6. Acide nucléique isolé codant pour un récepteur de GABA_{B} ou une protéine de récepteur de GABA_{B} comprenant une séquence d'acides aminés telle que représentée dans la SEQ ID N°1, la SEQ ID N°3, la SEQ ID N°5 ou la SEQ ID N°7.

7. Acide nucléique isolé selon la revendication 6, dans lequel la séquence d'acides nucléiques comprend la séquence nucléotidique telle que représentée dans les nucléotides 182 à 3061 de la SEQ ID N°1 ; telle que représentée dans les nucléotides 1 à 2379 de la SEQ ID N°3 ; telle que représentée dans les nucléotides 228 à 2759 de la SEQ ID N°5 ; ou telle que représentée dans les nucléotides 169 à 2700 de la SEQ ID N°7.

8. Acide nucléique isolé selon la revendication 6, dans lequel la séquence d'acides nucléiques est codée par un clone d'acide nucléique choisi dans le groupe constitué par les clones déposés au DSMZ, sous les numéros d'accès DSM 10689, DSM 11421 et DSM 11422.

9. Sonde d'acide nucléique qui est capable de s'hybrider à l'une quelconque des séquences d'acides nucléiques comprenant la SEQ ID N°1, la SEQ ID N°3, la SEQ ID N°5 ou la SEQ ID N°7, dans des conditions de stringence modérée à forte, les conditions de stringence modérée étant complétées par une étape de lavage à environ 60 - 62 °C, 1× SSC, SDS à 0,1 %, et les conditions de forte stringence étant complétées par une étape de lavage à environ 65 - 68 °C, 0,2 - 0,1× SSC, SDS à 0,1 %, et sous réserve que la sonde d'acide nucléique portant le numéro d'accès EMBL X90542 soit exclue.

10. Sonde d'acide nucléique selon la revendication 9, dans laquelle la sonde d'acide nucléique a une séquence d'acides nucléiques antisens en relation avec les séquences d'acides nucléiques telles qu'indiquées dans la revendication 9.

11. Anticorps généré contre un récepteur ou une protéine de récepteur tel(le) que représenté(e) dans la SEQ ID N°2, la SEQ ID N°4, la SEQ ID N°6 ou la SEQ ID N°8.

12. Anticorps selon la revendication 11, celui-ci étant un anticorps monoclonal, polyclonal ou chimère.

13. Anticorps selon la revendication 11, dans lequel l'anticorps est un fragment Fab, F(ab')₂, Fv ou ScFv.

14. Vecteur d'acide nucléique réplicable comprenant une séquence de codage constituée par un acide nucléique selon la revendication 6 lié de manière fonctionnelle à des séquences témoins.

15. Cellule hôte transformée par un vecteur selon la revendication 14.

16. Cellule animale non humaine transgénique comprenant un récepteur de GABA_{B} ou une protéine de récepteur de GABA_{B} selon la revendication 1, sous réserve que ladite cellule animale non humaine transgénique n'exprime pas de manière endogène un récepteur de GABA_{B} ou une protéine de récepteur de GABA_{B} selon la revendication 1.

17. Procédé d'identification d'un acide nucléique codant pour un récepteur de GABA_{B} ou une protéine de récepteur de GABA_{B}, comprenant les étapes consistant à :
préparer une banque d'expression codant pour des molécules d'ADNc qui codent potentiellement pour un récepteur de GABA_{B} ou une protéine de récepteur de GABA_{B};
cribler la banque d'expression avec un ligand spécifique capable de se lier à un récepteur de GABA_{B} ou à une protéine de récepteur de GABA_{B}, le ligand spécifique étant le composé de formule 1 : 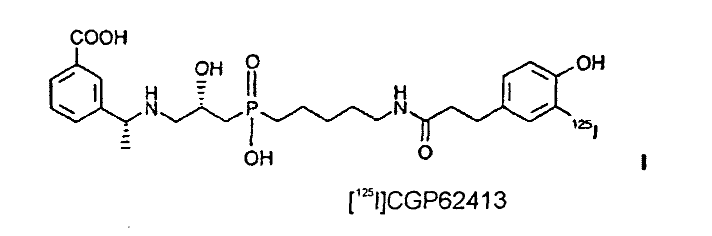
ou le composée de formule II : 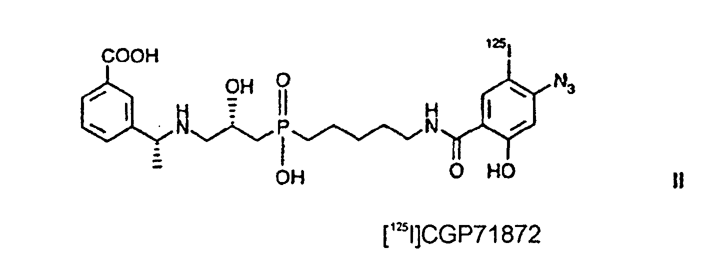 et isoler le clone d'ADNc codant pour un récepteur de GABA_{B} ou une protéine de récepteur de GABA_{B}.

18. Procédé d'identification d'un acide nucléique codant pour un récepteur de GABA_{B} ou une protéine de récepteur de GABA_{B}, comprenant les étapes consistant à :
(a) préparer une banque codant pour des molécules d'ADNc ou d'ADN génomique qui codent potentiellement pour un récepteur de GABA_{B} ou une protéine de récepteur de GABA_{B};
(b) cribler la banque par hybridation avec une sonde d'acide nucléique selon la revendication 10 ; et
(c) identifier les molécules d'acide nucléique qui s'hybrident à la sonde.

19. Procédé de criblage de composés ou de mélanges de composés qui sont des modulateurs potentiels de l'activité du récepteur de GABA_{B}, comprenant les étapes consistant à :
préparer un système d'essai comprenant un récepteur de GABA_{B} ou une protéine de récepteur de GABA_{B} recombinant(e) ;
exposer le système d'essai au composé ou au mélange de composés ;
identifier le composé ou le mélange de composés qui provoque une modulation de l'activité du récepteur de GABA_{B} telle que mesurée par le système d'essai.

20. Procédé de criblage de composés ou de mélanges de composés qui sont des modulateurs potentiels de l'expression du récepteur de GABA_{B}, comprenant les étapes consistant à :
fournir un système d'expression comprenant un gène d'essai lié de manière fonctionnelle à des séquences témoins normalement associées à un gène codant pour un récepteur de GABA_{B} ou une protéine de récepteur de GABA_{B};
identifier les composés qui provoquent une modification du niveau d'expression du gène d'essai.

21. Antagoniste sélectif du récepteur de GABA_{B} de formule I ou II selon la revendication 2.
